# EUROPEAN PATENT SPECIFICATION

(11) **EP 4 493 244 B1**
(45) Date of publication and mention of the grant of the patent: **06.05.2026**
(21) Application number: 23717366.1
(22) Date of filing: 16.03.2023
(51) Int. Cl.: A61M 16/00, A61M 16/08, A61M 16/20

(54) **AN APPARATUS FOR VENTILATING A PATIENT OR TREATING A PATIENT EXPERIENCING RESPIRATORY DISTRESS**
VORRICHTUNG ZUR BEATMUNG EINES PATIENTEN ODER BEHANDLUNG EINES PATIENTEN MIT ATEMNOT
APPAREIL POUR VENTILER UN PATIENT OU TRAITER UN PATIENT SOUFFRANT D'UNE DÉTRESSE RESPIRATOIRE

(30) Priority: 16.03.2022 US 202263269412 P
(43) Date of publication of application: 22.01.2025
(60) Divisional application: 26163056.0
(73) Proprietor: ZOLL Medical Corporation, Chelmsford, MA 01824 (US)
(72) Inventor: HARVEY, Brian P., Belmont, Massachusetts 02478 (US); BECK, George, Salem, Massachusetts 01970 (US); LECROY, Dorian, Parsippany, New Jersey 07054 (US); CHANEY, Tyler R., Rosharon, Texas 77583 (US); HUDAK, Jesse A., Pearland, Texas 77584 (US); PETTYS, Richard L., Pearland, Texas 77584 (US); SOLAREWICZ, Konrad S., Pearland, Texas 77581 (US); AJAZ, Rizwan, Manvel, Texas 77578 (US); SMOOT, David J., Manvel, Texas 77578 (US); WONG, Aaron S., Pearland, Texas 77584 (US); COCHRAN, Jack E., Houston, Texas 77251 (US)
(74) Representative: Potter Clarkson
(86) International application number: PCT/US2023/064508
(87) International publication number: WO 2023/178225

(56) References cited:
- EP-A1- 3 277 351
- WO-A1-2021/077173
- WO-A1-2021/077173
- WO-A1-2022/072438
- WO-A2-2021/211350
- WO-A2-2021/211350
- CN-A- 113 397 721
- CN-U- 214 105 509
- CN-U- 214 912 274
- US-A1- 2013 239 961
- US-A1- 2013 247 912
- US-A1- 2020 139 067
- US-A1- 2020 139 067
- US-A1- 2021 205 554
- US-A1- 2021 205 554
- US-A1- 2021 308 400
- US-A1- 2021 308 400
- US-B2- 10 960 158

## Description

### BACKGROUND

Respiratory distress is a common patient complaint that can sometimes signal a medical emergency. By some accounts, respiratory distress is a major complaint in approximately 12% of EMS calls. In an associated emergency response, responders may be posed with a number of complex but critical patient care related challenges, including, for example, providing mechanical ventilation in difficult settings.

Responding care providers may face a wide range of response settings, including pre- and out-of-hospital settings, from common indoor and outdoor public and private spaces to out-of-hospital settings that could even include battlefield or mass injury event settings. Other settings could include ambulance or other transportation contexts, hospital or emergency room arrival, hand-off and transitional contexts, or other inhospital or emergency room settings. In such settings, the responding care providers, sometimes with limited training or experience and with distractions, must urgently provide life-saving care, which may include providing mechanical ventilation, for substantial periods of time. The scope or effectiveness of their potentially life-saving interventions may be governed or limited based on the features of the equipment that they are able to carry with them and use at the scene. Such features may include, for example, portability, simplicity, ease of use, integration, efficiency, and reliability under potentially unstable, diverse or extreme physical conditions or other circumstances.

US2021308400A1 discloses a ventilator system including: a control system having a controller and a user interface; and a pneumatic system having an inspiratory channel and an expiratory channel. The inspiratory channel further comprises a blower connected to an oxygen source, wherein the blower is configured to be controlled by the controller, and to deliver oxygen and/or air to a patient via the inspiratory channel. The expiratory channel includes an exhaust valve configured to be controlled by the controller and an outlet. The pneumatic system further includes a plurality of sensors configured to provide oxygen measurements, pressure measurements, and flow measurements. The controller is configured to receive a user input via the user interface for controlling a treatment regimen for the patient, and to control the blower and the exhaust valve according to the treatment regimen.

WO2021077173A1 discloses a sensor module assembly for a respiratory therapy apparatus which may be configured for a seal-based securement within a respiratory therapy device that includes a separable pneumatic block to make assembly or replacement more efficient. The sensor module assembly may include a flow manifold. The flow manifold may have an inlet and an outlet and a main pneumatic path between the inlet and the outlet. The assembly may have a sensor module with sensor(s) on a printed circuit board. The sensor module may be pneumatically and fixedly coupled to the flow manifold. The inlet of the flow manifold may be configured for removable coupling with an outlet of the pneumatic block assembly. Moreover, the outlet of the flow manifold may be configured for removable coupling with a pneumatic path of a housing panel of the respiratory therapy apparatus. The pneumatic path is adapted for supplying breathable gas to a patient interface.

US2020139067A1 discloses a system for preventing cross-contamination in single-limb ventilators. In one embodiment, the system includes an airflow generator connected in-line to a humidifier, a first check valve and a patient interface by a gas flow circuit. A controller is electrically coupled to the airflow generator, and a cartridge is connected to the gas flow circuit between a first point downstream of the humidifier and a second point upstream of the patient interface. The cartridge includes a bacteria filter and the first check valve. A method for preventing cross-contamination in single-limb ventilators and a method for providing gaseous flow through a single-limb ventilator are also described.

US2021205554A1 discloses a respiratory treatment apparatus configured to provide a flow of breathable gas to a patient, including a breathable air outlet, an outside air inlet, and an pneumatic block module, wherein the pneumatic block module includes: a volute assembly including an inlet air passage, a mount for a blower and an outlet air passage; the blower being mounted in the mount such that an impeller of the blower is in a flow passage connecting the inlet air passage and the outlet air passage; a casing enclosing the volute assembly, wherein air passages within the casing connect air ports on the volute assembly, wherein the inlet air passage of the volute assembly is in fluid communication with the outside air inlet and the outlet air passage of the volute assembly is in fluid communication with the air outlet.

WO2021211350A2 discloses a medical breathing gas delivery system design employs a manifold delivering gas in a controlled fashion to patients which includes two inhaled gas one-way valves, at least one pressure sensor for patient airway pressure monitoring, and one controlled exhalation pressure proportional control valve which may be overridden by patient exhaled pressure or if there is a power loss. The manifold is connected to a controlled source of breathing gas which may, for example, be a variablespeed fan, or a pressure-based gas flow controller with dynamic self-calibration employing a fast-acting valve and a pressure sensor, either of which yield predictable gas flow control with a minimum of components. The manifold exhalation pressure control valve and gas flow source may, for example, be controlled with a computer system which adjusts the valve power waveforms to attain the time-varying flow and pressure curves required by clinicians, then stores and displays the waveforms to enable long-term trend monitoring and alarm generation. Accurate gas mixing using the pressure-based gas flow control yields automatically calibrated mixes which are of use for patients in, for example, intensive care ventilation and in anesthesia machines for operating rooms.

### SUMMARY OF THE INVENTION

According to the invention we provide an apparatus for ventilating a patient or treating a patient experiencing respiratory distress in accordance with claim 1, and having optional features as defined in the dependent claims.

### SUMMARY OF THE DISCLOSURE

Reference will now be made to various exemplary embodiments or aspects of the present disclosure. Embodiments or aspects disclosed herein which do not fall under the scope of the appended claims do not form part of the present invention, but are useful for understanding the principles of the invention. The scope of the present invention is defined by the appended claims.

One example of an apparatus for treating a patient experiencing respiratory distress, the apparatus includes: a portable ventilator, comprising: a housing; a mechanical ventilation apparatus, disposed within the housing, for providing mechanical ventilation to the patient, comprising: a gas flow generator disposed within the housing, and a gas delivery apparatus, disposed at least partially within the housing, coupled with the gas flow generator, configured to couple with a breathing circuit extending from the housing and configured to interface with the patient at least in part for delivery of gas to the patient, wherein the housing comprises a handle configured to allow operation, by a single hand, of the portable ventilator for the providing of the mechanical ventilation to the patient, the operation, by the single hand, of the portable ventilator comprising grasping the handle to support at least a portion of a weight of the portable ventilator using one or more grasping fingers of the single hand; an interface comprising a display, the display configured to display at least one ventilation related setting, visible while performing the operation, by the single hand, of the portable ventilator, the interface comprising one or more first buttons or dials, the one or more first buttons or dials configured to be operable to allow making a first selection relating to the at least one ventilation related setting; wherein the operation, by the single hand, of the portable ventilator comprises operating the one or more first buttons or dials using one or more first fingers of the single hand during the grasping of the graspable structure using the one or more grasping fingers of the single hand, wherein the one or more first fingers are different than the one or more grasping fingers; and a controller, disposed within the housing, the controller comprising a processor and a memory, the controller being configured to control the mechanical ventilation apparatus of the portable ventilator in the providing of the mechanical ventilation to the patient.

In some examples, the interface comprises one or more second buttons or dials configured to be operable to make a second selection relating to at least one second ventilation related setting, and wherein the operation, by the single hand, of the portable ventilator further comprises operating the one or more second buttons or dials using one or more second fingers of the single hand, wherein the one or more second fingers are different than the one or more first fingers and the one or more grasping fingers.

In some examples, the interface comprises one or more second buttons or dials configured to be operable to make a second selection relating to at least one second ventilation related setting, and wherein the operation, by the single hand, of the portable ventilator further comprises operating the one or more second buttons or dials using one or more second fingers of the single hand, wherein the one or more second fingers are different than the one or more first fingers and the one or more grasping fingers.

In some examples, the handle comprises a portion of the housing. In some examples, the handle at least in part forms an aperture through the housing. In some examples, at least a portion of the handle protrudes from a portion of the housing. In some examples, the one or more first buttons or dials comprises at least one dial, wherein the dial is configured to be turnable. In some examples, the one or more first buttons or dials comprises at least one dial, wherein the dial is configured to be scrollable. In some examples, the one or more first buttons or dials comprises at least one dial, wherein the dial is configured to be shiftable in position. In some examples, the one or more first buttons or dials comprise at least one physical button or dial. In some examples, the at least one physical button or dial comprises at least one knob. In some examples, the one or more first buttons or dials comprise at least one display based button or dial. In some examples, the display comprises the at least one display based button or dial. In some examples, the handle comprises at least one of the one or more first buttons or dials, and wherein the handle is configured to allow the grasping by the one or more grasping fingers during operation of the at least one of the one or more first buttons or dials by at least one of the one or more first fingers. In some examples, the at least one of the one or more first buttons or dials is disposed on a top portion of the handle. In some examples, the at least one of the one or more first buttons or dials is disposed on a side portion of the handle.

In some examples, the housing comprises a first portion comprising the handle and a second portion that does not comprise the handle. In some examples, the second portion of the housing comprises at least one of the one or more first buttons or dials. In some examples, the housing is configured such that the one or more grasping fingers comprise at least one finger other than a thumb, and wherein the one or more first fingers comprise the thumb. In some examples, the housing is configured such that the one or more grasping fingers comprise at least one finger other than a thumb, and wherein the one or more first fingers comprise an index finger. In some examples, at least a portion of the display is adjacent to at least a portion of the handle.

One example of an apparatus for treating a patient experiencing respiratory distress, the apparatus includes: a portable ventilator, comprising: a housing; a mechanical ventilation apparatus, disposed within the housing, for providing mechanical ventilation to the patient, comprising: a gas flow generator disposed within the housing, and a gas delivery apparatus, disposed at least partially within the housing, coupled with the gas flow generator, configured to couple with a breathing circuit extending from the housing and configured to interface with the patient at least in part for delivery of gas to the patient; a graspable structure configured to allow operation, by a single hand, of the portable ventilator for the providing of the mechanical ventilation to the patient, the operation, by the single hand, of the portable ventilator comprising grasping the graspable structure to control a position of the portable ventilator using one or more grasping fingers of the single hand; an interface configured to display at least one ventilation related setting, visible while performing the operation, by the single hand, of the portable ventilator, the interface comprising one or more first controls configured to be operable to allow making a first selection relating to the at least one ventilation related setting, wherein the operation, by the single hand, of the portable ventilator comprises operating the one or more first controls using one or more first fingers of the single hand during the grasping of the graspable structure using the one or more grasping fingers of the single hand, wherein the one or more first fingers are different than the one or more grasping fingers; and a controller, disposed within the housing, the controller comprising a processor and a memory, the controller being configured to control the mechanical ventilation apparatus of the portable ventilator in the providing of the mechanical ventilation to the patient.

In some examples, the interface comprises one or more second controls configured to be operable to make a second selection relating to at least one second ventilation related setting, and wherein the operation, by the single hand, of the portable ventilator further comprises operating the one or more second controls using one or more second fingers of the single hand, wherein the one or more second fingers are different than the one or more first fingers and the one or more grasping fingers. In some examples, the interface is configured to allow operating the one or more first controls simultaneously with operating the one or more second controls. In one or more examples, the first controls and the second controls are positioned at different locations on the housing or handle so that they are operable by different fingers. In some examples, the portable ventilator is configured such that the operation, by the single hand, of the portable ventilator comprises the grasping of the graspable structure to support at least a portion of weight of the portable ventilator using the one or more grasping fingers of the single hand. In some examples, the graspable structure comprises a handle. In some examples, the housing comprises at least a portion of the handle. In some examples, the graspable structure at least in part forms an aperture through the housing.

In some examples, the graspable structure is configured to be grasped by the one or more grasping fingers excluding at least a thumb. In some examples, the graspable structure is configured to be grasped by the one or more grasping fingers excluding at least a thumb and at least one of: a little finger, a ring finger, a middle finger and an index finger. In some examples, the graspable structure is configured to be grasped by the one or more grasping fingers including a little finger, a ring finger, a middle finger and an index finger. In some examples, the graspable structure is configured to be grasped by the one or more grasping fingers excluding at least an index finger. In some examples, the graspable structure is configured to be grasped by the one or more grasping fingers including a little finger, a ring finger, a middle finger and a thumb. In some examples, the graspable structure is configured to be grasped by the one or more grasping fingers excluding at least a thumb and an index finger. In some examples, the graspable structure is configured to be grasped by the one or more grasping fingers including a little finger, a ring finger and a middle finger. In some examples, the graspable structure is configured to be grasped by the one or more grasping fingers including a little finger and a ring finger. In some examples, the interface comprises a display configured to display the at least one ventilation related setting. In some examples, the display comprises at least one of: an LCD display, an LED display, an OLED display, an AMOLED display, a super AMOLED display, an IPS display, a TFT display, a PLS display, an LTPS display, and electronic ink display, and an LTPO display. In some examples, at least a portion of the one or more first controls are display-based. In some examples, at least a portion of the one or more first controls are GUI-based. In some examples, the display comprises at least a portion of the one or more first controls. In some examples, at least a portion of the one or more first controls comprises at least one physical, non-display based control. In some examples, the at least one physical, non-display based control comprises at least one pressable control. In some examples, the at least one at least one pressable control comprises at least one button. In some examples, the one or more first controls comprise at least one control configured to enable a selection between a plurality of choices.

In some examples, the at least one control configured to enable the selection between the plurality of choices comprises at least one of a movable control, a turnable control, a rotatable control, a scrollable control and a dialable control. In some examples, the at least one control comprises at least one of a scroll wheel, a dial and a knob. In some examples, the scroll wheel or dial may be positioned for control by the thumb or index finger, which may have the dexterity to operate such types of controls. In some examples, the one or more first controls comprises at least one control configured to enable increasing or decreasing a value of a parameter. In some examples, the one or more first controls comprises at least one control configured to enable a selection and an acceptance of a displayed mode of operation, a displayed parameter or a displayed parameter value. In some examples, at least a portion of the one or more first controls is disposed at least in part on or within the housing. In some examples, at least a portion of the one or more first controls is disposed at least in part on or within the graspable structure. In some examples, at least a portion of the one or more first controls is disposed at least in part on or within the handle.

In some examples, the display comprises at least one light or lighted area. In some examples, the at least one light or lighted area is disposed at least in part on or within the housing. In some examples, the at least one light or lighted area is disposed at least in part on or within the graspable structure. In some examples, the graspable structure comprises a handle, and wherein the at least one light or lighted area is disposed at least in part on or within the handle. In some examples, the at least one light or lighted area is configured to visually signal at least one condition. In some examples, the at least one condition comprises at least one of: an on condition, an off condition, an operating condition, or non-operating condition, a correct functioning condition, an incorrectly functioning condition, an alert and an alarm. In some examples, the at least one condition is visually signaled at least in part by at least one of: a color of the at least one light or lighted area and a flashing, flashing pattern or flashing frequency of the at least one light or lighted area.

In some examples, the one or more first controls are configured to be operated at least in part by the thumb. In some examples, the one or more first controls are configured to be operated at least in part by an index finger. In some examples, the one or more first controls are configured to be operated at least in part by the thumb and the index finger. In some examples, the one or more first controls are configured to be operated at least in part by the thumb, the index finger and the middle finger.

In some examples, the gas flow generator, in operation, produces a sound of between 40 and 70 decibels at one meter. In some examples, the housing of the portable ventilator has a volume of between 5.0 and 27 liters. In some examples, the housing of the portable ventilator has a volume of between 1.0 and 5.0 liters. In some examples, the housing of the portable ventilator has a volume of between 0.5 and 1.0 liter. In some examples, the housing of the portable ventilator has a volume of between 0.15 and 0.5 liters. In some examples, the housing of the portable ventilator has a volume of between 0.125 and 0.15 liters. In some examples, the portable ventilator has a weight of between 2.0 and 5.0 kilograms. In some examples, the portable ventilator has a weight of between 1.0 and 2.0 kilogram. In some examples, the portable ventilator has a weight of between 0.3 and 1.0 kilograms. In some examples, the portable ventilator has a weight of between 0.2 and 0.3 kilograms. In some examples, the portable ventilator is dimensioned such that none of a length, a height and a width of the portable ventilator is greater than 300 mm. In some examples, the portable ventilator is dimensioned such that none of a length, a height and a width of the portable ventilator is greater than 150 mm. In some examples, the portable ventilator is dimensioned such that none of a length, a height and a width of the portable ventilator is greater than 100 mm. In some examples, the portable ventilator is dimensioned such that none of a length, a height and a width of the portable ventilator is greater than 60 mm.

In some examples, the portable ventilator is configured for use in patient treatment outside of a hospital. In some examples, the portable ventilator is configured for use in patient treatment inside a vehicle. In some examples, the portable ventilator is configured for use in patient treatment in an outdoor setting.

In some examples, the gas delivery apparatus comprises a pneumatic manifold, comprising an exhalation channel and an inhalation channel, configured to couple with the breathing circuit. In some examples, the portable ventilator comprises an exhalation valve configured for use in restricting gas flow through at least a portion of the exhalation channel. In some examples, the exhalation valve is configured for use, during the providing of the mechanical ventilation to the patient, in maintaining specified exhalation periods and in maintaining a specified baseline airway pressure during the providing of the mechanical ventilation to the patient. In some examples, the gas flow generator comprises a blower. In some examples, the portable ventilator is configured such that all gas flow generated by the blower is delivered into an inhalation limb of the breathing circuit. In some examples, the portable ventilator comprises, disposed within the housing, an exhalation valve pressure regulator configured for use in providing a specified actuation pressure to the exhalation valve.

In some examples, the exhalation valve pressure regulator comprises at least two proportional valves comprising: at least one valve configured for use in increasing an amount of actuation pressure to the exhalation valve; and at least one valve configured for use in decreasing the amount of actuation pressure to the exhalation valve. In some examples, the portable ventilator comprises, disposed within the housing, an air characterizer comprising the at least one electrical circuit board comprising at least one pressure sensor and at least two flow sensors. In some examples, the breathing circuit comprises an exhalation limb and an inhalation limb. In some examples, the exhalation valve is disposed within the air characterizer. In some examples, the exhalation valve is disposed within the breathing circuit. In some examples, the exhalation valve is disposed within an exhalation limb of the breathing circuit. In some examples, the apparatus comprises an exhalation valve control tube extending from the breathing circuit to within the housing of the portable ventilator and configured for use in providing actuation pressure to the exhalation valve. In some examples, the apparatus comprises a patient airway pressure measurement tube extending from the breathing circuit to within the housing of the portable ventilator and configured for use in allowing measuring of a patient airway pressure using at least a second pressure sensor disposed within the housing.

In some examples, the apparatus comprises a connector, the connector comprising: a first port configured for attachment of an exhalation limb of the breathing circuit; and a second port configured for attachment of an inhalation limb of the breathing circuit; wherein the connector is configured to fixedly attach to the portable ventilator so as to couple the exhalation limb of the breathing circuit with the exhalation channel of the pneumatic manifold and to couple the inhalation limb of the breathing circuit with the inhalation channel of the pneumatic manifold. In some examples, The connector comprises: a first port configured for attachment of an exhalation limb of the breathing circuit; a second port configured for attachment of an inhalation limb of the breathing circuit; and a third port configured for attachment of the exhalation valve control tube; wherein the connector is configured to fixedly attach to the portable ventilator so as to couple the exhalation limb of the breathing circuit with the exhalation channel of the pneumatic manifold, to couple the inhalation limb of the breathing circuit with the inhalation channel of the pneumatic manifold, and to couple the exhalation valve control tube with the pneumatic manifold. In some examples, the connector comprises: a first port configured for attachment of an exhalation limb of the breathing circuit; a second port configured for attachment of an inhalation limb of the breathing circuit; a third port configured for attachment of the exhalation valve control tube; and a fourth port configured for attachment of the patient airway measurement tube; wherein the connector is configured to fixedly attach to the portable ventilator so as to couple the exhalation limb of the breathing circuit with the exhalation channel of the pneumatic manifold, to couple the inhalation limb of the breathing circuit with the inhalation channel of the pneumatic manifold, to couple the exhalation valve control tube with the pneumatic manifold, and to couple the patient airway measurement tube with the pneumatic manifold.

In some examples, the portable ventilator is configured to provide the mechanical ventilation to the patient in modes comprising assist-control (AC), continuous positive airway pressure (CPAP), synchronized intermittent mandatory ventilation (SIMV) and high flow nasal cannula (HFNC).

One example of a method for a care provider treating a patient experiencing respiratory distress includes: the care provider grasping a handle of a portable ventilator with one or more grasping fingers of a single hand of the care provider, so as to support at least a portion of weight of the portable ventilator, while providing mechanical ventilation to the patient using the portable ventilator; and the care provider, while grasping the handle of the portable ventilator and while providing the mechanical ventilation to the patient using the portable ventilator, operating one or more buttons or dials of the portable ventilator with one or more first fingers of the single hand, wherein the one or more first fingers are different than the one or more grasping fingers, wherein the care provider operating the one or more buttons or dials comprises the care provider making a first selection relating to at least one ventilation relating setting, wherein the at least one ventilation related setting is displayed on a display of the portable ventilator, wherein the display is visible to the care provider while grasping the handle of the portable ventilator and providing the mechanical ventilation to the patient using the portable ventilator.

One example of an apparatus for treating a patient experiencing respiratory distress, the apparatus includes: a portable ventilator, comprising: a housing; a mechanical ventilation apparatus, disposed within the housing, for providing mechanical ventilation to the patient, comprising: a gas flow generator disposed within the housing, and a gas delivery apparatus, disposed at least partially within the housing, coupled with the gas flow generator, the gas delivery apparatus comprising: a rigid pneumatic manifold, comprising an exhalation channel and an inhalation channel, configured to couple with a breathing circuit extending from the housing and configured to interface with the patient at least in part for delivery of gas to the patient, and at least one electrical circuit board, comprising at least one pressure sensor and at least two flow sensors comprising a first flow sensor and a second flow sensor, the at least one electrical circuit board being fixedly joined with the pneumatic manifold such that the first flow sensor is capable of measuring a first gas flow rate through the exhalation channel and such that the second flow sensor is capable of measuring a second gas flow rate through the inhalation channel; and a controller, disposed within the housing, the controller comprising a processor and a memory, the controller being configured to control the mechanical ventilation apparatus of the portable ventilator in providing the mechanical ventilation to the patient.

In some examples, the at least one pressure sensor comprises a first pressure sensor configured for measuring a patient airway pressure during the providing of the mechanical ventilation to the patient. In some examples, the at least one pressure sensor comprises two pressure sensors configured for measuring a patient airway pressure during the providing of the mechanical ventilation to the patient. In some examples, the at least one pressure sensor comprises a first pressure sensor and a second pressure sensor, and wherein the electrical circuit board is configured such that the first pressure sensor is capable of measuring a first gas pressure through the exhalation channel and such that the second pressure sensor is capable of measuring a second gas pressure through the inhalation channel. In some examples, the at least one pressure sensor comprises a first, a second, a third and a fourth pressure sensor, and wherein the electrical circuit board is configured such that the first and second pressure sensors are capable of measuring a first gas pressure through the exhalation channel and such that the third and fourth pressure sensors are capable of measuring a second gas pressure through the inhalation channel.

In some examples, the electrical circuit board and the pneumatic manifold are configured so as to be joined so as to create a fixed but flexible gas leak-free seal between the pneumatic manifold and at least one pressure sensor and between the pneumatic manifold and each of the at least two flow sensors. In one or more examples, the electrical circuit board and the pneumatic manifold are configured so as to be joined in fixed relation to one another such that relative movement between the electrical circuit board and the pneumatic manifold is limited to provide at least part of the fixed but flexible gas leak-free seal. This arrangement may provide more reliable measurement by the various sensors as the flow/pressure characteristics are controlled by the fixed relation join, which may avoid use of lengthy tubing that may become crimped. In one or more examples, the pneumatic manifold and the at least one pressure sensor as well as the pneumatic manifold and each of the at least two flow sensors are sealingly coupled by a flexible coupling which may, in one or more examples, provide a gas leak free seal. Thus, the fixed but flexible gas leak-free seal may be provided by joining the electrical circuit board and the pneumatic manifold in a way that holds them in fixed relation, and providing a flexible coupling between the various sensors and the pneumatic manifold to provide reliable sealing. In one or more examples, the electrical circuit board and the pneumatic manifold are arranged so as to limit flexing of the flexible coupling. In some examples, the fixed but flexible gas leak-free seal comprises at least one O-ring. In some examples, the fixed but flexible gas leak-free seal comprises at least one gasket. In some examples, the fixed but flexible gas leak-free seal has a temperature tolerance range of at least between 0 and 40 degrees Celsius. In some examples, the electrical circuit board is rigid. In some examples, the portable ventilator is configured to enable single-handed operation during the providing of the mechanical ventilation to the patient. In some examples, the portable ventilator is configured for use in patient treatment outside of a hospital.

In some examples, the portable ventilator comprises an exhalation valve configured for use in restriction of gas flow through at least a portion of the exhalation channel. In some examples, the exhalation valve is configured for use, during the providing of the mechanical ventilation to the patient, in maintaining specified exhalation periods and in maintaining a specified baseline airway pressure (BAP). In some examples, the exhalation valve is a proportional valve. In some examples, the exhalation valve is a pneumatically actuated valve. In some examples, the exhalation valve is a diaphragm valve. In some examples, the gas flow generator comprises a blower. In some examples, the gas flow generator comprises a centrifugal blower. In some examples, the portable ventilator is configured such that all gas flow generated by the blower is delivered into an inhalation limb of the breathing circuit. In some examples, the portable ventilator comprises, disposed within the housing, an exhalation valve pressure regulator configured for use in providing a specified actuation pressure to the exhalation valve. In some examples, the exhalation valve pressure regulator comprises at least two proportional valves comprising: at least one valve configured for use in increasing an amount of actuation pressure to the exhalation valve; and at least one valve configured for use in decreasing the amount of actuation pressure to the exhalation valve.

In some examples, the portable ventilator comprises, disposed within the housing, an air characterizer comprising the at least one electrical circuit board. In some examples, the exhalation valve pressure regulator and the air characterizer are configured to fixedly join so as to form at least a portion of an exhalation valve control channel extending from the exhalation valve pressure regulator to the air characterizer. In some examples, the apparatus comprises a breathing circuit comprising an exhalation limb and an inhalation limb. In some examples, the exhalation valve is disposed within the air characterizer. In some examples, the exhalation valve is disposed within the breathing circuit. In some examples, the exhalation valve is disposed within an exhalation limb of the breathing circuit. In some examples, the apparatus comprises an exhalation valve control tube extending from the breathing circuit to within the housing of the portable ventilator and configured for use in providing actuation pressure to the exhalation valve. In some examples, the apparatus comprises a patient airway pressure measurement tube extending from the breathing circuit to within the housing of the portable ventilator and configured for use in allowing measuring of a patient airway pressure using at least a second pressure sensor disposed within the housing.

In some examples, the apparatus comprises a connector, the connector comprising: a first port configured for attachment of an exhalation limb of the breathing circuit; and a second port configured for attachment of an inhalation limb of the breathing circuit; wherein the connector is configured to fixedly attach to the portable ventilator so as to couple the exhalation limb of the breathing circuit with the exhalation channel of the pneumatic manifold and to couple the inhalation limb of the breathing circuit with the inhalation channel of the pneumatic manifold. In some examples, the connector comprises: a first port configured for attachment of an exhalation limb of the breathing circuit; a second port configured for attachment of an inhalation limb of the breathing circuit; and a third port configured for attachment of the exhalation valve control tube; wherein the connector is configured to fixedly attach to the portable ventilator so as to couple the exhalation limb of the breathing circuit with the exhalation channel of the pneumatic manifold, to couple the inhalation limb of the breathing circuit with the inhalation channel of the pneumatic manifold, and to couple the exhalation valve control tube with the pneumatic manifold. In some examples, the connector comprises: a first port configured for attachment of an exhalation limb of the breathing circuit; a second port configured for attachment of an inhalation limb of the breathing circuit; a third port configured for attachment of the exhalation valve control tube; and a fourth port configured for attachment of the patient airway measurement tube; wherein the connector is configured to fixedly attach to the portable ventilator so as to couple the exhalation limb of the breathing circuit with the exhalation channel of the pneumatic manifold, to couple the inhalation limb of the breathing circuit with the inhalation channel of the pneumatic manifold, to couple the exhalation valve control tube with the pneumatic manifold, and to couple the patient airway measurement tube with the pneumatic manifold.

In some examples, the portable ventilator is configured to provide the mechanical ventilation to the patient in modes comprising assist-control (AC), continuous positive airway pressure (CPAP), synchronized intermittent mandatory ventilation (SIMV) and high flow nasal cannula (HFNC).

One example of an apparatus for treating a patient experiencing respiratory distress, the apparatus includes: a portable ventilator, comprising: a housing; a mechanical ventilation apparatus, disposed within the housing, for providing mechanical ventilation to the patient, comprising: a blower, disposed within the housing and comprising a blower motor, configured to generate gas flow, the blower being configured to have a rotor moment of inertia of between 1 and 30 g·cm²; a gas delivery apparatus, disposed at least partially within the housing, coupled with the blower, configured to couple with a breathing circuit extending from the housing and configured to interface with the patient at least in part for delivery of gas to the patient; a controller, disposed within the housing, the controller comprising a processor and a memory, the controller being configured to control the mechanical ventilation apparatus of the portable ventilator in providing the mechanical ventilation to the patient, the controller being configured to: receive or determine at least one of a target flow rate and a target pressure for gas being delivered to the patient; determine a blower motor control signal for causing at least one of a flow rate and a pressure of the gas being delivered to the patient to move toward the at least one of the target flow rate and the target pressure; and actuate the blower motor using the determined blower motor control signal.

In some examples, the blower is configured to have the moment of inertia of between 2 and 30 g·cm², between 3 and 30 g·cm², between 3 and 20 g·cm², between 1 and 10 10 g·cm², between 2 and 10 g·cm², between 3 and 10 g·cm², between 3 and 7 g·cm², or between 3 and 6.5 g·cm² or any other rotor moment of interia. In some examples, the blower is configured to have an acceleration of between 100 and 1,000 rpm/msec, between 200 and 1,000 rpm/msec, between 300 and 1,000 rpm/msec, or between 400 and 1,000 rpm/msec.

In some examples, the controller is configured to determine the blower motor control signal based at least in part on one of a flow rate and a pressure of the gas being delivered to the patient, such as a current flow rate or a current pressure of the gas being delivered or, e.g., a recent average thereof. In some examples, the controller is configured to determine the blower motor control signal, wherein causing the at least one of the flow rate and the pressure of the gas being delivered to the patient to move toward the at least one of the target flow rate and the target pressure comprises causing the at least one of the flow rate and the pressure of the gas being delivered to the patient to increase or to decrease so that the at least one of the flow rate and the pressure gets closer to the at least one of the target flow rate and the target pressure. In some examples, the portable ventilator is configured such that all gas flow generated by the blower is delivered into an inhalation limb of the breathing circuit. In some examples, the portable ventilator is configured such that all gas flow generated by the blower is delivered to the patient. In some examples, the portable ventilator is configured such that no portion of gas flow generated by the blower is exhausted from the portable ventilator before being delivered into an inhalation limb of the breathing circuit. In some examples, the portable ventilator is configured such that no portion of gas flow generated by the blower is exhausted from the portable ventilator before being delivered to the patient. In some examples, the portable ventilator is configured so as not to require use of a valve to regulate a portion of gas flow generated by the blower that is delivered into an inhalation limb of the breathing circuit. In some examples, the portable ventilator is configured so as not to require use of a valve to restrict a portion of gas flow generated by the blower that is delivered to the patient. In some examples, the portable ventilator is configured so as not to require use of a valve to direct a portion of gas flow generated by the blower to be exhausted from the portable ventilator without being delivered into an inhalation limb of the breathing circuit. In some examples, the portable ventilator is configured so as not to require use of a valve to direct a portion of gas flow generated by the blower to be exhausted from the portable ventilator without being delivered to the patient.

In some examples, the controller is configured to determine the blower motor control signal using a two algorithmic loop control scheme. In some examples, the controller is configured to determine the blower motor control signal using a single algorithmic loop control scheme. In some examples, the blower motor control signal corresponds with a blower motor torque for causing the at least one of the flow rate and the pressure of the gas being delivered to the patient to move toward the at least one of the target flow rate and the target pressure. In some examples, the controller determining the blower motor control signal does not comprise the controller determining a blower motor speed. In some examples, the blower motor control signal corresponds with the blower motor torque for causing gas flow generated by the blower to result in the at least one of the flow rate and the pressure of the gas being delivered to the patient to move toward the at least one of the target flow rate and the target pressure. In some examples, the controller is configured to: determine the target flow rate for the gas being delivered to the patient; determine the blower motor control signal for causing the flow rate of the gas to be delivered to the patient to move toward the target flow rate while the pressure of the gas being delivered to the patient remains within a maximum pressure limit; and actuate the blower motor using the determined blower motor control signal. In some examples, the controller is configured to determine the blower control signal, wherein the maximum pressure limit is a maximum safe pressure limit.

In some examples, the controller is configured to: determine the target pressure for gas being delivered to the patient; determine the blower motor control signal for causing the pressure of the gas being delivered to the patient to move toward the target pressure while the flow rate of the gas being delivered to the patient remains within a maximum flow rate limit; and actuate the blower motor using the determined blower motor control signal. In some examples, the controller is configured to determine the blower control signal, wherein the maximum flow rate limit is a maximum safe flow rate limit. In some examples, the controller is configured to execute at least one algorithm that receives as input or determines at least one of a target flow rate and a target pressure, and that determines, as output, a blower motor control signal. In some examples, the blower comprises a compressor. In some examples, the blower comprises an impeller. In some examples, the portable ventilator is configured for use in patient treatment outside of a hospital. In some examples, the portable ventilator is configured to enable single-handed operation during the providing of the mechanical ventilation to the patient.

In some examples, the gas delivery apparatus comprises a pneumatic manifold, comprising an exhalation channel and an inhalation channel, configured to couple with the breathing circuit. In some examples, the portable ventilator comprises an exhalation valve configured for use in restricting gas flow through at least a portion of the exhalation channel. In some examples, the exhalation valve is configured for use, during the providing of the mechanical ventilation to the patient, in maintaining specified exhalation periods and in maintaining a specified baseline airway pressure during the providing of the mechanical ventilation to the patient. In some examples, the portable ventilator comprises, disposed within the housing, an exhalation valve pressure regulator configured for use in providing a determined pressure to the exhalation valve. In some examples, the exhalation valve pressure regulator comprises at least two proportional valves comprising: at least one valve configured for use in increasing an amount of actuation pressure to the exhalation valve; and at least one valve configured for use in decreasing the amount of actuation pressure to the exhalation valve. In some examples, the portable ventilator comprises, disposed within the housing, an air characterizer comprising at least one electrical circuit board comprising at least one pressure sensor and at least two flow sensors. In some examples, the apparatus comprises the breathing circuit, wherein the breathing circuit comprises an exhalation limb and an inhalation limb.

In some examples, the exhalation valve is disposed within the air characterizer. In some examples, the exhalation valve is disposed within the breathing circuit. In some examples, the exhalation valve is disposed within the exhalation limb of the breathing circuit. In some examples, the apparatus comprises an exhalation valve control tube extending from the breathing circuit to within the housing of the portable ventilator and configured for use in providing actuation pressure to the exhalation valve. In some examples, the apparatus comprises a patient airway pressure measurement tube extending from the breathing circuit to within the housing of the portable ventilator and configured for use in allowing measuring of a patient airway pressure using at least a second pressure sensor disposed within the housing.

In some examples, the apparatus comprises a connector comprising: a first port for attachment of an exhalation limb of the breathing circuit; and a second port for attachment of an inhalation limb of the breathing circuit; wherein the connector is configured to fixedly attach to the portable ventilator so as to couple the exhalation limb of the breathing circuit with the exhalation channel of the pneumatic manifold and to couple the inhalation limb of the breathing circuit with the inhalation channel of the pneumatic manifold. In some examples, the connector comprises: a first port configured for attachment of an exhalation limb of the breathing circuit; a second port configured for attachment of an inhalation limb of the breathing circuit; and a third port configured for attachment of the exhalation valve control tube; wherein the connector is configured to fixedly attach to the portable ventilator so as to couple the exhalation limb of the breathing circuit with the exhalation channel of the pneumatic manifold, to couple the inhalation limb of the breathing circuit with the inhalation channel of the pneumatic manifold, and to couple the exhalation valve control tube with the pneumatic manifold. In some examples, the connector comprises: a first port configured for attachment of an exhalation limb of the breathing circuit; a second port configured for attachment of an inhalation limb of the breathing circuit; a third port configured for attachment of the exhalation valve control tube; and a fourth port configured for attachment of the patient airway measurement tube; wherein the connector is configured to fixedly attach to the portable ventilator so as to couple the exhalation limb of the breathing circuit with the exhalation channel of the pneumatic manifold, to couple the inhalation limb of the breathing circuit with the inhalation channel of the pneumatic manifold, to couple the exhalation valve control tube with the pneumatic manifold, and to couple the patient airway measurement tube with the pneumatic manifold.

In some examples, the portable ventilator is configured to provide the mechanical ventilation to the patient in modes comprising assist-control (AC), continuous positive airway pressure (CPAP), synchronized intermittent mandatory ventilation (SIMV) and high flow nasal cannula (HFNC).

One example of an apparatus for treating a patient experiencing respiratory distress such as by ventilating a patient comprises a ventilation apparatus, a housing and a controller, disposed within the housing, the controller comprising a processor and a memory, the controller being configured to control the ventilation apparatus in the providing of the ventilation to the patient. The apparatus may have one or more advantageous features relating to one or more of: positioning of the controls; the configuration of how the housing is held during use; the configuration of a manifold; and the determination of a blower control signal for control of a blower that is part of the ventilation apparatus or combinations thereof. In some examples, the apparatus includes one or more of: a gas flow generator; a graspable structure; a handle; an interface comprising a display; rigid pneumatic manifold configured to interface with the patient; and a blower. In other aspects, we provide a controller for an apparatus for ventilating a patient. The controller may be configured to receive feedback from one or more of a pressure sensor and a flow sensor and may be configured to control the blower control signal based on a function of the feedback.

### BRIEF DESCRIPTION OF THE DRAWINGS

Various aspects of embodiments of the present disclosure are discussed below with reference to the accompanying figures, which are not intended to be drawn to scale. The figures are included for illustrative purposes and a further understanding of the various aspects and examples. The figures are incorporated in and constitute a part of this specification, but are not intended to limit the scope of the disclosure. In the figures, identical or nearly identical components that are illustrated in various figures may be represented by like numerals. For purposes of clarity, not every component may be labeled in every figure.
FIG. 1 illustrates an example emergency care environment including an example portable ventilator, a CCM/defibrillator and a tablet.
FIGs. 2-3 illustrate example front and rear views of example portable ventilators.
FIG. 4 is a block diagram of an example portable ventilator.
FIGs. 5-10 are schematic diagrams of example pneumatic manifolds and other components of example portable ventilators.
FIG. 11 illustrates an example side view of an example pneumatic manifold and other components of an example portable ventilator.
FIG. 12 illustrates a cut-away side view of an example inhalation limb and other components of an example portable ventilator.
FIG. 13 illustrates a top view of an example pneumatic manifold and other components of an example portable ventilator.
FIG. 14 illustrates an example side cut-away view of an exhalation limb of an example portable ventilator.
FIG. 15 illustrates an example side cut-away view of an inhalation limb of an example portable ventilator.
FIG. 16 illustrates a top view of a portion of a pneumatic manifold and other components of an example portable ventilator.
FIG. 17 illustrates an exploded view of a blower and other components of an example portable ventilator.
FIG. 18 illustrates views of an air characterizer and other components of an example portable ventilator.
FIG. 19 illustrates a view of an exhalation valve pressure regulator, example source and exhaust valves, an example exhalation valve, and other components of an example portable ventilator.
FIG. 20 includes block diagrams of example multiple port connectors for use with example portable ventilators.
FIG. 21 illustrates views of an example multiple port connector for use with an example portable ventilator.
FIGs. 22-27 illustrate example portable ventilators.
FIGs. 28A-C illustrate example display/GUI features of example portable ventilators.
FIG. 29 illustrates aspects of an example of the mechanical ventilation apparatus for a ventilation system such as a portable ventilator.
FIG. 30 illustrates aspects of example patient circuits that can be used with a portable ventilator.
FIG. 31 illustrates an example of components of various devices described with reference to FIGs. 1-30.

### DETAILED DESCRIPTION

The description set forth below in connection with the appended drawings is intended to be a description of various, illustrative embodiments of the disclosed subject matter. Specific features and functionalities are described in connection with each illustrative embodiment; however, it will be apparent to those skilled in the art that the disclosed embodiments may be practiced without each of those specific features and functionalities.

Some embodiments described herein provide portable ventilators that provide advantages, and solutions to associated technical problems, that may include, for example, portability, simplicity, ease of use, integration, efficiency, and reliability, such as under potentially unstable, diverse or extreme physical conditions or circumstances. Portable ventilators are provided with various aspects and features that contribute to these advantages. These aspects and features can be particularly important for uses that may include, for example, out-of-hospital settings and operation by less trained or less experienced operators who may need to contend with multiple responsibilities and potential distractions. Some embodiments provide an apparatus for treating a patient experiencing respiratory distress which may include ventilating the patient, assisting the respiration of the patient, monitoring the respiration of the patient and/or monitoring the ventilation of the patient.

Some embodiments provide a portable ventilator with features that include a rigid pneumatic manifold and at least one electrical circuit board. The electrical circuit board may include or be coupled with pressure and flow sensors. The electrical circuit board is fixedly joined with the pneumatic manifold so as to allow necessary leak-free mechanical ventilation operation of the portable ventilator under a wide range of conditions. These features can contribute to advantages of the portable ventilator including simplicity, durability and reliability, including potentially during field use that may include movement, physical instability and diverse physical and environmental circumstances. In some embodiments, the electrical circuit board is fixedly joined with the rigid pneumatic manifold so as to create a flexible but leak-free seal between the rigid pneumatic manifold and the sensors of the electrical circuit board.

In some embodiments, a rigid pneumatic manifold is provided that is structured and shaped to allow or provide for necessary leak-free (which can include sufficiently leak-free) gas flow channels to be formed, and for sufficiently accurate, precise coupling with coupled components (e.g., at least one electrical circuit board including or coupled with sensors). Surfaces of the rigid pneumatic manifold may include accurate, precise structuring and shaping to allow for necessary channeling to be formed to components coupled with the rigid pneumatic manifold (e.g., to allow for porting to valves and sensors of a coupled electrical circuit board). In some embodiments, this can include a pneumatic manifold that is formed using precise machining, cutting, molding or other techniques to achieve the necessary precise structure and shape.

Furthermore, in some embodiments, the rigid pneumatic manifold is formed from multiple portions or components (e.g., a blower-based component of the portable ventilator, an exhalation valve pressure regulator and an air characterizer, or portions thereof). The portions or components of the pneumatic manifold may be structured and shaped to align and join, when the pneumatic manifold is assembled or formed as a rigid whole, precisely and accurately enough to allow or provide for necessary continuous, unbroken, leak-free gas flow channels to be formed, such to sensors, for example. In some embodiments, this may require use of leak-free sealing of the porting between the manifold portions, such as the areas where the surfaces meet to form or allow for continuous channeling between the portions or components of the pneumatic manifold. The structuring and shaping may include internal surfaces of the pneumatic manifold (e.g., internal channeling) as well as external surfaces of the pneumatic manifold (e.g., external surfaces of the pneumatic manifold itself, even though, when coupled with other components and when included within a housing, these surfaces may not be external to the assembled or formed overall internal structure within the housing, or to the portable ventilator itself as a whole).

In some embodiments, by use of a rigid pneumatic manifold including precise internal channeling, such as to sensors, much or all of the tubing, such as flexible tubing that might otherwise be required or used within the pneumatic manifold can be avoided. In some embodiments, this can impart advantages, and solve technical problems, such as by providing greater stability (including under potentially challenging physical and environmental conditions), greater reliability, less risk of breakdown, malfunction or leakage, and more efficient and less expensive manufacture, particularly at scale. In varying embodiments, some, most or even all tubing within the portable ventilator can be avoided. As such, in some embodiments, portable ventilators are provided with pneumatic manifolds including limited tubing or even, in some embodiments, no tubing at all.

In some embodiments, the channeling may include channels not just within the pneumatic manifold (and partially within the pneumatic manifold, in cases in which a channel is formed partially within the pneumatic manifold and partially within or by a coupled component, for example), but also channeling formed between the pneumatic manifold portions or components (e.g., in some embodiments, channeling that runs between the exhalation valve pressure regulator and the air characterizer for use in exhalation valve control via pressurization). Channeling may include relatively large, wide channels in an exhalation limb and an inhalation limb of a pneumatic manifold. In the case of the exhalation limb, this may include channeling that allows gas to flow into the exhale port, through a portion of the pneumatic manifold, and out of the exhaust port to ambient air. In the case of the inhalation limb, this may include channeling that allows gas to flow into the intake port, through a portion of the pneumatic manifold, and out of the inhale port into the breathing circuit leading to the patient. Channeling may also include a number of smaller, narrower channels (e.g., exhalation valve control channeling and patient airway measurement channeling).

Although embodiments are described herein that include a pneumatic manifold that includes a blower-based component, an exhalation valve pressure regulator and an air characterizer (or portions thereof), some embodiments include variations with regard to these components or portions. For example, in some embodiments, some or all of these components or portions may be excluded or combined, or some or all of their functions may be combined, distributed differently, or omitted, or the pneumatic manifold may include one or more other components or functions.

Some embodiments provide a portable ventilator that allows for single-handed operation by an operator. This can be especially advantageous, for example, in various out-of-hospital settings. In some settings, it may be an advantage for the operator to operate the portable ventilator with one hand, including grasping and positioning the portable ventilator, such as by a handle, and operating controls of the portable ventilator where the control/user interface is proximately and conveniently located relative to the handle for such single-handed operation, while having the other hand available for other activities or uses. The single-handed operation may include an ability of the operator to grasp and control the position of the portable ventilator, which may, for example, include supporting at least a portion of its weight, using one or more grasping fingers of a single hand. The single-handed operation may further include an ability of the operator to operate one or more first controls of an interface of the portable ventilator to make a first selection relating to at least one ventilation related setting using one or more first fingers of the single hand. The single-handed operation may further include an ability of the operator to operate one or more second controls of the interface to make a second selection relating to at least one second ventilation related setting using one or more second fingers of the single hand. In some embodiments, the single-handed operation may include the ability of the operator to simultaneously operate the one or more first controls and the one or more second controls, while grasping the portable ventilator, such as by grasping a graspable structure or handle of the portable ventilator. The one or more first fingers, the one or more grasping fingers, and the one or more second fingers may be different from each other. In various embodiments of such operation, the thumb may be included as one of the fingers of the hand.

Some embodiments provide a portable ventilator with a gas mover/gas flow generator, such as a blower or centrifugal blower, which may be part of a blower-based component or portion of the portable ventilator, and a controller. The portable ventilator may be capable of operating, in providing mechanical ventilation to a patient, without requiring a valve to regulate, restrict or divert a portion of gas flow that is delivered to the patient. Such diversion might otherwise be required, for example, to divert a determined and variable portion or proportion of gas flow away from the patient in order to rapidly increase or decrease flow rate or pressure of gas being delivered to the patient. This can provide advantages, and solutions to associated technical problems, such as greater simplicity, greater energy efficiency, less oxygen use/waste and lighter weight, which can be especially important, for example, in connection with portability and maximizing practicality and ease of carrying and portable use of the portable ventilator.

Furthermore, some embodiments provide for control of the blower by the controller (or provide a controller that implements such control) for such operation without a valve as described. The control may be implemented by a controller using or executing, for example, software, programming, modeling and/or algorithms of embodiments described herein, which may be, in whole or in part, stored in a memory of the controller and/or elsewhere.

Some embodiments include a centrifugal blower. In some embodiments, the centrifugal blower includes a low moment of inertia impeller which allows for high acceleration of the blower to quickly reach its maximum speed. The blower, along with appropriate control, can facilitate allowing for sufficiently rapid increases and decreases in flow rate and pressure for gas being delivered to the patient without the need for a valve as described to divert a particular portion of the gas flow away from the patient.

Some embodiments provide a portable ventilator including a controller which, based at least in part on a target flow rate and/or a target pressure for gas being delivered to a patient, determines signaling to actuate a blower motor of a blower of the portable ventilator so as to cause a flow rate and/or a pressure of gas being delivered to the patient to move toward the target flow rate and/or the target pressure. In some embodiments, all or substantially all gas flow generated by the blower is delivered to an inhalation limb of a breathing circuit coupled with the portable ventilator to reach the patient. Furthermore, in some embodiments, no gas flow, or substantially no gas flow, generated by the blower is exhausted from the portable ventilator before being delivered to the inhalation limb of the breathing circuit to reach the patient. Some embodiments do not require use of a valve to regulate or restrict a portion of gas flow generated by the blower that is delivered to the inhalation limb of the portable ventilator to reach the patient. In some embodiments, the controller determines the signaling for actuating the blower motor to correspond with an appropriate blower motor torque.

In some embodiments, the blower is actuated to cause the pressure of gas being delivered to the patient to move toward the target pressure while maintaining the flow rate at or below a maximum permissible flow rate, or to cause the flow rate to move toward the target flow rate while maintaining the pressure at or below a maximum permissible pressure. Some embodiments provide for algorithmic single loop blower control. For example, the single loop blower control may include determining an appropriate blower motor torque and corresponding blower motor actuation signal based at least in part on a flow rate target and/or a pressure target for gas being delivered to the patient without, for example, an intermediary step of determining an appropriate blower motor speed target.

In various embodiments, a pneumatic manifold of a portable ventilator may be rigid and include portions (of the pneumatic manifold) that include (or include portions of) a blower-based component of the portable ventilator, an exhalation valve pressure regulator and an air characterizer, examples of which are described herein. In some embodiments, portions of the pneumatic manifold (which may include the blower-based component, the exhalation valve pressure regulator and the air characterizer, or portions thereof) may be fixedly joined together so as to form continuous gas path channels, such as may run between portions of the pneumatic manifold. In some embodiments, each of the blower-based component, the exhalation valve pressure regulator and the air characterizer may be or include a rigid portion that forms a portion of the pneumatic manifold. However, in some embodiments, each may also include other portions that do not form part of some embodiments of the pneumatic manifold (e.g., in some embodiments, an impeller of a blower-based component, for example). Additionally, other components of a portable ventilator may be coupled with portions of the pneumatic manifold (e.g., one or more electrical circuit boards may be coupled with the air characterizer, examples of which are described herein). In other embodiments, the pneumatic manifold may include various such components.

Reference throughout the specification to "one embodiment" or "an embodiment" means that a particular feature, structure, or characteristic described in connection with an embodiment is included in at least one embodiment of the subject matter disclosed. Thus, the appearance of the phrases "in one embodiment" or "in an embodiment" in various places throughout the specification is not necessarily referring to the same embodiment. Further, the particular features, structures or characteristics may be combined in any suitable manner in one or more embodiments. Further, it is intended that embodiments of the disclosed subject matter cover modifications and variations thereof.

As used in the specification and the appended claims, the singular forms "a," "an," and "the" include plural referents unless the context expressly dictates otherwise. That is, unless expressly specified otherwise, as used herein the words "a," "an," "the," and the like carry the meaning of "one or more." Additionally, it is to be understood that terms such as "left," "right," "top," "bottom," "front," "rear," "side," "height," "length," "width," "upper," "lower," "interior," "exterior," "inner," "outer," and the like that may be used herein merely describe points of reference and do not necessarily limit embodiments of the present disclosure to any particular orientation or configuration. Furthermore, terms such as "first," "second," "third," etc., merely identify one of a number of portions, components, steps, operations, functions, and/or points of reference as disclosed herein, and likewise do not necessarily limit embodiments of the present disclosure to any particular configuration or orientation.

Furthermore, the terms "approximately," "about," "proximate," "minor variation," and similar terms generally refer to ranges that include the identified value within a margin of 20%, 10% or 5% in certain embodiments, and any values therebetween.

All of the functionalities described in connection with one embodiment are intended to be applicable to the additional embodiments described below except where expressly stated or where the feature or function is incompatible with the additional embodiments. For example, where a given feature or function is expressly described in connection with one embodiment but not expressly mentioned in connection with an alternative embodiment, it should be understood that the inventors intend that that feature or function may be deployed, utilized or implemented in connection with the alternative embodiment unless the feature or function is incompatible with the alternative embodiment.

In some instances, variations of a term may be utilized that may refer to the same or similar concepts, and certain terms may have meanings that are informed by a particular context. Generally, sending, receiving, or transmitting of data may include by wired and/or wireless connection, and/or within one or more wired or wireless networks. Furthermore, sending from a first entity to a second entity, or to be received by the second entity, can include sending from the first entity to the second entity, or to be received by the second entity, directly from the first entity to the second entity, or indirectly via one or more intermediary entities. Herein, air may include any gas, including but not limited to ambient air, and can include provided gas.

Some embodiments include providing mechanical ventilation in connection with, for example, a patient experiencing respiratory distress. Respiratory distress may include, for example, any form of respiratory or breathing difficulty, impairment or problem, including respiratory failure. Respiratory parameter data can include data relating to respiratory, pulmonary or lung related parameters, as may, for example, be associated with respiratory, pulmonary or lung related characteristics, attributes or functions. Respiratory parameter data can include, for example, data related to pulmonary associated pressure, flow, volume or capacity related parameters, including parameters that may be measured using one or more flow sensors, pneumotachometers or spirometers. Respiratory parameter data can include, for example, data relating to respiratory mechanics, such as respiratory compliance (Crs), respiratory elastance (E) and respiratory resistance (Rrs). Respiratory parameter data can also include, for example, parameters such as vital capacity (VC), forced vital capacity (FVC), forced expiratory volume (FEV) at timed intervals (e.g., between 0.5 and 10 seconds, less than 0.5 second, 0.5 second, 1.0 second or FEV1, 2.0, 3.0 seconds, 4.0 seconds or 5.0 seconds), forced expiratory flow (FEF) at, e.g., 10%-90% capacity, such as 25%-75% or FEF25-75, peak expiratory flow rate (PEF or PEFR) and maximum breathing capacity (sometimes called maximal voluntary ventilation or MVV). Respiratory parameter data may be presented in various different ways or forms, such as may include, for example, in raw terms, such as liters or liters per second, or as percentages. Respiratory parameter data may also be presented, for example, as "predicted" values, such as percent predicted, which can, for example, include results as a percentage value in connection with a reference, average, or other "predicted" value. "Predicted" values may, in some cases, be associated with patients or hypothetical patients of one or more similar characteristics.

A respiratory status may, for example, relate to, identify, or indicate the presence or absence of, one or more respiratory, pulmonary or lung related conditions, and may include respiratory distress. A condition may include a state, disease, or problem, or several thereof, or a type, group or category thereof. A respiratory status may include an etiology relating to a respiratory condition or a non-respiratory condition (e.g., respiratory distress associated with acute heart failure). A non-respiratory condition may, for example, be associated with one or more non-respiratory systems, e.g., cardiac, endocrine, exocrine, circulatory, immune, lymphatic, nervous, muscular, renal, skeletal, or others. Additionally, a respiratory status may include one or more determined, assessed, estimated, probable or possible conditions, or determined, assessed, probable or possible associated etiologies. A respiratory status may also include data associated any of the foregoing, which may be called respiratory status data.

Ventilators, such as portable ventilators, may include, for example, devices or systems capable of delivering ventilation, whether such delivered ventilation is controlled internally, remotely, or with aspects of both.

Various ventilation parameter related terms or abbreviations, including fraction of inspired oxygen (FiO2), positive end-expiratory pressure (PEEP) and others, refer to ventilation related settings, even though the word "setting" may or may not be stated. Furthermore, reference to a ventilation parameter or parameter setting may be used to refer to the parameter in a conceptual or definitional sense, or the value associated with a particular setting (e.g., "PEEP of 5 cm H2O"). A user may include an individual operating, supervising or in whole or in part responsible for operation of a device such as a portable ventilator, even if, during a particular period of time while the device is operating, the user may not be interacting with the device.

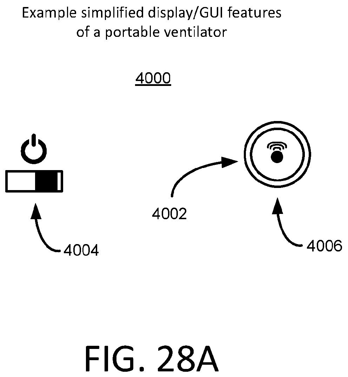

Ventilation related settings may include, for example, any setting, such as a current, selected, set or entered ventilation parameter, parameter value, or other setting relating to ventilation, any aspect of ventilation, operation of a ventilator, such as a portable ventilator, in association with providing or provided ventilation, including mechanical ventilation. Ventilation related settings may include, for example, settings related to a mode or form of ventilation, power, communication, network connection, remote or internal control, ventilation parameters such as FiO2, PEEP (or baseline airway pressure (BAP)), closed loop control (CLC), etc., among other things. Ventilation related settings may include, for example, various of the displayed or controlled parameters shown in FIGs. 28A-C, among other things.

In one or more examples, the provision for single-handed control of the one or more first buttons or dials or first controls, which may be configured to be operable to allow the making of a first selection relating to the at least one ventilation related setting, is advantageous. For example, the user may require their free hand, during ventilation, for maintaining a mask on the patient while pressure and/or flow measurements are taken in response to operation of the one or more first buttons or dials or first controls, which may allow or improve the accuracy of those measurements. In other examples, the user's free hand may be required to measure the pulse rate or other vital signs at the time the ventilation related setting is changed, e.g., to more effectively gauge the patient's response to the change in the ventilation related setting.

Additionally, during ventilation, the user may require or advantageously use their free hand to manually grasp and/or apply appropriately directed light pressure or pressing to ensure a good seal between the mask and the patient's face, such as while also ensuring that the patient has the correct head tilt and jaw thrust orientation. For example, with the mask on the patient's face, the user may grasp a portion of the mask with the thumb and index finger of the free hand while also grasping the patient's face around the jaw line with the other fingers of the free hand, in order to accomplish the mentioned objectives.

Furthermore, the user's fee hand may be useful or needed during movement of the patient. For example, while the patient is moved, the user's free hand can be used to grasp and maintain stability of the patient's artificial airway, including portions of the patient circuit, such as to ensure that there is no pull on any endotracheal tube and that it does not become dislodged during movement. For example, during patient movement, sometimes a ventilator might need to be disconnected to avoid dislodgment. However, use of a portable ventilator, as described in embodiments described, may provide a solution to this problem, since the user may use their free hand to make physical adjustments as may be needed to ensure that the portable ventilator stays properly connected, thus allowing the portable ventilator to stay connected during patient movement. Additionally, during the patient move, the user may use their free hand to physically support aspects of the move. Notably, during the move, for example, the user may simultaneously operate the portable ventilator with their other hand, including potentially operating controls thereof, such as to make necessary operation adjustments.

Still further, the user may use their free hand for other tasks, such as may be necessary or allow optimizing of treatment. For example, the user may use their free hand to secure or hold another device or piece of equipment, such as a therapeutic or diagnostic device (e.g., a pulse oximeter, which may be placed or adjusted on the patient), or a computing device (e.g., a smart phone, tablet, laptop computer or monitor). Also, in high vibration environments (e.g., during transport or in the presence of vibrating equipment or potential disruptions such as in a military or disaster setting), the free hand may be used to implement fine or delicate adjustments that may be needed due to shifting that may occur in such environments.

An alert or alarm may be presented for the attention of a user, such as by being visually or audibly presented, such as via a display, graphical user interface (GUI) or speaker of a device, and may or may not be included in context sensitive guidance (CSG). However, an alert or alarm may also include an alert or alarm condition that is algorithmically identified, recognized or determined by a computerized device, and not necessarily presented or displayed. The term optimizing may include, for example, improvement or improved operation in one or more aspects, for example, relative to an actual, potential or hypothetical less optimized situation or less optimized operation. The term adjusting can include changing as well as not changing or maintaining without change, as may be appropriate. A determined parameter value can include a determined estimated or determined approximated value for the parameter. The term monitoring can refer to or include, for example, monitoring or tracking performed by a computerized device utilizing one or more algorithms and not by a person or user, or monitoring by a person or user, or both. The term continuous can include, among other things, on a periodic basis (with identical or different periods), on a frequent basis, on a repeated basis, or cyclically, for example.

In some embodiments, a controller of a ventilator internally signals a mechanical ventilation system (or apparatus) of the ventilator in order to implement control thereof, whether such internal signaling implements internal control or remote control. Internal control includes a controller of a portable ventilator internally signaling the mechanical ventilation system of the portable ventilator to implement control according to a setting value (or more than one setting value) that the controller determines, even if the determined setting value is determined based at least in part on a setting value of a received request/command. Remote control includes the controller of the portable ventilator internally signaling the mechanical ventilation system of the portable ventilator according to a setting value received by the controller via a request/command from a remote control device or system.

In some embodiments, a ventilator may be capable of either internal or remote control, or operation with aspects of both, and may be capable of determining which, or what particular state or mode, is employed or used at a predetermined or particular time. Furthermore, a portable ventilator may be capable of switching between modes or states of operation, such as may include an internal control mode, a remote control mode, or a mode that includes aspects of both.

A controller can include physical aspects such as one or more processors and one or more memories, as well as software based aspects, which may include one or more programs, algorithms or software based aspects. The software based aspects may, for example, be stored, in the one or more memories of the controller, accessed by the one or more processors of the controller and used or executed by the processor.

The term controls may include physical controls, display/GUI based controls, or both. Controls may, for example, allow user interaction with a device or system, such as may affect operation of the device or system, which may include, for example, making selections or providing input to set, select, change, increase, decrease, confirm or override implementation of a parameter setting value or a mode. A device or system with controls may, in various aspects, operate without user interaction, with user interaction, or with aspects of both. This may include operation that may proceed, in various aspects or instances, without user interaction, but may permit of, or require, user interaction in some aspects or instances. A display/GUI may include controls, and/or controls may include a display/GUI.

In some embodiments, a set of controls, such as of a particular device, may include one or more respiratory aspects. Respiratory aspects of controls may include, for example, display/GUI aspects relating to any aspects of ventilation, ventilation parameters or aspects relating to respiration, respiratory distress or respiratory parameters. Respiratory aspects of controls may or may not include CSG.

Herein, remote control may include control, or aspects of control, provided from outside of, or separately from, a controlled device or system, such as by wired or wireless signaling or communication, such as may include, for example, Bluetooth or ultrawide band. As such, remote control may be implemented via a wired only connection that connects a remote control device and a remotely controlled device, or by a wireless only connection, or by a combination of wired connection and wireless connection, for example. Remote control, such as remote control by a remote control device or system, may include control with or without aspects of user interaction with the remote control device or system, such as, for example, by the user using controls of the remote control device or system. Internal control, such as a device internally controlling itself, may or may not include aspects of user interaction with the internally controlled device, such as, for example, by the user using controls of the internally controlled device. User interaction may include, for example, a user using controls to input, set, select, change, increase, decrease, confirm or override implementation of a parameter setting value or a mode. User interaction may or may not be prompted, guided, or interactively guided, e.g., via CSG, one or more GUI based prompt messages, alarms or alerts that may relate, for example, to patient parameters or ventilation parameters, such as may relate to ventilator operation, performance or components, such as sensors.

Any of various types of gas movers / gas flow generators may be used in various embodiments of a portable ventilator, including blowers, compressors, compressor-based and turbomachinery. In some embodiments shown herein, centrifugal blowers are included (or used). Herein, an electronic circuit board may include a printed circuit board (PCB) or boards.

In some embodiments, a ventilator, such as a portable ventilator, is configured for integration with one or more other devices or systems, such as onsite or offsite remote devices, systems or interfaces, aspects of which may or may not be included in various embodiments. For example, in some embodiments, the ventilator may couple, in a wired and/or wireless fashion, with a device such as a patient monitor or critical care monitor (CCM). In some embodiments, for example, a defibrillator, portable ventilator, or other device, such as another medical device, monitoring device or computing device, may be, include or function as a CCM. Furthermore, in some embodiments, the ventilator may couple with one or more computing systems, computers, computing devices or portable computers, which can include, for example, a cloud-basing computing system, computer, notebook computer, tablet, touch-based device, smartphone, wearable device or implantable device, among other things.

In some embodiments, smaller size, lighter weight and/or greater simplicity may be desirable or optimized in a portable ventilator, as well as simpler, easier or more efficient or portable use. Optimization may take into account various factors such as the actual, predicted, likely or anticipated setting, context, environment or application, such as pre-hospital contexts. Optimization may also take into account allocation of roles of various devices in such contexts. Some embodiments provide a portable ventilator that is optimized in various ways, as well as related apparatuses, systems and methods. The portable ventilator may be smaller and/or lighter than various other ventilation devices or systems. Furthermore, the portable ventilator may be optimized for users with limited training or experience. Still further, the respiratory portable ventilator may be optimized for use in environments that may include other integrated devices or systems. Additionally, some embodiments provide a system of devices, including a portable ventilator, in which each device and the system as a whole are optimized and capable of optimized operational integration, in view of such factors and potentially others.

For example, the space immediately surrounding a critical care patient can be very crowded with equipment or items such as, for example, hoses, tubes, wires and various devices. It can be beneficial or advantageous to minimize, for example, the spatial impact of a portable ventilator, as well as to optimize aspects of its portability and portable use. In some embodiments, for example, a portable ventilator may provide advantages by allowing modularization of, or allowing a higher degree of modularization of, a system including a portable ventilator, which may allow for a much smaller, simpler portable ventilator close to the patient. Furthermore, in some embodiments, the controls in or of a remote control device or system may be some variable distance from the patient (e.g. between 0-3 feet, between 3-6 feet, between 6-9 feet, between 9-12 feet, further than 12 feet, or offsite). Some embodiments provide benefits or advantages to patient care by, for example, simplifying the immediate clinical environment of the patient. For example, in some embodiments, even if the combined weight and/or size of a combination of a portable ventilator and different remote control device, together, are the same or similar to a system in which the controls are provided in or with the portable ventilator, the combination of the ventilator and the different remote control device may be more usable, or more practically or optimally useable, in various clinical contexts, for example, since only a relatively small ventilator may be required to be at the patient's side or very close to the patient. Some embodiments provide portable ventilators that provide these advantages.

In some embodiments, an overall environment may include a portable ventilator that may couple and operationally integrate with one or more other devices, each of which may provide certain roles or functionality in the overall environment. These roles may relate to, for example, device and patient related sensing, operation, processing, software, algorithms, applications, data storage, communication, integration (of devices and systems, roles, functions, and physical, software based and conceptual components and aspects), user interaction and guidance, and patient related assessments or interventions. Within the integrated environment, roles of each device may be optimized, taking into account, for example, factors relating to each device or system as well as the overall environment and anticipated settings. Such optimization may also take into account factors relating to the need for the portable ventilator to be of a particular or sufficiently small size, light weight, and/or degree of simplicity or ease of portability or use. In some embodiments, a portable ventilator augments the ability to accurately assess or screen a patient's condition, to determine or select, and initiate and deliver, an effective intervention, and/or to ongoingly control or adjust, or assist in control and adjustment of, parameters relating to an applied intervention. Furthermore, this augmentation may be optimized in view of the overall environment, available devices, systems or users at a given time or time period, and available communication between them at a given time or time period, as well as changing aspects thereof over time.

In some embodiments, for example, one or more devices or systems coupled with a portable ventilator, or integrated into the environment thereof, may supply, or partially supply, components or other aspects that may augment or enhance the operation of the portable ventilator, and/or otherwise might be, or need to be, included with or in the portable ventilator. Some such aspects may include physiological and operational signaling, sensing or measuring aspects. Other aspects may relate to power, communication, device recognition, authentication or coupling. Other aspects may relate to processing, hardware and software, such as for data processing and management, data and device integration, or operational aspects such as CLC of patient oxygenation or other ventilation related parameters or settings. Other aspects may related to patient respiratory or status assessment, diagnostic screening, diagnosis, treatment or intervention. Other aspects may include controls and control relating to the portable ventilator (physical, display/GUI based, or both), as well as features thereof. Still other aspects may include determination and/or presentation of context sensitive guidance (CSG).

In some embodiments, a portable ventilator may have a set of (one or more) included controls, while one or more remote control devices, systems or monitors may also have a set of (one or more) remote controls relating to the portable ventilator. A set of remote controls may for allow remote control of the portable ventilator, such as may relate, for example, to aspects of operation of a mechanical ventilation system of the portable ventilator. In various embodiments, a set of included controls of portable ventilator may vary across a spectrum of possibilities.

In various embodiments, a set of remote controls may include respiratory aspects that are separate from other aspects, or may include respiratory aspects that are mixed, combined or integrated, for example, into a larger display/GUI or set of controls that may include non-respiratory aspects as well. Furthermore, in various embodiments, a set of remote controls may be separate or separable from other aspects of controls, or may be integrated with other aspects. In some embodiments, the portable ventilator may have no included controls at all, or may have a limited set of included controls. For example, any or many controls relating to the portable ventilator may be provided by one or more sets of remote controls of one or more other devices, systems or monitors. In some embodiments, a set of remote controls, or a portion thereof, may, in whole or in part, correspond to, emulate, mirror, duplicate, replicate, simulate, or be in any of varying degrees similar to a set of included controls of the portable ventilator. Furthermore, in some cases in which the portable ventilator has no controls or a limited set of controls, a set of remote controls may, to some degree, be similar to or replicate a hypothetical set of included controls that might be included in a portable ventilator.

As such, in various embodiments, a set of remote controls may be the same, may replicate, may be similar to some degree, or may be different than a set of included controls. Furthermore, various particular individual controls or aspects of the two sets may include ones that are the same or different, or have the same or different scopes, and which may overlap, partially overlap or not overlap. In various embodiments, the set of included controls may be, in at least some aspects, more limited or more simplified than the set of remote controls, or vice versa. For example, a set of included controls may or may not be, or include, in whole or in part, a subset of the set of remote controls, or vice versa. The set of remote controls may include individual controls or aspects that are, or are not, similar or identical to, reflect or mirror individual controls or aspects of the included controls. The set of remote controls may provide visual or GUI features or information relating to, or allowing control of, some or all of the aspects, modes, settings or other parameters that are reflected by or incorporated into the included controls.

In some embodiments, curating, selecting or limiting the set of included controls of a portable ventilator, such as to an optimized degree, may provide various advantages. Such advantages may include, for example, ease or range of portability (which may include being lightweight, being of small size or having a small footprint), practicality, ease or range of usability, simplicity of control, simplicity of user interaction, or simplicity of operation.

In some embodiments, the set of included controls of the portable ventilator may be optimized, for example, taking into account factors such as actual, anticipated or likely environments or settings, which can include considerations relating to locations, situations and use case scenarios. Other factors may relate to users and their training, experience and potential or likely degree of availability or distraction at a scene. Other factors may relate to patients or types of patients. Still other factors may relate to devices, systems, communication, reliability or speed of communication, or data and data management aspects.

The set of remote controls of a remote control device may also be optimized, including taking into account the foregoing factors, as they apply to the remote control device, as well as other factors. For example, the set of remote controls may include a display/GUI, that is, to a selected or optimized degree, more inclusive, larger, broader in scope, more comprehensive, more sophisticated, more complex and/or more granular than the set of included controls of the portable ventilator. In some embodiments in which the set of remote controls is, for example, more inclusive or comprehensive than the set of included controls of the portable ventilator, control by or using the set of remote controls, or by the associated remote control device, may allow various advantages. These advantages may include, for example, greater, additional, more sophisticated, more complex, or more granular control, aspects of control, user based control, user interactivity, or user based control with greater available display/GUI. The greater available display/GUI may include, for example, information such as parameter values, alarms, alerts, user messages or CSG and user interactivity features.

In some embodiments, a portable ventilator may be capable of being remotely controlled, or may be capable of both remote control or internal control, or aspects of both, such as may include use of a controller of the portable ventilator. In some embodiments, for example, a portable ventilator may be capable of determining whether to enable and use remote or internal control, or aspects of both, and may be capable of switching between remote and internal control modes, or modes that include aspects of both. For example, in some embodiments, remote control, or aspects thereof, may be generally preferred when available or optimal, and internal control may be used only when external control, or aspects thereof, is determined to be unavailable, unsafe, not practical, or not optimal relative to internal control. In various embodiments, remote control may be determined to be unavailable or not optimal if, for example, there is no or insufficient communication with the remote control device or system, or other problems exist such as may relate to effective, optimal or safe control or operation of the portable ventilator by the remote control or by particular remote control requests/commands.

In some embodiments, remote control may include more complex or sophisticated forms or aspects of ventilation or ventilation control, such as may, for example, include CLC aspects, while internal control may include less complex or sophisticated control, such as continued use of current parameter values without adjustment, or use of default or backup parameter values. Furthermore, in some embodiments, a portable ventilator may include a controller that monitors and allows remote control within certain limits, parameters, or setting value ranges, but may override external control and utilize internal control if and when needed to maintain required setting ranges, for example.

In some embodiments, a portable ventilator, or the controller thereof, may be configured to allow remote control at times, when a fault mode condition is determined not to exist. In some embodiments, a fault mode condition may include a condition that warrants utilizing internal control, or aspects thereof, as opposed to enabling or allowing remote control. In some embodiments, lack of, or insufficient, communication capability between the portable ventilator and remote control device or system may constitute a fault mode condition. For example, if such communication is determined not to exist (or be lost), or be insufficient or insufficiently reliable, the portable ventilator may use (or switch to) internal control (or an internal control mode). The internal control mode may effectively operate as a back-up mode or default mode in the event that control by the remote control device is or becomes unavailable or does not meet particular required conditions. In some embodiments, fault mode conditions may include conditions relating to the remote control device or data received from it, such as may relate, for example, to device configuration or permissions, communication, data integrity, ventilator settings or alarm conditions.

In some embodiments, ventilation delivered by a portable ventilator may include use of various forms of CLC in ventilation. In some embodiments, such CLC may be available when remote control is available, when particular physiological sensing capability is available, or both. While the portable ventilator may deliver the ventilation, control of aspects of the delivered ventilation may be from a remote source, and the remote source may employ control aspects or features that may include or incorporate forms or aspects of CLC. Forms of CLC that may be available may include CLC relating to fraction of inspired oxygen (FiO2), which may be driven at least in part by measured patient oxygen saturation or SpO2. Forms of CLC may also include CLC relating to positive end-expiratory pressure (PEEP), which may, for example, be driven by current FiO2 as well as a current PEEP. This may include associated PEEP change eligibility rules and PEEP selection rules. For example, in some embodiments, a portable ventilator, and/or one or more remote control sources, may each include a controller with CLC capability (e.g. of FiO2, PEEP or both, and/or other parameters). As such, in various embodiments, a controller of a portable ventilator may be capable of internal control including CLC, or of implementing remote control that includes CLC, or both. In some embodiments, a portable ventilator that is capable of internal control or of being remote controlled may only provide mechanical ventilation including CLC capability, or some forms of CLC capability, when being remote controlled.

In some embodiments, a portable ventilator may include one or more sensors, flow sensors, pneumotachometers or spirometers that may be coupled with inspiratory or expiratory patient circuits or patient circuit limbs of the portable ventilator, which may be capable of sensing signals that can be used in determining at least one patient respiratory parameter. This, in turn, may be used in patient respiratory status determination, screening or diagnosis, or in determining an appropriate intervention. For example, included flow sensors or pneumotachometers may be used both in connection with sensing and measurement associated with a patient's respiratory function as well as in connection with portable ventilator performance and operation.

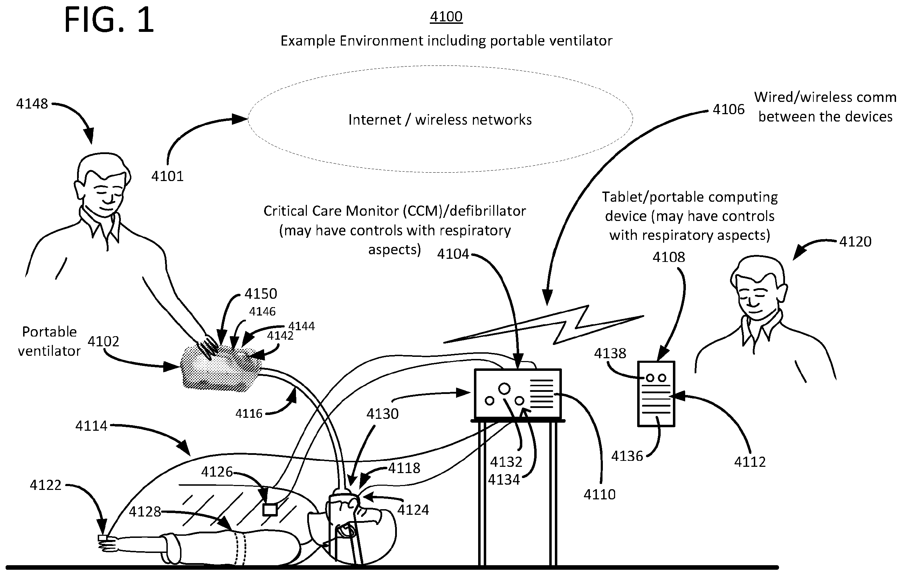

FIG. 1 illustrates an example emergency care environment 4100 including an example portable ventilator 4102, a CCM, such as a CCM/defibrillator 4104, and a tablet 4112. The various devices and systems may be communicatively coupled by wired and/or wireless connection. In various embodiments, in addition to a portable ventilator, many different combinations of devices and systems, and roles for each, may be used. In some embodiments, a portable ventilator may be capable of being remote controlled by one or more of other depicted devices or systems, which may include, for example, the CCM/defibrillator, defibrillator, medical or monitoring device, computing device, portable computing device, tablet, smartphone, or offsite person(s), monitor, system, facility, medical care or monitoring facility, hospital, or other group or entity. In various embodiments, the portable ventilator may be internally controllable or remotely controllable, and may be able switch between internal and remote control modes.

In various embodiments, particular sensing capabilities and components may be distributed in various ways between the various devices or systems in an environment. These may include, for example, one or more electrodes, capnographic sensors, pulse oximeters, flow sensors, pneumotachometers, spirometers, pressure sensors, barometric sensors, humidity sensors, oxygen sensors, temperature sensors, electrical or magnetic sensors, light/electromagnetic spectrum/optical sensors, blood pressure monitors, heart rate monitors, electrocardiogram (ECG) sensors and others. Sensing capabilities and components may relate, for example, to patient parameters and various patient systems, such as respiratory and circulatory systems. Sensing capabilities and components may also relate to parameters associated with devices and their operation, including a portable ventilator and ventilation, or a defibrillator and electrotherapy, among other things. In some embodiments, any of various sensing capabilities, such as those of a portable ventilator, CCM/defibrillator or tablet, may instead be provided by one or more other devices, or may be distributed between multiple devices. Also, in various embodiments, various sensing components, or all or part of a patient circuit, may or may not be considered to be part of a portable ventilator, or other device, even if they are connected thereto. Furthermore, in various embodiments, roles and functions of the various devices may be distributed differently between the devices.

In FIG. 1, a user 4148 is shown, who is operating the portable ventilator 4102, including grasping it by its handle 4150. Another user 4120 is also shown, which user 4120, and/or one or more other users, may monitor or interact, for example, with the patient 4114, the portable ventilator 4102, the CCM/defibrillator 4104 and/or the tablet 4108. In the embodiment depicted, the devices 4102, 4104, 4108 are coupled with each other by wired and/or wireless connection 4106, such as including over the Internet and/or one or more wireless networks 4101. However, in some implementations, at various times, such connection might be unavailable, lost, limited, or of differing speeds. Furthermore, their operation may be integrated in various ways. In various embodiments, devices, including the portable ventilator 4102, may have housings that may be separate and spaced from each other, that are adjacent or touching, or that are or may be physically or mechanically coupled, connected or attached (fixedly or movably).

When delivering mechanical ventilation, the portable ventilator 4102 may provide breathing gas to the patient 4114 via a mechanical ventilation apparatus including a gas flow generator, such as a blower, and a gas delivery apparatus including a patient circuit 4116 that includes a facemask 4118. However, in some embodiments, ventilation may be provided via intubation or other patient interface such as nasal cannula rather than via a facemask 4118.

In the example depicted in FIG. 1, the CCM/defibrillator 4104 includes a pulse oximeter 4122 for measuring patient SpO2, a capnographic sensor 4124, which may be used in measuring EtCO2, a blood pressure sensor/monitor 4128, electrodes 4126 that may be used in providing electrotherapy to the patient 4114, and may include various other components such as one or more flow sensors, pressure sensors, airway sensors or spirometers. The portable ventilator 4102, CCM/defibrillator 4104, tablet 4108 and other devices may also include various sensing, measuring, computerized, electrical, mechanical, coupling and output aspects or components, including power supplies and batteries.

In the example depicted in FIG. 1, the portable ventilator 4102 may or may not be coupled with a supplemental oxygen (O2) source (not depicted). In various embodiments, the portable ventilator 4102 may be capable of supplying oxygen, using the supplemental oxygen source, in a number of different ways, or the portable ventilator 4102 may be capable of supplying oxygen in any one of several different ways. In various embodiments, the manner in which oxygen is supplied may be determined without user selection, or may be selected by a user, and may or may not require user confirmation.

In some embodiments, a reservoir bag may be used that allows entrainment of oxygen from a low pressure oxygen source. For example, a user may adjust the flow rate of oxygen based at least in part on current SpO2, which may be monitored by one or more of the devices 4102, 4104, 4108. In the embodiment depicted in FIG. 1, the CCM/defibrillator 4104 continuously monitors SpO2. In various embodiments, current SpO2 may be displayed for viewing by the user via a display/GUI of one or more of the devices 4102, 4104, 4108.

In some embodiments, the portable ventilator 4102 includes and uses a variable rate regulatory valve, in which an oxygen output rate allowed or facilitated by the variable rate regulatory valve may be varied and changed to a particular oxygen flow rate of a range of possible oxygen flow rates, for example, from 0 l/min to 200 L/min (or, e.g., up to 220, 250 or 275 L/min). The variable rate regulatory valve 4may be used in providing a variable and controllable oxygen flow rate for gas provided by the portable ventilator 4102 during the providing of mechanical ventilation. In the embodiment depicted in FIG. 1, the variable output regulatory valve may attach to the portable ventilator 4102, a high pressure oxygen source, or the CCM/defibrillator 4104 (which monitor SpO2), for example.

In some embodiments, the variable rate regulatory valve may be controlled automatically or without need for user interaction. For example, this may be based at least in part on the patient's continuously monitored SpO2 and in accordance with an FiO2 setting or adjustment that may be determined based at least in part on the current SpO2. In some embodiments, this arrangement may be used in providing CLC of FiO2 (FiO2 CLC). In some embodiments, a portable oxygen concentrator (POC) may be used. FiO2 CLC may be used in controlling and regulating the output of the POC for entrainment of oxygen into the portable ventilator 4102 to be used in gas delivered by the portable ventilator 4102 during mechanical ventilation. Furthermore, in some embodiments, including embodiments in which the variable output regulatory valve or a POC is used, CLC of PEEP (PEEP CLC) may also be included in control of the portable ventilator 4104. In some embodiments, PEEP CLC may be based least in part on a current FiO2 setting and a current PEEP setting. In various embodiments, control with FiO2 CLC and/or PEEP CLC may be provided internally by the portable ventilator 4102 or by a remote control device for the portable ventilator 4102, such as the tablet 4108 or the CCM/defibrillator 4104.

In some embodiments, the portable ventilator 4102 may operate, or may have one or more modes of operation, that do not include, or at times or during certain periods of time do not include, delivering mechanical ventilation. Furthermore, the portable ventilator 4102 may include operation, or modes of operation, for use outside of an emergency care context. For example, in some embodiments, the portable ventilator 4102 may include and/or may be coupled with one or more spirometers that can be used for patient respiratory assessment outside of an emergency care situation. For example, such assessment may be performed on patients or individuals that may not be presently receiving mechanical ventilation, may not require mechanical ventilation, and may not be presently experiencing respiratory distress. In some embodiments, such assessments may include, for example, assessing and measuring the change in one or more respiratory parameter values (e.g., FEV1 or FVC) before and after some change of circumstances or procedure, such as, for example, bronchodilator treatment, or respiratory parameter and respiratory status assessments on a patient during a routine, scheduled or other doctor's office, or medical facility or hospital. Furthermore, some assessments may be made while the patient is be laying, sitting or standing, and while the patient is not experiencing respiratory distress.

In the embodiment depicted in FIG. 1, the portable ventilator 4102 includes sensors 4130, such as one or more flow sensors, pneumotachometers or pressure sensors, which may include sensors disposed within the patient circuit 4116 and within the portable ventilator 4102, for sensing signals representative of gas flow or pressure within the gas delivery apparatus of the portable ventilator 4102 and/or to the patient 4114. In some embodiments, one or more of the sensors 4130 may be coupled with or part of one or more spirometers, for example. In some embodiments, a controller of the portable ventilator 4102 receives the signals representative of the gas flow or pressure. The controller may control aspects of mechanical ventilation provided by the portable ventilator 4104 based at least in part on the signals representative of gas flow or pressure. Furthermore, in some embodiments, based at least in part on the signals representative of the gas flow or pressure, the controller may generate respiratory parameter data corresponding with at least one respiratory parameter of the patient, such as, for example, Crs, Rrs, VC, FVC, FEV at a timed interval such as FEV1, FEF, or FEF25-75, PEF, PEFR, MVV or others.

The controller may transmit the respiratory parameter data to be received (directly or via one or more intermediary entities) by, e.g., the tablet 4108, CCM/defibrillator 4104, or elsewhere, such as for use in determining a respiratory status of the patient. However, in some embodiments, the portable ventilator 4102 may determine, or participate in determining, the respiratory status of the patient.

In some embodiments, aspects of a system of devices including a portable ventilator 4102, such as distribution and integration of roles between the devices as well as aspects of particular devices, may be optimized based on factors that may include a desired degree of portability or ease of operation of the portable ventilator 4102, for example. Furthermore, aspects of individual devices may be optimized. Such aspects may include, for example, sets of controls, processing and memory aspects, software, algorithms, and CSG related aspects. In some embodiments, various capabilities may be included with the portable ventilator 4102, or included with one or more other devices or systems, or may be distributed or integrated between them. This may include, for example, processors, memories, controllers, or stored software or algorithms. These capabilities may be used, for example, in processing signals, generating respiratory parameter data or determining a respiratory status of the patient. They may also be used in performing coordination or integration between devices. As such, in this and other ways, the roles and processing capability and components of the portable ventilator 4102 may be greater or fewer, or determined or allocated, based at least in part on the roles of one or more other devices in an environment. In some embodiments, reducing or limiting the roles, processing capability or components of the portable ventilator 4102, as well as its included set of controls 4144, can increase or improve the portability or portable practicality of the portable ventilator 4102, such as by allowing reduced portable ventilator size, weight, complexity, bulk, footprint, or noise during operation, for example. This, in turn, can allow for various implementations that may be balanced or optimized for particular anticipated or likely scenarios, which can increase positive, potentially life-saving patient outcomes.

In some embodiments, for example, a portable ventilator may have a size of between 0.125 - 27 L, such as, e.g., between 5.0 - 27 L, between 1.0 - 5.0 L, between 0.5 - 1.0 L, between 0.15 - 0.5 L, or between 0.125 - 0.15 L . Furthermore, in some embodiments, a portable ventilator may have physical dimensions of between 50 x 50 x 50 mm - 300 x 300 x 300 mm, such as, e.g., between 50 x 50 x 50 mm - 60 x 60 x 60 mm, between 60 x 60 x 60 mm - 75 x 75 x 75 mm, between 75 x 75 x 75 mm - 100 x 100 x 100 mm, between 100 x 100 x 100 mm - 200 x 200 x 200 mm, or between 200 x 200 x 200 mm - 300 x 300 x 300 mm. Furthermore, in some embodiments, a portable ventilator may have a weight of between 0.2 - 5.0 kg, such as, e.g., between 1.0 - 2.0, between 0.3 - 1.0, or between 0.2 - 0.3 kg. Furthermore, in some embodiments, a portable ventilator may have a maximum noise (or noise during a particular operating mode) of 40 - 70 dB at 1 m, such as, e.g., between 40 - 50 dB at 1 m, between 50 - 60 dB at 1 m, or between 60 - 70 dB at 1 m. Furthermore, in some embodiments, a portable ventilator may have a battery life of between 2-10 hours, such as, e.g., between 2-4 hours, between 4-6 hours, between 6-8 hours, or between 8-10 hours. In some embodiments, the battery can be charged while the portable ventilator is operating.

In the embodiment depicted in FIG. 1, each of the portable ventilator 4102, the CCM/defibrillator 4104 and the tablet 4108 includes a set of one or more controls (which can be physical, display/GUI based, or both). Particularly, as depicted, the portable ventilator 4102 has a set of included controls 4144, which may include one or more physical controls 4146 (e.g., one or more physical switches, buttons, knobs, dials, etc.) and/or one or more GUI/display based controls 4142. However, in some embodiments, the set of included controls 4144 may include only one or more physical controls, only one or more display/GUI based controls, or no controls at all. The CCM/defibrillator 4104 (which may, in some embodiments, be an automated external defibrillator, or AED) has a set of CCM/defibrillator controls 4134, including one or more physical controls 4132 and one or more GUI/display based controls 4110. The tablet 4108 has a set of tablet controls 4112, including one or more physical controls 4138 and one or more GUI/display based controls 4136. In various embodiments, aspects of various sets of controls 4144, 4134, 4112 may be distributed in various ways, and may or may not include similar, identical or overlapping aspects.

In some embodiments, the set of tablet controls 4112 may display data relating to various patient physiological, respiratory and ventilation related parameters, and may include other output or presentation components, such as a speaker and/or a microphone. For example, a microphone could be used to determine the level of ambient noise, which could be used in determining and setting, or using particular settings to achieve, an appropriate volume level or volume control of the portable ventilator or its components. Furthermore, a combination of a speaker and microphone, used with appropriate software that could be stored on the portable ventilator, remotely, or both, could be used (such as in combination with, or in some instances instead of, GUI based interaction) in implementing or delivering user interactive CSG or other interaction with the user. For example, this could involve voice input provided by the user via the microphone, such as may include commands, confirmations, questions, responses or answers. It could also include voice output such as may include prompts, confirmations, questions, responses, answers, guidance, instructions or step-by-step instructions delivered to the user using the speaker. In various embodiments, the voice output may be algorithmically determined by the portable ventilator or an associated remote system, or may be provided by another user or care provider speaking to the user via the speaker, where the other user or care provider uses a microphone of a device of the other user or care provider. Furthermore, the user input could be directed to the portable ventilator itself, or to another care provider, or both. The other user or care provider could be relatively close to the user, such as in the area or a different portion of the care scene, or could be distant from the user, such as at a remote interface, which could include, for example, a doctor or other care provider at a hospital or medical facility, for example. In some embodiments, for example, the microphone and speaker of the portable ventilator may allow the user to interact in real time with, or take step by step instructions from, the doctor or other care provider concerning emergency care to the patient, whether relating to ventilation or other care.

Additionally, the set of tablet controls 4112 may include display of integrated data relating to operation of the portable ventilator 4102 as well as other devices that may also be in use, such as, for example, the CCM/defibrillator 4104. In some embodiments, the set of tablet controls 4112 may allow user interaction, including to obtain or display data, change settings of the portable ventilator 4102, accept or confirm suggested or recommended settings changes, or view or respond to alarms, alerts, or messages, among other things.

In the embodiment depicted in FIG. 1, any, some or all of the set of included controls 4144 of the portable ventilator 4102, the set of CCM/defibrillator controls 4134 or the set of tablet controls 4112 may have one or more respiratory aspects. Either or both of the set of CCM/defibrillator controls 4134 and the set of tablet controls 4112 may include remote controls relating to the portable ventilator 4102, such as may relate to controlling aspects of provided mechanical ventilation, among other things. Furthermore, any of the sets of controls 4144, 4134, 4112 may, for example, include display/GUI aspects relating to respiratory parameter data, respiratory status, or CSG. In some embodiments, the set of included controls 4144 of the portable ventilator 4102 may be limited, while the set of CCM/defibrillator controls 4134, and/or the set of tablet controls 4112, may be more comprehensive, or otherwise.

Furthermore, in some embodiments, remote control may include or allow relatively sophisticated or complex aspects of mechanical ventilation operation or control, such as may include, for example, CLC such as FiO2 CLC, PEEP CLC or CLC of one or more other ventilation parameters. Also, in some embodiments, the portable ventilator 4102 may be configured to switch to, or operate using, internal control as a backup or default form of control.

In various implementations, any of the depicted devices, systems or monitors may include controls with respiratory aspects and may include remote controls relating to the portable ventilator 4102. Furthermore, in various implementations, the portable ventilator 4102 may have any of a spectrum of different sets of included controls, from no included controls to a large set of included controls. Still further, in various implementations, various sets of remote controls may or may not in whole or in part reflect aspects of any included controls of a portable ventilator 4102. Additionally, in various implementations, roles or sensing capabilities may be variously included and distributed between devices, systems, monitors or users.

In some embodiments, the portable ventilator 4102 may obtain and communicate patient respiratory parameter data to one or several of the other devices or systems and/or data may be exchanged between various devices or systems. One or more devices or systems may use the communicated patient respiratory parameter data in determining a patient respiratory status (including associated data). Patient respiratory parameter data and/or respiratory status data may be used, for example, in determining CSG. In some embodiments, however, the portable ventilator 4102 may determine, or participate in determining, and may store, the patient respiratory parameter data and/or the patient respiratory status data, and may then communicate determined data to other devices or systems, for example.

In various embodiments, the portable ventilator 4102 may be capable of remote control by one or more of the CCM/defibrillator 4104, the tablet 4108 or one or more other devices, systems or entities.

In some embodiments, the portable ventilator may include a pulse oximeter for measuring patient SpO2, a capnographic sensor, which may be used in measuring EtCO2, and may include other sensing components for sensing other patient parameters. In some embodiments, however, some or all sensing components may be included with or distributed between other devices, for example.

While a particular combination of devices is shown in FIG. 1, in various embodiments, various combinations and roles between a portable ventilator and other devices can provide various advantages. For example, in some embodiments, a portable ventilator can be used with a CCM/defibrillator, as shown in FIG. 1. In some implementations, a portable ventilator can, in some ways, serve or function as an accessory treatment device to the CCM/defibrillator. For example, the CCM/defibrillator may provide sensing, or patient parameter sensing, capability, such as oximetry, capnography, blood pressure monitoring, and/or other sensing capability. Additionally, the CCM/defibrillator (or, in some embodiments, a tablet, or both) may play an important role in providing display/GUI features, which may include CSG. As such, in various implementations that include a CCM/defibrillator, the portable ventilator may include or provide more limited sensing capability, or display/GUI or CSG capability. As such, in some implementations, the portable ventilator may be simpler, smaller, lighter, and or more easily, quickly or conveniently portable, and/or its functionality, hardware and software may be less or simpler. For example, in some implementations, a care provider with a portable ventilator may arrive at a patient scene at which a different care provider (or several) is already providing care with (at least) a CCM/defibrillator, where the portable ventilator may be able to integrate, providing respiratory monitoring and ventilation capability, even after care and sensing has been initiated using at least the CCM/defibrillator. Alternatively, a care provider with a portable ventilator may arrive at the patient scene first, and one or more other devices may arrive and be integrated later.

In some implementations, a tablet or other portable computing device can serve as a device with an integrated and comprehensive display/GUI. As such, in some implementations, a portable ventilator may include less display/GUI and/or CSG features, and/or associated hardware or software, since the tablet or other portable computing device may provide, or to a degree provide, these features. In some embodiments, this can allow the portable ventilator to be simpler, smaller, lighter and/or more easily, quickly or conveniently portable.

In some embodiments, a remote, offsite care provider, and/or offsite computer system, may be included. In some implementations, the offsite care provider (and/or computer system) can provide additional support (computational or human), or expertise, beyond that which may be available locally. For example, in some embodiments, a more trained care provider or medical expert, such as a medically trained individual, nurse or doctor, may provide CSG, support, guidance, and/or even step by step instructions via a remote interface (and potentially utilizing offsite computing system capabilities, including hardware and software), such as may be delivered to the user of the portable ventilator, for example, via a display/GUI, and potentially speaker, of a tablet, CCM/defibrillator or portable ventilator, where the user of the portable ventilator may interact via the display/GUI and/or potentially a microphone of one of the local devices.

In some implementations, by integrating the role of the offsite care provider and/or offsite computing system, additional or enhanced care may be provided, which could include additional interaction and care enhancements or capabilities. Furthermore, in some embodiments, a video camera and display at the scene of the user of the portable ventilator could additionally enhance this interaction and care capabilities, with the user of the portable ventilator potentially taking step by step instructions from the offsite care provider, who may also use a video camera and microphone to provide video and audio feed to the user. The offsite care provider may be informed in part by communicated real-time video and audio data, feed or stream, which could include a view of and/or sounds of the patient as well as the user of the portable ventilator (and/or one or more other local care providers and/or devices). In some embodiments, this could include, for example, 3D video or augmented or enhanced reality presentation, to either or both of the user of the portable ventilator and the offsite care provider. For example, in some embodiments, either or both may use or wear one or more augmented or enhanced reality devices in this regard, such as a watch, head-worn or body-worn device, with video and audio capability. In various embodiments, this could provide hands-free use, or could even include motion or other sensors to track and display movements of the user and/or the offsite care provider.

In some embodiments including a portable ventilator, a CCM/defibrillator may not be included and, in some embodiments, a portable ventilator may be used without a CCM/defibrillator, tablet or portable computing device or remote monitoring system. In some implementations, by not including a CCM/defibrillator, the overall system used in monitoring and treatment of the patient may be more compact, simper, smaller, lighter, and/or more easily, quickly or conveniently portable. However, in many embodiments and implementations, the flexible, integrated, quickly adaptable nature of the system can allow for changes to included or excluded devices or systems, while still maintaining optimal patient care with presently available devices and systems. This can include, for example, adding, removing or shifting functionality or roles provided by currently available remaining devices or systems (or users), such as when a device or system (or user) is removed (or otherwise unavailable) or added (or otherwise available) to the overall system.

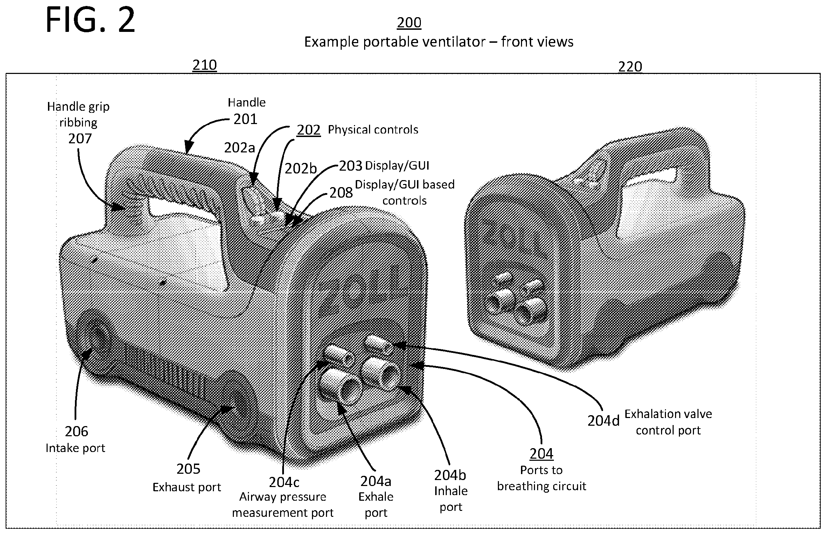

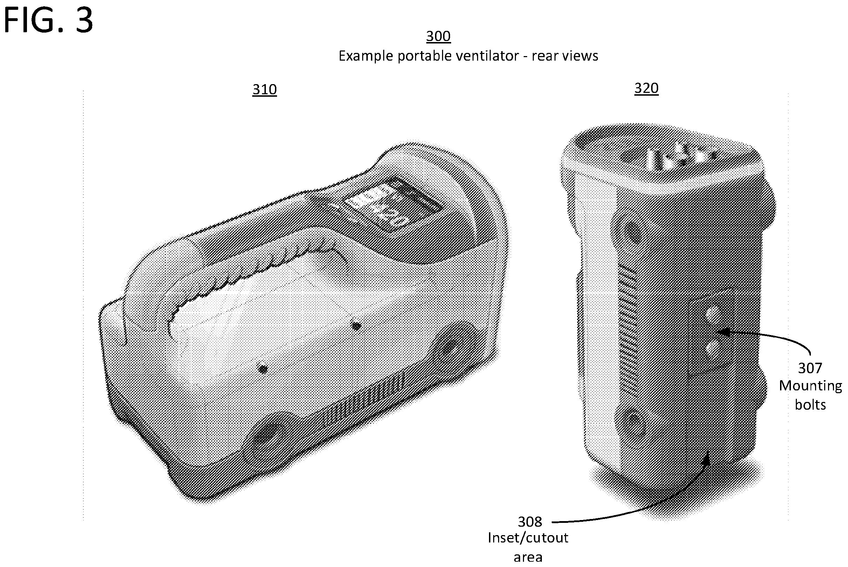

FIGs. 2-3 illustrate example front views 210, 220 and rear views 310, 320 of example portable ventilators 200, 300. As shown in FIG. 2, the portable ventilator 200 includes an intake port 206 and exhaust port 205. In the embodiment depicted, the portable ventilator of view 210 further includes ports 204 to one or more limbs of a breathing circuit, including an exhale port 204a, an inhale port 204b, an airway pressure measurement port 204c and an exhalation valve control port 204d. As shown in FIG. 3, the portable ventilator 300 also includes mounting bolts 307, or other mounting components, which may be provided for flexible use in coupling, attaching or mounting the portable ventilator to another object, surface or device, for example. In some embodiments, the mounting bolts 307 or other components may be located on the bottom of the housing of the portable ventilator, and may be positionally adjustable (e.g. slidable), for example, so as to accommodate different mounting brackets or other components and variable mounting positions. In some embodiments, the housing of the portable ventilator may include an inset portion to facilitate flush or secure mounting.

As further shown in FIG. 2, the portable ventilator 200 includes a handle 201, which may be part of or coupled with a housing of the portable ventilator 200, which allows for single-handed operation of the portable ventilator 200 in delivering mechanical ventilation to a patient. In the embodiment depicted, the handle 201 includes a ribbed grip surface area 207, or may include other surface features or aspects for enhancing, securing or guiding grip or physical control by an operator.

A set of included controls of the portable ventilator 200 include physical controls 202, such as a scroll wheel 202a and buttons 202b as shown, as well a set of display/GUI based controls 208 on a display/GUI 203 of the portable ventilator 200. In various embodiments, various included controls of the portable ventilator 200 may include controls placed on the handle 201, elsewhere on the portable ventilator 200, or partially or overlapping between the two. Furthermore, in various embodiments, the portable ventilator may have one or more display/GUI areas placed on the handle 201, elsewhere on the portable ventilator 200, or overlapping between the two.

In some examples, the one or more first buttons or dials or first controls, which may be display based, are arranged on the handle or on the housing in an area extending up to 5cm, up to 6cm, up to 7cm, up to 10 cm, up to 15 cm around the handle where it meets the housing or overhangs the housing. In some examples, the one or more first buttons or dials or first controls are located on one or more of: a top surface of the handle; a first side surface of the handle; an underside surface of the handle; on the handle where the handle meets the housing; and on the housing in a region around where the handle meets the housing, such as for accessing by at least the thumb of the user. In some examples, the one or more first buttons or dials or first controls are located on one or more of: a top surface; a first side surface; a second side surface opposed to the first side surface; an underside surface of the handle; the housing in a region around where the handle meets the housing; on the handle where the handle meets the housing; on the housing in a region where the handle overhangs the housing, such as for accessing by at least the fingers other than the thumb of the user. It will be appreciated that the top surface of the handle may face away from the housing and the underside surface of the handle may face the housing. The provision of the one or more first buttons or dials or first controls first side surface and the second side surface may, for example, depend on whether the handle is intended to be gripped by a left or right hand.

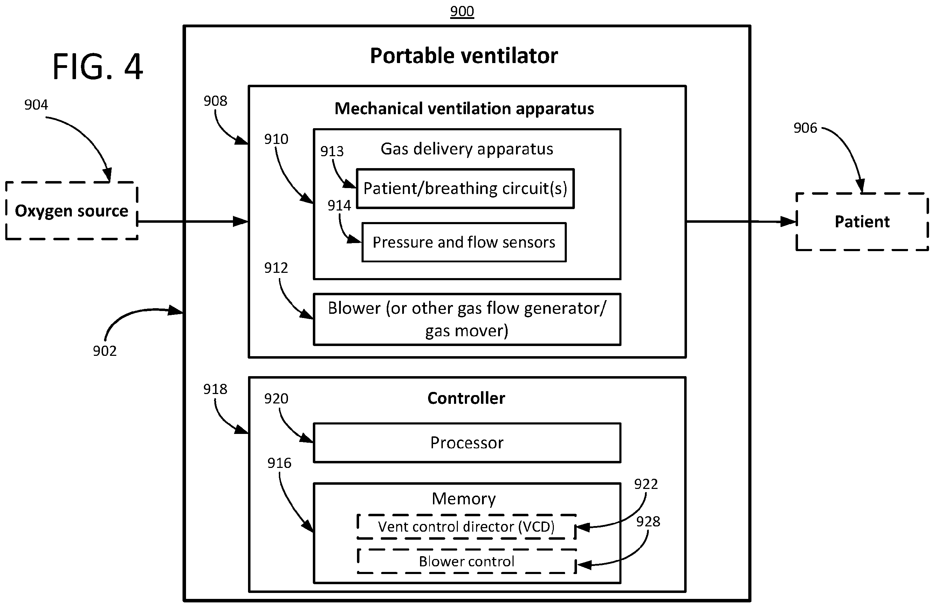

FIG. 4 is a block diagram of an example portable ventilator 900, in accordance with some embodiments, capable of providing ventilation, and potentially other interventions or treatments, to a patient 906. The portable ventilator 900 includes a housing 902 (which may include a handle, or a handle may be coupled with the housing), a mechanical ventilation apparatus 908 and at least one controller 918, and may include or be connected to an oxygen source 904, and may include a handle (not shown). The mechanical ventilation apparatus 908 includes a gas delivery apparatus 910 including one or more patient circuits, pressure and flow sensors 914, and a blower 912 or other gas flow generator / gas mover, such as a blower. The mechanical ventilation apparatus 908 may include or partially include, or may be included within or partially within, various other components, such as, for example, a pneumatic manifold and at least one electrical circuit board, examples and components of which are described further herein. In various embodiments, the one or more patient circuits or breathing circuit 913, or portions thereof, may be coupled with the portable ventilator 900 or may be included as part of portable ventilator 900.

In some embodiments, at least some of the pressure and flow sensors 914 may provide signals received and used by the controller 918 (or, in some embodiments, by a controller, computer, or computerized device or system separate or remote from the portable ventilator 900) for purposes including controlling aspects of mechanical ventilation provided to the patient 906, which may include control or actuation of the blower 912, as well as in connection with exhalation valve control.

In some embodiments, the portable ventilator 902 is capable of sensing signals representative of gas flow that can be used in determining at least one patient respiratory parameter, such as may include use or one or more of the pressure and flow sensors 914, pneumotachometers or spirometers, that may, in some embodiments, be included as part of, or within or partially within, the mechanical ventilation apparatus 908, or may at least in part be separate but communicatively coupled by wired or wireless connection. In various embodiments, one or more spirometers may be included within the portable ventilator 900 and/or may be coupled (physically and/or communicatively by wired or wireless connection) with the portable ventilator 900. Although depicted in a separate box from the patient circuits 913, it is to be understood that components such as the pressure and flow sensors 914, pneumotachometers and spirometers, may be included within or partially within the patient circuits 913, such as a patient inspiratory circuit and/or a patient expiratory circuit. In some embodiments, one or more of the pressure and flow sensors and other associated components may be used in sensing or obtaining respiratory parameter data, which respiratory parameter data may be used in determining or generating a respiratory status (which can include associated data). The respiratory parameter data and respiratory status data may also be used in connection with control of operation of the portable ventilator 900, such as in control of aspects of mechanical ventilation providing by the portable ventilator 900, whether such control is internally provided by the portable ventilator 902, remotely provided, or with aspects of both. Furthermore, in some embodiments, the respiratory parameter data and respiratory status may be used in determining or generating CSG.

The controller 918 includes at least one processor 920 and at least one memory 916 for storing data, and may also include software that may be stored in the at least one memory 916, such as may include, or may include aspects of, a ventilation control director (VCD) 922 and blower control 928. The VCD 922 may represent software aspects of the controller 918 used in control relating to internal and remote control of mechanical ventilation by the portable ventilator 900. For example, in some embodiments, the VCD 922 may perform a fault mode condition analysis to determine whether a fault mode condition exists, such as lack of communication between the portable ventilator 900 and the remote control device (or other fault mode conditions, as previously described herein). If no fault mode condition is determined to exist, the VCD 922 may allow control of mechanical ventilation of the portable ventilator 922 by the remote control source. However, if a fault mode condition is determined to exist, then the internal control of mechanical ventilation of the portable ventilator, by the controller 918, may instead be utilized. The VCD 922 may also use various algorithms or rules to make other determinations relating to remote or internal control, such as in determining switching between internal control and control by a remote control device, and in selecting between multiple remote control devices, for example.

Blower control 928 may represent software aspects of the controller 918 used in blower control, such as described in embodiments herein.

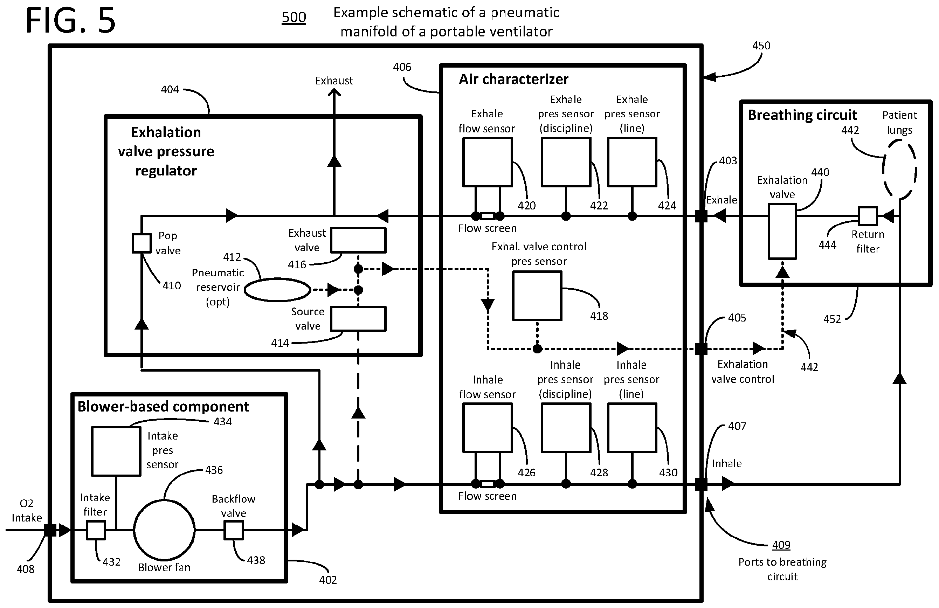

FIGs. 5-10 are example schematic diagrams of example pneumatic manifolds and other components of example portable ventilators. In the diagrams, black arrow heads are used in illustrating gas flow and gas flow direction.

In each of the portable ventilators according to the embodiments of FIGs. 5-10, gas pressures and gas flow rates in the exhalation and inhalation paths of the pneumatic manifold are measured using sensors associated with the air characterizer, such as sensors of at least one electrical circuit board, or a portion thereof, coupled with (or part of) the air characterizer. The electrical circuit board(s) may be or include one or printed circuit boards (PCBs), or groups or stacks thereof, that mount precisely (with or without additional mounting-relating or seal-related components) to the air characterizer in order to allow leak-free sensor porting. Signals from the sensors are transmitted to the controller and used to determine the associated pressures and flow rates. The controller uses the determined pressures and flow rates in controlling and optimizing mechanical ventilation parameters associated with mechanical ventilation provided by the portable ventilator to the patient, which may include the controller using or executing of one or more algorithms stored in the memory of the controller. In some embodiments, among other things, such sensor signals are used by the controller in blower control and in exhalation valve control.

The embodiments depicted in FIGs. 5-10 vary in aspects including, for example, placement of an exhalation valve and whether an exhalation valve control tubing based path is included, whether a patient airway pressure measurement port, tubing based path and associated pressure tap is included, and in the sensors included in the air characterizer.

In various embodiments, various types of exhalation valves, and exhalation valve control, such as may be implemented by the controller using one or more algorithms, are used. For example, in some embodiments, an exhalation valve (sometimes called an exhalation control valve) may be or include an air-piloted diaphragm or mushroom type valve, such as an elastomeric valve. In various embodiments and placements, a disposable or re-useable exhalation valve may be used.

In some embodiments, one or more valves (which, in various embodiments, can include one, two, three or more than three), or other components, are used in control of the exhalation valve, which valves may be controlled by the controller based using one or more algorithms. The one or more valves may be used to deliver and decrease pressure provided to an exhalation valve, which may be used in opening or closing the exhalation valve, whether completely or to various degrees (e.g., proportional control).

In some embodiments, the one or more valves include a source valve (which may be called a source proportional valve), which is controlled and used to increase or add to the pressure provided to the exhalation valve, and an exhaust valve (which may be called a vent valve or vent proportional valve), which is controlled and used to decrease or subtract from the pressure provided to the exhalation valve (associated gas flow from which may be expelled out of the exhaust port). The controller may control both the source valve and the exhaust valve (which, together, may function as a source proportional valve) such that the combined operation of both valves causes a determined appropriate amount of pressure to be delivered to the exhalation valve, for appropriate control of the exhalation valve.

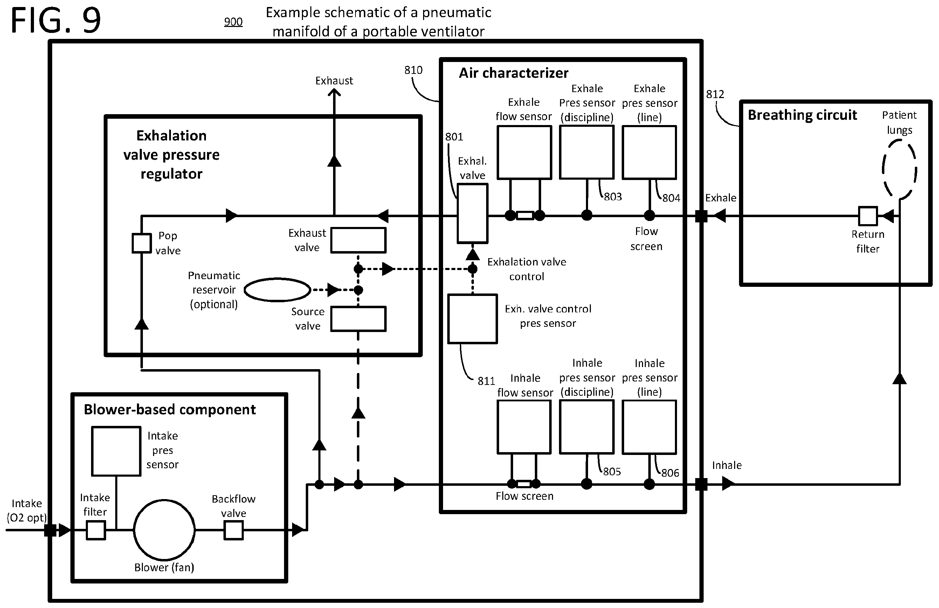

With regard to exhalation valve placement and an exhalation valve control tubing based path, in some embodiments, including those depicted in FIGs. 5-8, the exhalation valve is disposed within the breathing circuit. Given this placement, an exhalation valve control tubing based path may be included that forms at least part of the path from the portable ventilator to the exhalation valve in the breathing circuit. In some embodiments, both the breathing circuit and the exhalation valve within the breathing circuit may be disposable. In other embodiments, however, including those depicted in FIGs. 9-10, the exhalation valve is disposed within the air characterizer, and, as a result, no exhalation valve control tubing based path to the breathing circuit is included.

As such, in some embodiments, locating the exhalation valve within the breathing circuit may provide advantages, in some applications or contexts, including that, although it may require a tubing based path from the pneumatic manifold to the exhalation control valve, the exhalation valve may be of a disposable variety. Furthermore, since the exhalation control valve is not located in the pneumatic manifold, it does not itself add a component to the pneumatic manifold. However, in some embodiments, locating the exhalation valve within the pneumatic manifold may provide advantages, in some applications or contexts, including that, although it does add a component to the pneumatic manifold, no exhalation valve control tubing based path must be included from the portable ventilator to the exhalation valve within the breathing circuit, and that component may be avoided.

It is to be noted that, in other embodiments, the exhalation valve could be placed elsewhere than described above, such as elsewhere in the pneumatic manifold, elsewhere in the portable ventilator, or elsewhere outside of the portable ventilator. In some such embodiments, gas paths within and/or outside of the portable ventilator may be included and arranged as necessary to allow for pressurization and control of the exhalation valve.

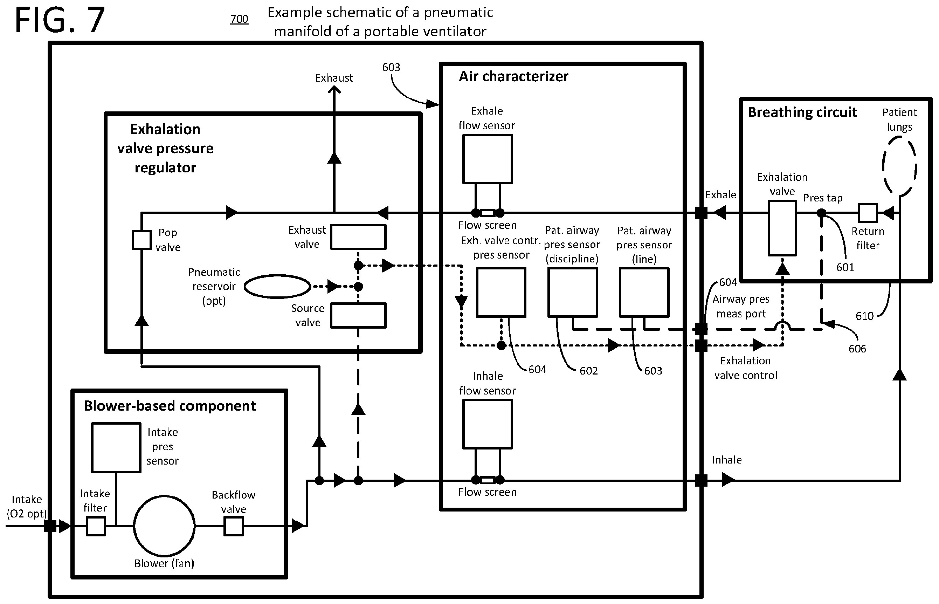

With regard to a patient airway pressure measurement port, in some embodiments, such as those depicted in FIGs. 7-8, patient airway pressure is measured directly via a pressure tap within the breathing circuit. Given this placement, a patient airway measurement line (tube based line) is included that runs from the air characterizer through a port in the housing of the portable ventilator to the pressure tap within the breathing circuit. One or more pressure sensors are included in the air characterizer that are used in measurement of the patient airway pressure.

In other embodiments, however, such as those depicted in FIGs. 5-6 and FIGs. 9-10, patient airway pressure is less directly determined using a computation or estimation using pressures determined in each of the exhalation and inhalation paths of the air characterizer. Given this placement, no such tubing based path to a pressure tap in the breathing circuit is included. However, one or more pressure sensors are included in each of the exhalation and inhalation paths of the air characterizer in order to measure pressures in each of those lines, which are used in computing or estimating patient airway pressure.

In some embodiments, pressure and flow rate measured in inhalation and exhalation lines, are used in computing or estimating patient airway pressure and flow, using one or more appropriate techniques, such as may include one or more computational models or algorithms used by the controller. As one example, in some embodiments, a technique that may be used in computing or estimating patient airway pressure and/or flow rate is described in the published article: "Monitoring Respiratory Mechanics During Mechanical Ventilation: Where Do the Signals Come From?", by Warren G Sanborn, PhD - Respiratory Care - January 2005 Vol. 50 No. 1.

It is to be noted that, in other embodiments, patient airway pressure may be determined using one or more pressure measurements at one or more other locations within or outside of the portable ventilator or breathing circuit. In such embodiments, gas paths within and/or outside of the portable ventilator would be included and arranged as necessary to allow for such measurement.

With regard to sensors included in the air characterizer, various arrangements are used in the various embodiments depicted in FIGs. 5-10. With regard to each instance of measurement of each of pressure and flow rate, in some embodiments, a single sensor may be used, if the sensor meets the necessary requirements. For example, such requirements may include that the sensor is associated with sufficient gain, and that offset associated with the sensor is at or below an acceptable threshold. This may include being at or below an acceptable threshold over an entire range of anticipated or likely temperatures, which can tend to increase offset, and which range may be considerable in out-of-hospital and field contexts.

In other embodiments, however, a combination of two sensors are used in each pressure measurement. In such instances, one of the two pressure sensors may provide gain at or above a particular acceptable threshold (herein, this type of sensor is referred to as a "line sensor"), while the other may have offset that is at or below an acceptable threshold (herein, this type of sensor is referred to as a "discipline sensor"). In some embodiments, both line sensors and discipline sensors sense, and are used in measuring, a difference in pressure between an airway pressure and ambient pressure. For example, in some embodiments, the controller uses one or more algorithms to determine a pressure measurement based on the signals from both sensors.

In various embodiments, sensors with various characteristics and specifications may be used, some examples of which follow. In some embodiments, flow sensors may be used that have a measurement range of, e.g., -300 to 300 l/min, a measurement accuracy of, e.g., plus or minus 10% of reading + 0.2 l/min, a step response time (T63, or the amount of time the measurement takes to reach 63% of final value upon an instant change in the variable being measured) of, e.g., less than 3 msec. In some embodiments, pressure sensors, such as may be used to detect airway pressure, may be used that have a measurement range of, e.g., -10 to 100 cm H2O, a measurement accuracy of, e.g., plus or minus 8% of reading + 2 cm H2O, a step response time (T63) of, e.g., less than 3 msec, and an offset stability of, e.g., less than 0.03 Pa/year. Furthermore, in some embodiments, both flow and pressure sensors may have environmental specifications that include an operating temperature range of, e.g., -20 to 85 degrees C, a storage temperature range of, e.g., -40 to 70 degrees C, an altitude range of, e.g., - 2250 to 15,000 feet, a humidity range of, e.g., 15-95% non-condensing, a vibration (10 Hz to 2 kHz) to, e.g., 15 g, and shock (6 ms) to, e.g., 100 g.

It is to be noted that, in other embodiments, other combinations of two or more sensors or types of sensors may be used for particular pressure, flow rate, or other measurements.

As described as follows, the embodiments shown in FIGs. 5-10 provide example schematics of various example pneumatic manifolds with various combinations of features. It is to be noted, however, that many other combinations are possible. These may include particular embodiments that use a different combination of approaches to each of the feature aspects, as described above, including as relate to exhalation valve placement, patient airway pressure measurement and sensors included in the air characterizer, among other things.

As depicted in FIG. 5, a pneumatic manifold 500, contained within a housing 450 of a portable ventilator, includes a blower-based component 402, an exhalation valve pressure regulator 404 and an air characterizer 406 (or portions thereof). As depicted, ports 409 included from the housing 450 to a breathing circuit 452 include an exhale port 403, an inhale port 407 and an exhalation valve control port 405, from which an exhalation valve tubing based path 442 leads to an exhalation valve 440 within the breathing circuit 452. Additionally, as depicted, an Oxygen (O2) intake port 408 is included. In some embodiments, the intake port 408 may be a low pressure intake port or valve facilitating connection to a high pressure oxygen source, for example. In some embodiments, the breathing circuit 452 is a consumable item that is periodically replaced.

The blower-based component 402 includes or contains a blower 436, an intake filter 432, an intake pressure sensor 434 and a backflow valve 438.

The exhalation valve pressure regulator 404 includes or contains a pop valve 410, a pneumatic reservoir 412, a source valve 414 and an exhaust valve 416, with the source valve 414 and the exhaust valve 416 being mounted to a at least a portion of the exhalation valve pressure regulator 404 that forms a portion of the pneumatic manifold. In some embodiments, the pop valve 410 is a pressure relief valve that is used to limit the pressure of gas delivered to the patient, for example, to some pre-determined maximum. The pop valve may, for example, be a one-way valve, such as an elastomer valve or elastomer umbrella valve, that may connects airway pressure to ambient pressure, to relieve airway pressure, if at least a threshold pressure is delivered to the pop valve, such as, for example, 100 cm H2o (or, e.g., between 75-125 cm H2O). The exhalation valve control pressure sensor is used in measurement of pressure associated with the relatively small flow of gas being provided by the exhalation valve pressure regulator 404 to provide pressure to the exhalation valve 440.

In other embodiments, the exhalation valve pressure regulator may be of reduced complexity and size. For example, in some embodiments, the source valve and the vent valve may be mounted on (or included as part of) the air characterizer, and/or the pop valve may be included with or coupled with the air characterizer or elsewhere, allowing for reduction in the functionality and structure of the exhalation valve pressure regulator, or its omission altogether.

The breathing circuit 452 includes or contains the exhalation valve 440 and a return filter 444. The patient's lungs are also depicted 442, coupled with the breathing circuit 452.

In the embodiment 500 shown in FIG. 5, various sensors are included in association with the air characterizer 406. As mentioned above, this may include sensors that are included in an electrical circuit board coupled with the air characterizer, for example. The sensors include exhale path and inhale path flow sensors 420, 426, along with associated flow screens. In some embodiments, the flow screens may be precisely shaped and structured, using injection molding, for example. The sensors also include exhale path and inhale path pressure sensors, including discipline sensors 422, 428 and line sensors 424, 430, and the exhalation valve control pressure sensor 418. As described in examples herein, the air characterizer 406 includes a portion of each of the exhalation and inhalation channels (sometimes called chambers) of the pneumatic manifold, which facilitates pressure and flow rate measurements in the exhalation and inhalation limbs using sensors of the electrical circuit board coupled with the air characterizer 406.

In various embodiments, various manufacturing processes and techniques may be used in manufacturing the air characterizer 406 and other components of the pneumatic manifold, such as, for example, injection molding, joining of portions via glue, welding or ultrasonic welding, mechanical joining such as bolting with gasket seals, or other techniques. In some embodiments, the air characterizer is structured and shaped precisely and accurately enough to provide for the various necessary channeling and porting.

In the embodiment 500 shown in FIG. 5, the exhalation valve control pressure sensor 418 is coupled with the air characterizer 406. This may provide an advantage in that no sensor or associated electronic circuit board must be included that is coupled with the exhalation valve pressure regulator 404. It may provide a further advantage in that ambient reference pressure porting available in association with the air characterizer 406 may be utilized for the exhalation valve control pressure sensor 418.

In the embodiment depicted in FIG. 5, gas flow through the exhalation path leads, sequentially relative to each other, to the line pressure sensor 424, the discipline pressure sensor 422 and the flow sensor 420, while gas flow through the inhalation path leads, sequentially relative to each other, to the flow sensor 426, the discipline pressure sensor 428, and the line pressure sensor 430. In some embodiments, this arrangement may be optimal or necessary for accurate, or most accurate, measurements. For example, flow screens used with or as part of flow sensors may cause a small non-transitory decrease in pressure. As such, in the exhalation flow, if gas flow reaches the flow sensor before the one or more pressure sensors, then the measurement associated with the pressure sensor use may be rendered inaccurate or less accurate as representative of pressure associated with gas exhaled by the patient (which is the pressure of the gas prior to reaching the flow sensor). Conversely, in the inhalation flow, if gas reaches the pressure sensors before the one or more flow sensors, then the measurement associated with the pressure sensor use may be rendered inaccurate or less accurate as representative of pressure associated with gas inhaled by the patient (which is the pressure of the gas after reaching the flow sensor). Therefore, the arrangement in FIG. 5 may provide optimal accuracy for pressure measurements. Similar sequencing (regarding pressure and flow sensing), for similar reasons, may be used in other embodiments in which a single pressure sensor is used instead of two pressure sensor combinations as shown in FIG. 5, and similar sequencing may also be used in embodiments in which more three or more pressure sensor combinations are used, or in other embodiments.

In some embodiments, some or all sensors used in association with the air characterizer are differential pressure sensors, which must be connected to a reference pressure reflecting ambient pressure. In some embodiments, to prevent the need, for example, for a connection to ambient pressure outside of the portable ventilator without liquid or particulate protection, an internal ambient pressure reference volume is provided within the portable ventilator, which is ported to the sensor. This volume is connected to ambient pressure via an immersion proof breather device. The immersion proof breather device permits bidirectional gas flow with no minimum pressure difference but prevents and protects against passage of liquid or particulates. Among other things, this arrangement provides a solution to the technical problem of connecting the sensors to an ambient reference pressure without the otherwise associated potential exposure to liquid and particulates.

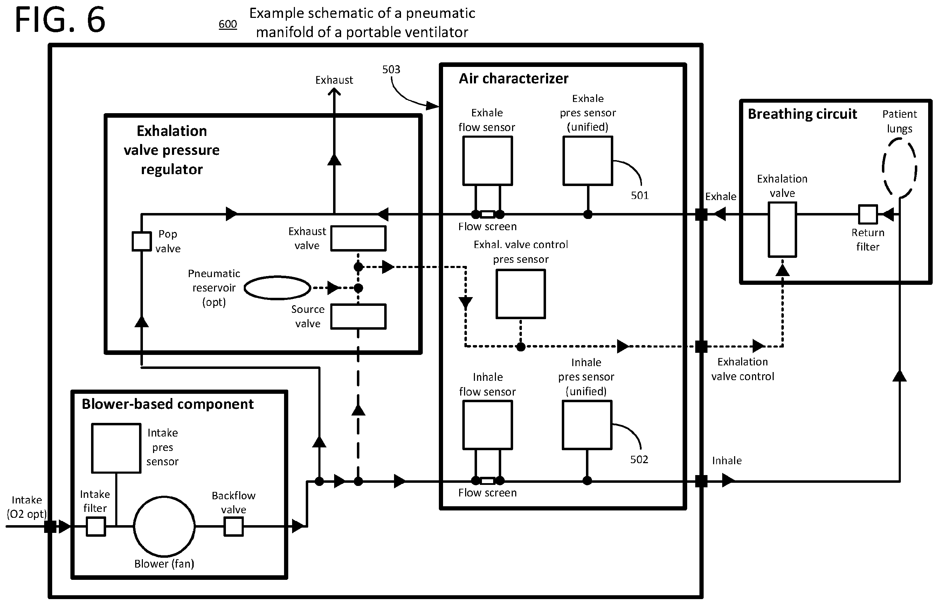

The embodiment 600 shown in FIG. 6 differs from that of FIG. 5 in that single (or unified) pressure sensors 501, 502 are used in association with each of the exhale path and the inhale path of the air characterizer 503.

The embodiment 700 shown in FIG. 7 differs from that of FIG. 6 in several ways. No pressure sensors are included in association with each of the exhale path and the inhale path of the air characterizer 603. However, a pressure tap 601 is included in the breathing circuit 610, and a path 606 is included from the pressure tap 601 through an airway pressure tap port 604 in the housing of the portable ventilator, which path 606 includes a tubing based path from the breathing circuit 610 to the housing of the portable ventilator and further includes channeling within the pneumatic manifold. The path 606 leads to patient airway pressure sensors, including discipline and line sensors 602, 603, associated with the path 606 in the air characterizer 603.

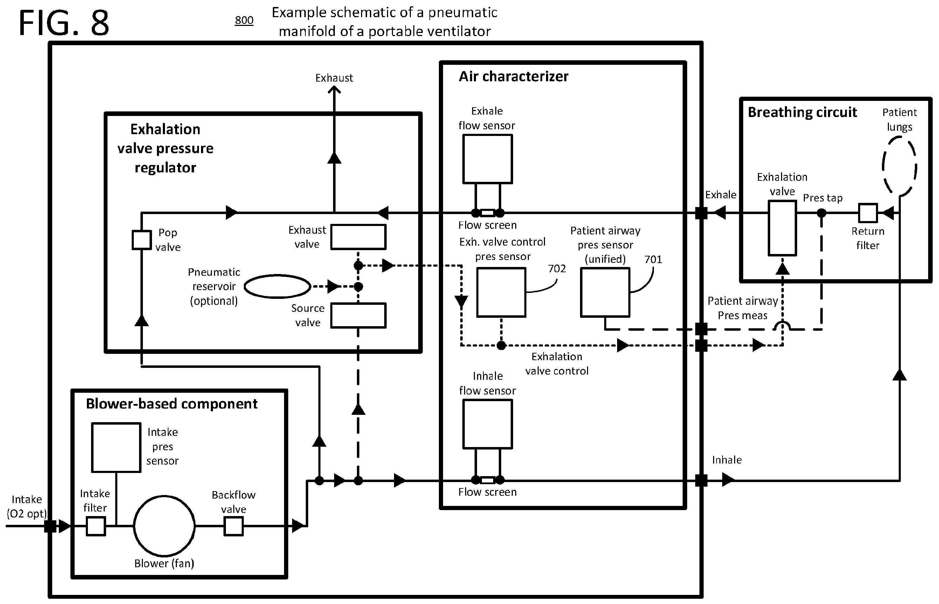

The embodiment 800 shown in FIG. 8 differs from that of FIG. 7 in that a single patient airway pressure sensor 701 is used, rather than a discipline and a line sensor as in the embodiment shown in FIG. 8. An exhalation valve control pressure sensor 702 is included.

The embodiment 900 shown in FIG. 9 differs from that of FIG. 8 in several ways. The exhalation valve 801 is included within the air characterizer 810, rather than in the breathing circuit 812. As such, no exhalation valve control tubing based path is included to the breathing circuit 812. Given the different placement of the exhalation valve relative to the embodiment shown in FIG. 8, the path associated with exhalation valve control is also different, but an exhalation valve control pressure sensor is included 811. Also, in the embodiment 900 shown in FIG. 9, no patient airway pressure tap is included within the breathing circuit 812 and no patient airway pressure measurement path is included (including no tubing based path from the breathing circuit). Furthermore, no patient airway pressure sensor(s) are included within the air characterizer 810. Accordingly, however, pressure sensors are included in association with the air characterizer 810 for measurement of pressure in the exhalation path and the inhalation path of the air characterizer 810, including discipline sensors 803, 805 and line sensors 804, 806, allowing for calculation or estimation of patient airway pressure. It is also noted that, in some embodiments, different types of exhalation valves and associated control may be provided. For example, in some embodiments, an electrically rather than pneumatically controlled exhalation valve may be provided.

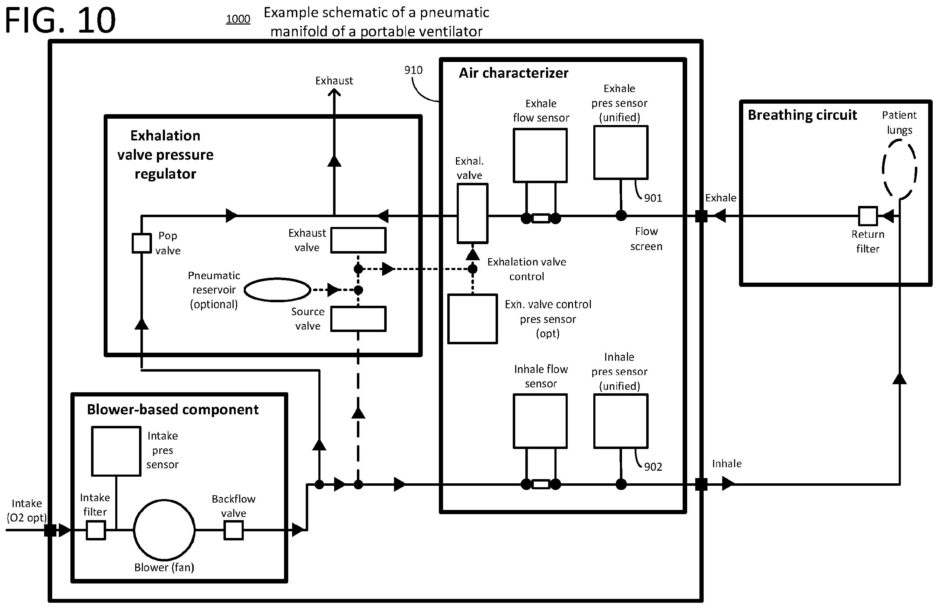

The embodiment 1000 shown in FIG. 10 differs from that of FIG. 9 in that single sensors 901, 902 are used to measure pressure in each of the exhale path and the inhale path of the air characterizer 910, rather than combinations of discipline and line sensors.

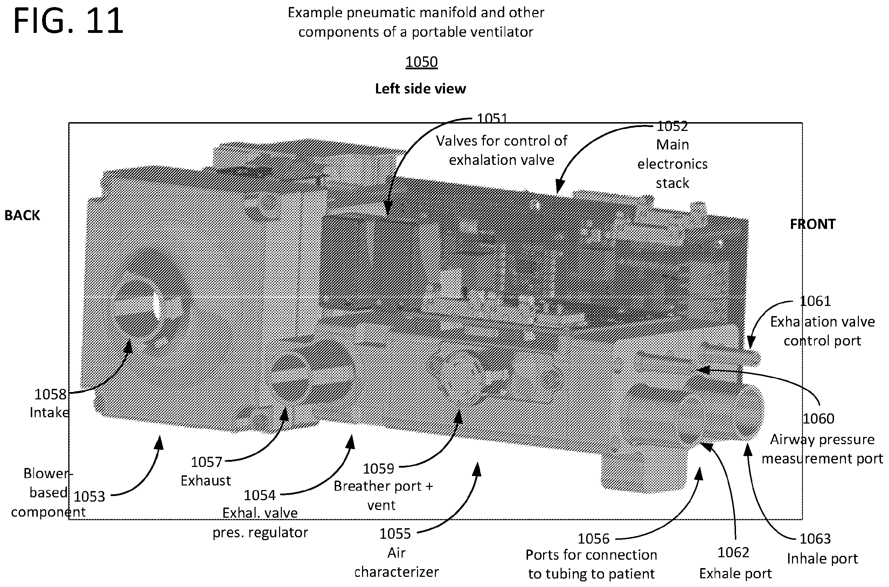

FIG. 11 illustrates an example perspective view 1050 of the left side of a pneumatic manifold and other components of an example portable ventilator. In the embodiment depicted, the pneumatic manifold is a rigid pneumatic manifold that includes a blower-based component 1053, an exhalation valve pressure regulator 1054 and an air characterizer 1055 (or portions thereof). Ports 1056 are included for connection to tubing leading to the patient, including exhale and inhale ports 1062, 1063, an exhalation valve control port 1061 and an airway pressure measurement port 1060. Exhaust and intake ports 1057, 1058 are also included, as well as a breather port and vent 1059, to which an immersion proof breather device may be attached. Valves for control of the exhalation valve, which in this embodiment include source and exhaust valves 1051, are coupled with the exhalation valve pressure regulator 1054 for use in regulating pressure to the exhalation valve. A main electronics stack 1052 is also included, which may include one or circuit boards (e.g., a stack of PCBs) including (or otherwise coupled with) various sensors, among other components, for example. The main electronics stack 1052, or portions thereof, may be coupled with (or, in some embodiments, included as part of) the air characterizer 1055, for example.

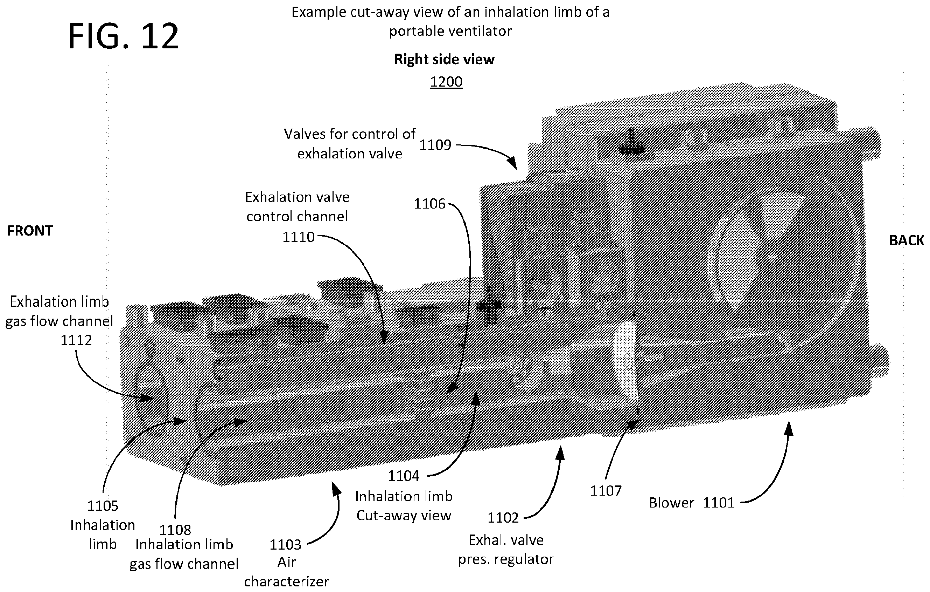

FIG. 12 illustrates a cut-away side view 1200 of an example inhalation limb 1105 of a pneumatic manifold and other components of an example portable ventilator. An exhalation valve control channel 1110 can be seen, leading from the source and exhaust valves 1109 through the pneumatic manifold, ultimately leading to an exhalation valve included within the patient circuit (although, in other embodiments, the exhalation valve is placed elsewhere, such as within the air characterizer).

The air characterizer 1103 is rigidly and precisely joined with the exhalation valve pressure regulator 1102, as generally suggested by the visible exterior seam 1106, which joining includes rigid, flush mating of portions of the surfaces of each within the pneumatic manifold, and may including use of joining materials or components, such as a gasket. Similarly, the exhalation valve pressure regulator 1102 is joined with the blower-based component 1101, as generally suggested by the visible exterior seam 1107, although the joining includes rigid, flush mating of portions of surfaces of each within the pneumatic manifold. Such joining of these components of the pneumatic manifold allows for continuous gas flow channels to be formed by and within the pneumatic manifold itself, including between components of the pneumatic manifold. Such channels may include, for example the inhalation limb gas flow channel 1108, the exhalation limb gas flow channel 1112, as well potentially other channels, such as an exhalation valve control channel and, in some embodiments, a patient airway pressure measurement channel.

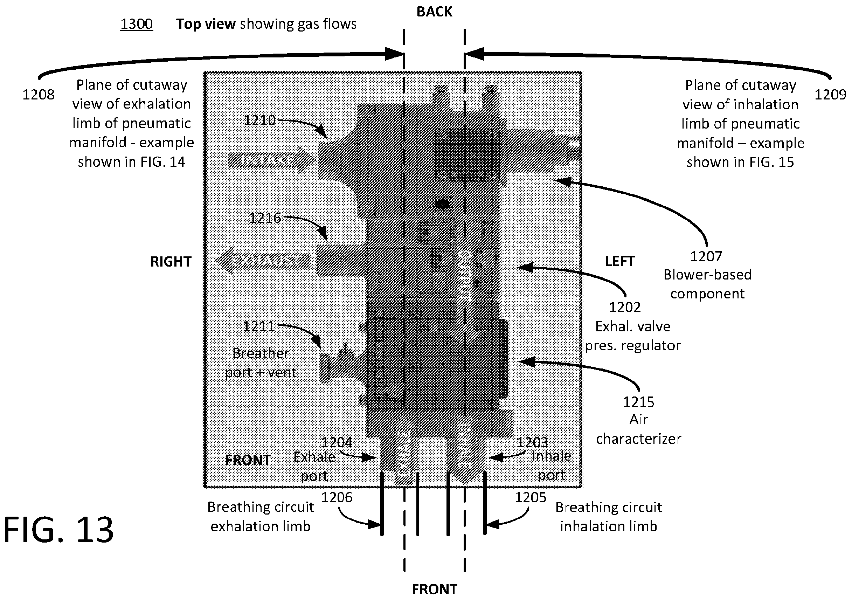

FIG. 13 illustrates a top view 1300 of an example pneumatic manifold and other components of an example portable ventilator, showing gas flow paths. As depicted, in use, gas flows into the blower-based component 1207 via intake 1210, and continues to flow through from the blower-based component 1207 through the pneumatic manifold inhalation limb generally on the left side, including through the exhalation valve pressure regulator 1202 and the air characterizer 1215, and out of the inhale port 1203 to the breathing circuit inhalation limb 1205 and to the patient. Gas exhaled by the patient flows from the breathing circuit exhalation limb 1206 into the exhale port 1204, and continues to flow through the exhalation limb of the pneumatic manifold generally on the right side, including through the air characterizer 1215 and the exhalation valve pressure regulator 1202, and is exhausted out of the exhaust port 1216. In the embodiment depicted, gas flow channels (sometimes called chambers) of the inhalation limb and the exhalation limb of the pneumatic manifold are connectable via a valve, such as a pop valve, that, when needed, such as if pressure rises past a safe threshold, allows relief of excess pressure in the inhalation limb 1205 by allowing gas from the inhalation limb 1205 to be expelled from the exhaust 1216.

It is to be noted that, generally, embodiments shown and described herein, or portions thereof, can also be implemented in a left-to-right mirror image fashion, so that, in alternative embodiments, aspects and components on the left side may instead be on the right side and vice versa. Such left-to-right mirrored alternatives are within the scope embodiments described herein, even though only one of the two left-to-right mirrored alternatives may be depicted and/or described as an example.

Some embodiments described herein take into account various optimization factors in order to optimize the structure and operation of a portable ventilator a whole, and to provide solutions to the overall technical problems of providing optimally fast and efficient care to a patient experiencing respiratory distress, as well as solutions to various more specific technical problems associated therewith. For example, some embodiments reflect a recognition that such optimization factors and solutions include minimizing the size, weight and footprint of a portable ventilator. For example, such minimizations can optimize practicality, ease of carrying, ease of grasping and positioning, and overall ease of use of the portable ventilator. Furthermore, such minimizations can solve the problems of the negative impacts of overcrowding or over-complication in the area near the patient, which area may include a number of care providers and devices, which may interact with the patient or each other. This can include, for example, minimizing the impact of the portable ventilator and its use regarding such potential over-crowding. Such overcrowding can slow down, complicate or otherwise negatively impact patient care, which care may require fast and efficient care provider movements and actions, fast and efficient deployment of devices, and fast and efficient attachment of device components to the patient. Such advantages can be realized, for example, in connection with providing patient care and for sensing and measurement of patient physiological parameters including, for example, heart rate, blood pressure, heart rate, patient oxygenation, patient respiratory status and patient respiratory parameters.

Additionally, optimization factors, and solutions to technical problems associated therewith, can include, for example, simplifying and optimizing particular portions and components of the portable ventilator, simplifying and optimizing the arrangement, geometry and placement of such portions and components within the portable ventilator as a whole and relative to each other, and simplifying and optimizing gas flow within the portable ventilator. As such, with respect to portable ventilators described herein, various aspects contribute to providing solutions to the overall problem of providing a portable ventilator that provides optimized performance and efficiency in operation and its role in providing patient care. These aspects may include, for example, particular optimized portions and components used with and as part of the portable ventilators, optimized arrangements and geometric relationships of components within portable ventilators, and optimized and simplified gas flow patterns within the portable ventilator as impacted by such components, arrangements and geometries. Accordingly, some embodiments take into account a recognition that unnecessarily complex or unnecessarily non-uniform shaping or geometry of the portable ventilator can jeopardize, add difficulty or add complexity to providing for aspects of operation of the portable ventilator. Additionally, embodiments described herein are optimized to provide sufficient ingress protection or to keep the size or footprint of the portable ventilator to a minimum. In some embodiments, aspects of the portable ventilator and pneumatic manifold include optimization in these regards, including with regard to arrangement, orientation and sequencing of portions of the portable ventilator and pneumatic manifold, and size and shape of the portable ventilator and its various components, as described further herein.

For example, in some embodiments, gas flow channels within the exhalation and inhalation limbs are arranged to provide relatively simple and efficient gas flow paths and associated path and component relationship-related geometries. In particular, for example, bends, turns and flow restrictions in gas flow paths can decrease operational efficiency and can cause pressure drops. Furthermore, non-straight gas flow into screens, such as screens associated with flow sensors, can decrease the accuracy of associated measurements, such as flow rate measurements. As such, providing relatively simple and efficient gas flow paths provides a solution to the technical problem of maximizing the efficiency of the portable ventilator in this regard, and also thereby contributes to optimizing overall efficiency and performance of the portable ventilator. Additionally, in some embodiments, the arrangement of the portions of the pneumatic manifold, including the side-by-side positioning of the exhalation and inhalation limbs, may add practicality, and spatial and volume efficiency/minimization. This can include allowing positioning components over, and coupled to top areas of, portions of the pneumatic manifold, including the exhalation valve pressure regulator and the air characterizer. Such components may include, for example, valves, one or more electronic circuit boards (such as sensors of printed circuit boards or PCBs, or stacks thereof), and a battery. In particular, in some embodiments, the air characterizer must be coupled to such sensors, which this arrangement efficiently allows. These features provide solutions to technical problems associated with the portable ventilator and its operation and use, including minimizing structural complexity as well as overall portable ventilator volume and footprint.

One aspect that may contribute to such optimizations is that, in some embodiments, such as the embodiment depicted in FIG. 13, the exhalation and inhalation limbs of the pneumatic manifold may be arranged generally parallel and adjacent or relatively close to, each other, and the inhale and exhale ports may be arranged side by side with each other. Furthermore, in some embodiments, inhalation and exhalation flow with the air characterizer may be run in parallel.

Additionally, in some embodiments, such as the embodiment depicted in FIG. 13, a centrifugal blower may be provided that has an inherent ninety degree change of direction from intake to output. In such embodiments, the blower-based component may be oriented within the portable ventilator so that intake is through a side of the portable ventilator (as depicted in FIG. 13, the right side, but, as mentioned above, in an alternative left-to-right mirrored version, the intake could be on the left side instead, and likewise with other left or right components, arrangements and geometries). However, output from the blower-based component is in a direction ninety degrees different, in the direction from the back to the front of the device.

In some embodiments, various aspects of inhalation and exhalation flow are also simplified and optimized. For example, with regard to inhalation flow, output from the blower may flow in a straight path through the exhalation valve pressure regulator and the air characterizer and then out through the inhalation port to the breathing circuit. Furthermore, exhalation flow may return through the exhalation port and through the pneumatic manifold in a straight path before being exhausted to the right (or, in other embodiments, on the left) of the portable ventilator. Exhalation flow inside the air characterization module flows in a straight path, parallel to the output inhalation flow from the blower-based component. Airway pressure measurement gas flow and exhalation valve control gas flow also exits through front ports, further contributing to a relatively simple, and efficient, flow geometry.

In some embodiments, such as the embodiment depicted in FIG. 13, all (or, in other embodiments, many or most) device connection features or ports are placed (or placed and grouped relatively close to each other) on one side or face of the portable ventilator. This can provide advantages and solutions in terms of simplicity, minimizing over-crowding, efficiency and ease of use, including efficient access and ingress with regard to managing and arranging such connections. Similarly, in some embodiments, all breathing circuit or patient connection features or ports are placed (or placed and grouped relatively close to each other) on the front of the portable ventilator, also providing advantages and solutions in terms of simplicity, efficiency, minimizing over-crowding, and ease of use. Furthermore, in some embodiments, placing or grouping all breathing circuit connections on the front of the portable ventilator can enable or facilitate use of multiport connectors, as described further herein, such as may be used to, at once, correctly connect all breathing circuit connections between the portable ventilator and the breathing circuit.

In various embodiments, various alternative arrangements and geometries of the pneumatic manifold, portions and components thereof, and components coupled therewith, are provided. For example, as mentioned previously, left-to-right mirrored alternative arrangements are possible, including, for example, of the pneumatic manifold, arrangements of particular groups of components or portions thereof, orientation of individual components or portions thereof, or groups or individual other portable ventilator components. In a particular alternative embodiment, some aspects or portions of the pneumatic manifold or other portions or components of the portable ventilator may be left-to-right mirror image arrangements relative to the arrangements described herein, such as with regard to the embodiment shown in FIG. 13, even while others are not mirror image arrangements, such as with regard to the embodiment shown in FIG. 13.

In some embodiments, for example, the inhalation and exhalation limbs may be vertically spaced relative to each other as opposed to being horizontally adjacent and vertically at the same level. As one example, in some embodiments, the inhalation limb and path may be positioned below the exhalation path, with the inhale port being positioned under the exhale port. While such vertically spaced embodiments may not provide all of the advantages of, for example, a parallel arrangement, such non-parallel (or other non-adjacent or less adjacent) embodiments may provide advantages, and solve technical other problems. For example, vertically spaced embodiments may, in some cases or situations, minimize, or reduce or eliminate any potential risk of, any unintended gas exchange or absorption between the exhalation and inhalation limbs.

In some non-parallel embodiments, other changes may also be made relative to parallel embodiments. For example, in some non-parallel embodiments, space and positioning for one or more electronic circuit boards (or PCBs or one or more stacks of PCBs) may be provided on one or both sides, or partially on one or both sides, of the exhalation valve pressure regulator and the air characterizer. In various non-parallel embodiments, one or more electrical circuit boards may be positioned on the same side of both the exhalation valve pressure regulator and the air characterizer, may be positioned on different sides of the exhalation valve pressure regulator and the air characterizer, may be positioned orthogonally relative to the exhalation valve pressure regulator as compared with positioning relative to the air characterizer, or some combination thereof. In some embodiments, arrangements of electronic circuit boards may influence needed and provided ingress protection measures for the portable ventilator, such as providing such protection on the left side, right side, front, back, top and bottom, as opposed to potentially only on the left side, right side and front. In some embodiments, non-parallel embodiments may have notable use differences or needs relative to parallel embodiments, as well. For example, in some non-parallel embodiments, a user may need to ensure that both sides of the portable ventilator remain unobstructed.

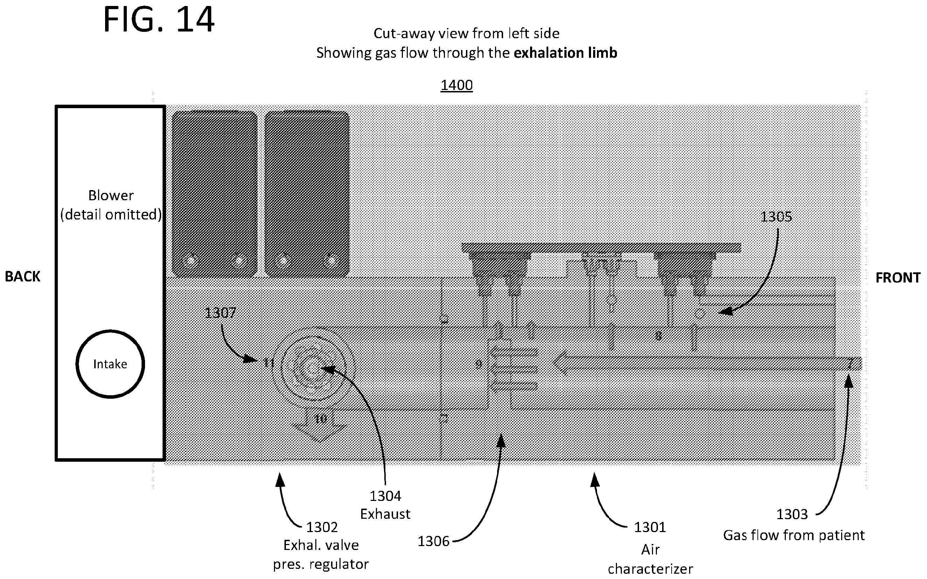

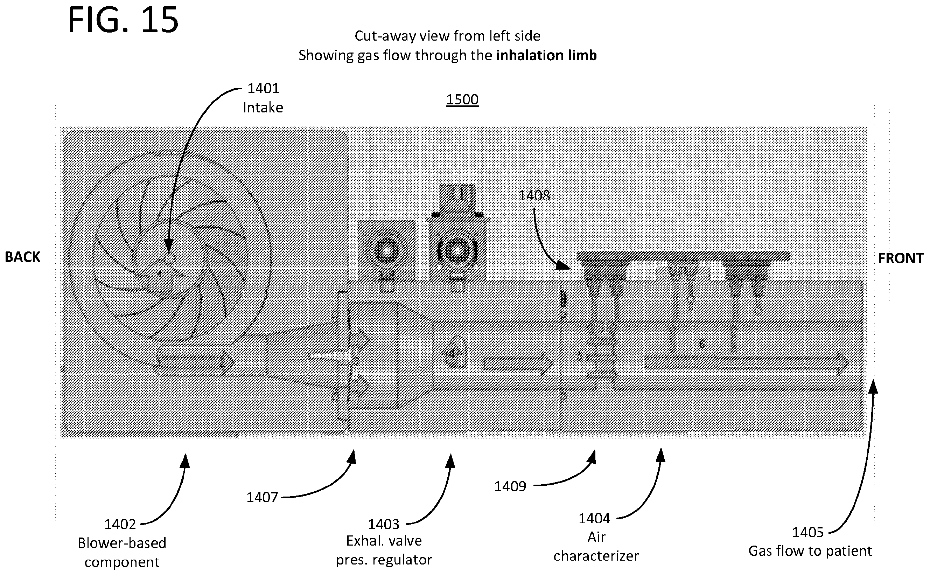

In FIG. 13, the broken lines 1208, 1209 show planes of cutaway views of an exhale limb, with examples of cutaway views 1400, 1500 of an exhale limb as shown in FIG. 14 and an inhale limb as shown in FIG. 15.

FIG. 14 illustrates an example side cut-away view 1400 of an exhalation limb of an example portable ventilator. Gas flow from the patient 1303 flows from the exhale limb of the patient circuit (not shown) into the pneumatic manifold, flowing through the exhalation limb, through the air characterizer 1301 and the exhalation valve pressure regulator 1302 and out of the exhaust 1304. As gas passes through the air characterizer 1301, one or more pressure sensors 1305 included in an electrical circuit board (or other component), coupled with (or part of) the air characterizer, are used to sense, and for measurement of, gas pressure. Furthermore, one or more flow sensors 1306, along with associated flow screens, are used in sensing, and for measurement of, gas flow rate.

FIG. 15 illustrates an example side cut-away view 1500 of an inhalation limb of an example portable ventilator. Gas flow is shown through the pneumatic manifold, flowing though the inhalation limb. Gas is drawn into the blower-based component 1402, passes through a one way valve 1407 and continues through the exhalation valve pressure regulator 1403 and the air characterizer 1404, and to the patient 1405 via the inhale limb of the breathing circuit (not shown). As gas passes through the air characterizer 1404, one or more pressure sensors 1408, such as are shown included in an electrical circuit board or other component coupled with (or included as part of) the air characterizer, are used to sense, and for measurement of, gas pressure. Furthermore, one or more flow sensors 1409, along with associated flow screens, are used in sensing, and for measurement of, gas flow rate.

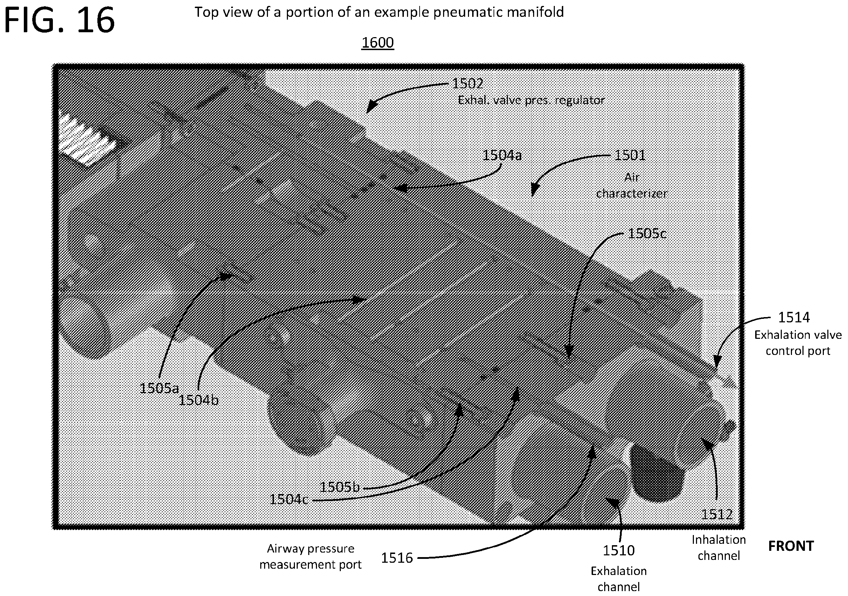

FIG. 16 illustrates a top view 1600 of a rigid pneumatic manifold and other components of an example portable ventilator. In particular, the view 1600 shows portions of the pneumatic manifold formed by the air characterizer 1501 (or a portion thereof) and the exhalation valve pressure regulator 1502 (or a portion thereof), as well as the blower-based component (or a portion thereof) (not shown). At numerous locations and areas of the upper surfaces of the pneumatic manifold formed by the air characterizer 1501 and the exhalation valve pressure regulator 1502, channeling 1504a-c can be seen (note that only an exemplary portion of the visible channeling is labelled). Additionally, inset areas, such as inset areas 1505a-c, are provided to allow for coupling components, such as screws, which may be used in fixedly joining portions of the pneumatic module.

The channeling 1504a-c may be formed at least in part within and by portions of the pneumatic manifold itself, allowing gas flow through the inhalation channel 1512 (including branched portions thereof) of the inhalation limb, and through the exhalation channel 1510 (including branched portions thereof) of the exhalation limb. It is noted that not all portions of all channeling can be seen in the depicted view 1600. Channeling 1504c runs beyond the airway pressure measurement port 1516. Channeling 1504a runs from the air characterizer 1501 through the exhalation valve control port 1514, which channeling can be seen running continuously between the exhalation valve pressure regulator 1502 and the air characterizer 1501, which are joined to allow continuous gas passage through the channeling 1504a. Furthermore, channeling, such as may include, for example, channeling 1504a-c, or other channeling, is included, as part of both the exhalation channel 1510 and the inhalation channel 1512, to allow gas flow to reach pressure and flow sensors of the electrical circuit board (not shown), which may be coupled with the air characterizer, for sensing of pressure and flow rate.

In some embodiments, portions of the channels and valves may be accurately and precisely machined, cut, aligned and/or sealed within or into portions and surface areas of the pneumatic manifold, including portions and surfaces of the exhalation valve pressure regulator 1502 and the air characterizer 1501, to allow the channeling configuration described to be accomplished and to allow proper, leak-free operation of the portable ventilator. Furthermore, portions of the pneumatic manifold, including portions and surfaces of the exhalation valve pressure regulator 1502 and the air characterizer 1501, are accurately and precisely machined, cut, aligned and/or sealed so as to join correctly to allow the channeling configuration described to be accomplished. Additionally, as part of the configuration, ambient reference ports may be routed to exterior ambient pressure or an interior ambient pressure reference volume.

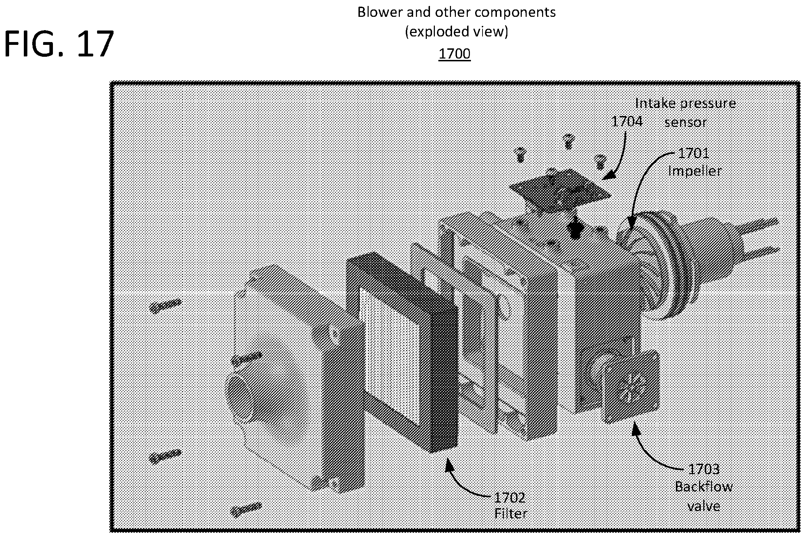

FIG. 17 illustrates an exploded view 1700 of a blower and other components of an example portable ventilator, including a centrifugal blower. The view 1700 shows, among other components, an impeller 1701, a backflow valve 1703, an intake filter 1702 and an intake pressure sensor 1704 mounted to a PCB.

The blower is configured to be adequate to allow the portable ventilator to provide mechanical ventilation with parameters such as gas flow rate and pressure as needed for delivering mechanical ventilation, including to maintain required patient airway pressures throughout breathing cycles, such as peak airway pressure (PIP) and PEEP. In some embodiments, a centrifugal blower is included that redirects an accelerated airstream at a ninety degree angle. As described previously herein, in some embodiments, the blower-based component includes a centrifugal blower with a low inertia impeller, allowing for sufficiently rapid increases and decreases in gas flow rate and pressure for gas delivered to the patient to deliver mechanical ventilation to the patient without need for a valve as previously described to divert a particular portion of the gas flow away from the patient. The low inertia impeller allows for high acceleration of the blower which enables the portable ventilator to quickly achieve inspiratory pressure targets (up to, for example, 55-75 or 65 cm H₂O) and flow targets (up to, for example, 125-175 L/min or 150 L/min) with relatively short inspiratory times (down to, for example, 0.2 - 0.4 or 0.3 sec). In some embodiments, the centrifugal blower has a rotor moment of inertia of approximately 6 g·cm² (or, e.g., in g· cm², between 0.1 and 30, between 0.5 and 30, between 1 and 30, between 2 and 30, between 3 and 30, between 3 and 20, between 3 and 15, between 3 and 10, between 1 and 10, between 3 and 9, between 3 and 8, between 3 and 8, between 3 and 7, between 3 and 6.5, between 3 and 6.25, between 4 and 7, between 5 and 7, between 6 and 7, between 6 and 6.5 or between 6 and 6.25), a blower weight of 240 g (or, e.g., between 100 and 1,000 g), maximum blower acceleration of 400 rpm/msec (or, e.g., in rpm/msec, between 100 to 1,000, between 200 and 1,000, between 300 and 1,000, between 400 and 1,000, between 100 and 600, between 100 and 500, between 100 and 450, between 100 and 425, between 100 and 400, between 200 and 425, between 300 and 425, between 350 and 425 or between 375 and 425), a maximum speed of 60,000 rpm (or, e.g., between 10,000 and 100,000 rpm), a maximum flow rate with zero back pressure of 435 L/min (or, e.g., between 200 to 800 L/min), a maximum flow rate into 80 cm H₂O of back pressure of 200 L/min (or, e.g., between 100 and 400 L/min), and an ambient temperature operating condition from -20 °C (or, e.g., between -40 to 0 °C) to 50 °C (or, e.g., between 40 to 60 °C).

In various embodiments, blowers are provided with various other characteristics and specifications. In some embodiments, the blower may include a direct current motor including Hall sensors. The blower may have a maximum speed of 60,000 rpm/sec (or, e.g, between 30,000 and 90,000 rpm/sec or between 50,000 and 70,000 rpm/sec, a maximum flow rate (no back pressure) of 435 l/min (or, e.g., between 300 and 500 l/min), a maximum flow rate (80 cm H2O back pressure) of 200 l/min (or, e.g., between 150 and 25o l/min), an oxygen (O2) concentration range of between 0-100%, an ambient temperature range (operating) of between-20 and 50 degrees C, an ambient temperature range (storage) of between -20 to 70 degrees C, a relative humidity range (noncondensing) of between 10 and 90%, a lifetime (L10 at 25 degrees C ambient temperature) of 20,000 hours, a sound pressure level @1 m of 47 dB(A), and a blower mass of 240g (or, e.g., between 200 and 300 g).

In some embodiments, sensor signals are used by the controller, along with one or more techniques, models or algorithms, in blower control. The controller may determine a blower motor control or actuation signal that is transmitted to the blower to actuate the blower accordingly. Such techniques may be used, for example, in determining target flow rate and target pressure for gas being delivered to a patient, as well as for determining a blower motor actuation signal for appropriate blower control based at least in part thereon.

In some embodiments, the controller determines a target flow rate and a target pressure for gas being delivered to the patient. Based at least in part thereon, the controller determines a signal for causing at least one of a flow rate and a pressure of gas being delivered to the patient to move toward (or, in some instances or embodiments, remain near or at) at least one of a target flow rate and a target pressure. This may include causing the flow rate and/or the pressure to increase or decrease, such as at a determined appropriate rate.

In some embodiments, the blower control signal, which is output to the blower, is determined to correspond with a blower motor torque or rotational velocity that is appropriate for such control. As such, the blower control signal may be a blower motor torque actuation signal, which signal, or group(s) of signals, may be or represent a command or commands. In some embodiments, the blower control signal is determined to be proportional to motor torque, rather than blower motor speed (although, in other embodiments, the blower control signal may be proportional to motor speed or one or more other parameters). Furthermore, in some embodiments, this determination may include determining an equilibrium speed determined based at least in part on a calculated balance between torque, work done on the gas during compression, and frictional losses, potentially among other things.

In some embodiments, the controller bases blower control at least in part on received or determined objectives or requirements relating to the mechanical ventilation being delivered to the patient. Additionally, in some embodiments, the rate (or rates) at which the flow rate or pressure may be increased or decreased corresponds with a determined associated gain. Furthermore, the gain, or gain scheduling, may be determined based at least in part on performance parameters or profiles, which may be based at least in part on a particular ventilation mode. In some embodiments, for example, such modes, and associated profiles, may include pressure or flow rate targeted assist-control (AC) mode, flow targeted high flow nasal cannula (HFNC) mode, pressure-targeted continuous positive airway pressure (CPAP) mode, and pressure-targeted synchronized intermittent mandatory ventilation (SIMV) mode, and/or others.

In some embodiments, in AC mode, the breathing cycle may be divided into inspiratory and expiratory phases. In the inspiratory phase, the blower (and blower motor) may be controlled to achieve goals of inspiration. Two sub-modes may include flow-targeted inspirations and pressure-targeted inspirations. In flow-targeted inspirations, the blower may be controlled to achieve a particular constant flow rate target delivered to the patient's lungs. The target may be computed for each breath to deliver an operator-specified tidal volume into the lungs during the fixed inspiration time. In bi-criterion control schemes, the pressure target is set to a maximum pressure based on safety. In pressure-targeted inspirations, the blower may be controlled to achieve a particular PIP, which may be specified by the operator, while the flow rate target may be set to a maximum flow rate based on safety. Since inspiratory times are typically short (such as approximately 0.3 second), fast response in blower control may be critical, and is achieved in embodiments of portable ventilators and control schemes described herein. This provides solutions to technical problems, associated with providing these modes of mechanical ventilation, with portable ventilators and blowers, examples of which are described in embodiments herein.

In some embodiments, in CPAP mode, the controller may jointly control the exhalation valve and the blower to achieve a target airway pressure that may be specified by the operator. CPAP mode may be a pressure-targeted blower control mode, as may be pressure-targeted AC mode. However, because the blower motor is controlled to operate jointly with the exhalation system, different performance profiles may be required to optimize the pressure response. Therefore, CPAP blower control may operate on a different gain schedule than AC mode.

In some embodiments, for SIMV mode, blower control may operate on similar bases as that of AC mode or CPAP mode. As such, from a blower control perspective, patienttriggered breaths in SIMV mode may be treated similarly to CPAP breaths, which may include providing less-invasive pressure support. Also, from a blower control perspective, time-triggered breaths in SIMV mode may be treated similarly to assist-control breaths.

In some embodiments, in HFNC mode, exhalation control, and the exhalation valve, are not active. The goal of this mode of ventilation may be to provide a steady flow of gas through the pneumatic circuit to the patient. From a blower control perspective, this may be achieved by operating the blower in a flow-targeted control mode. Because the breathing circuit may have different characteristics than the breathing circuit used in the other modes, HFNC blower control may use a different gain schedule than the assist-control flow-targeted mode, for example.

In some embodiments, the controller determines a target flow rate and/or pressure as well as a maximum or threshold flow rate and/or pressure, such as a maximum or threshold safe or appropriate flow rate and/or pressure, for gas being delivered to the patient. The controller may, for example, determine a blower motor control signal that causes the flow rate to move toward the target flow rate while maintaining the pressure at or below the maximum pressure. Similarly but conversely, the controller may determine a blower motor control signal that causes the pressure to move toward the target pressure while maintaining the flow rate at or below the maximum flow rate. In some embodiments, if a maximum flow rate is reached before a maximum pressure, or vice versa, the controller will control based on the maximum pressure (which may include treated the maximum as a target), and vice versa. In some embodiments, blower control is based on what may be viewed as a bi-criterion control scheme. In some such embodiments, both an airway pressure target and a flow rate target are input to, obtained or determined by, the controller, as criteria. The controller determines and selects which target criterion to control to (e.g., cause to be increased or decreased based on the target value), such as depending on which criterion is input, obtained or determined first during an inspiration (or based on one or more other factors or parameters). While controlling primarily to the target criterion (i.e., flow rate or pressure), a maximum relating to the other criterion (i.e., the other of flow rate or pressure) serves as a limit, but becomes the primary criterion should the maximum be reached.

In some embodiments, what can be viewed as an algorithmic two loop control scheme may be utilized in blower control. In some embodiments, an algorithmic two loop control scheme may include an outer loop and an inner loop. The inner loop may part of or within the outer loop, such as a nested loop within the larger outer loop, and the inner loop may iterate or execute more rapidly than the outer loop. The two loops may operate in an integrated fashion, with one loop providing output that is used as input to the other loop, to ultimately determine and output a result, such as a blower motor control signal, such as based on a pressure target, flow rate target, or both.

In one example of a two loop control scheme, based at least in part on a flow rate target and/or pressure target, an algorithmic outer control loop determines an appropriate blower motor speed target and, based at least in part on the determined blower motor speed target, an algorithmic inner control loop determines an appropriate blower motor torque and a corresponding blower motor torque actuation signal. However, various different types of two looo control schemes may be used in various embodiments.

However, in some embodiments, a simpler and more direct single loop control scheme, including only an algorithmic single control loop, is used. For example, in some algorithmic single control loop scheme embodiments, based at least in part on a flow rate target and/or a pressure target, or both, the algorithmic single control loop determines an appropriate blower motor control signal.

Single loop control schemes may have the advantages of being much simpler and/or direct than two loop control schemes. In some embodiments, single loop control schemes operate on an implicit working assumption that the blower motor is in equilibrium with the patient's lung dynamics. This assumption may not be precisely accurate. However, some embodiments include a recognition that such a potential slight inaccuracy relating to this assumption may have only a very small effect on control signal determination. This is particularly true when using a blower and blower motor with fast dynamics, as are included in embodiments herein. Furthermore, in some embodiments, in single loop control schemes, appropriate gain determination and scheduling further mitigate any small deviation that may result from the slight potential inaccuracy caused by this assumption. As such, in some embodiments, single loop control schemes may be advantageously used along with portable ventilators with blowers as described herein. As such, some embodiments provide a solution to the technical problem of the complication of using a two loop control scheme in blower control, by instead using a single loop control scheme along with a blower and other components as described in embodiments herein.

In some embodiments, in one or more blower motor control algorithms, an empirical model may be used in which pressure and flow rate changes caused by the blower are modelled as a function of factors or parameters including blower angular speed and an operating condition parameter that represents the operating condition of the blower motor. The operating condition parameter may be based at least in part on conditions associated with the inhalation and exhalation paths of the pneumatic manifold. The empirical model may model the relationship between parameters including pressure rise, flow rate and blower motor speed, for example.

Some embodiments include an electrically powered centrifugal blower or compressor. The blower or compressor may be placed within the pneumatic manifold such that it may provide a source for pressure sensing (e.g., via an intake pressure sensor, as described in embodiments herein), and may be used along with a backflow valve (as described in embodiments herein). Output gas flow may be directed to the breathing circuit and the patient, or to the exhaust, which may depend on the controlled status of the exhalation valve.

The intake filter 1702, such as a pleated filter (or, in other embodiments, a flat or foam filter) serves to filter out and protect both the patient and sensitive components of the portable ventilator from harm that might be caused by particulates or other debris. As an example, some embodiments include an intake filter 1702 that includes a HEPA grade filter placed between two rubber gaskets, inside of a housing. A filter cover may provide clamping pressure for seal. A pleated filter may offer an advantage of long service life, while a flat filter may provide an advantage of requiring less space. In some embodiments, a pleated filter may be used along with an elastomer housing that surrounds the pleated filter fabric, while, in other embodiments, other sealing components, such as one or more gaskets, may be used. Furthermore, in some embodiments, one or more hydrophobic filter materials may be used.

As depicted, the intake pressure sensor 1704 is a differential pressure sensor used to sense, and in measuring, the gas pressure on the intake side of the blower relative to ambient reference pressure, which ambient reference pressure may differ, for example, based on geographic location elevation.

In some embodiments, the backflow valve 1703 is used to prevent gas flow that has passed beyond the blower from returning through the blower, and may be necessary both for preventing backflow and maintaining PEEP.

In various embodiments, various types of backflow valves (or, in some embodiments, other devices) may be included (or used), which must function properly over required pressure and flow rate ranges. In some embodiments, a valve may be included such as a single piece valve, an elastomer valve, or a medical grade silicone single piece elastomer valve. Elastomer valves may have advantages other types of valves, such as spring-actuated or other types of backflow valves, including that elastomer valves may create less flow restriction. In some embodiments, an umbrella style backflow valve is included (or used). In some embodiments, an umbrella style valve may have advantages over other types of valves, such as duckbill style valves, including that an umbrella valve may provide a better backflow seal, better performance, such as at low pressures, and may be smaller or have a smaller footprint. However, in some embodiments, other types of valves may be included, including various types of one-way valves, check valves, control valves, non-control valves, spring-actuated valves, or duckbill style valves.

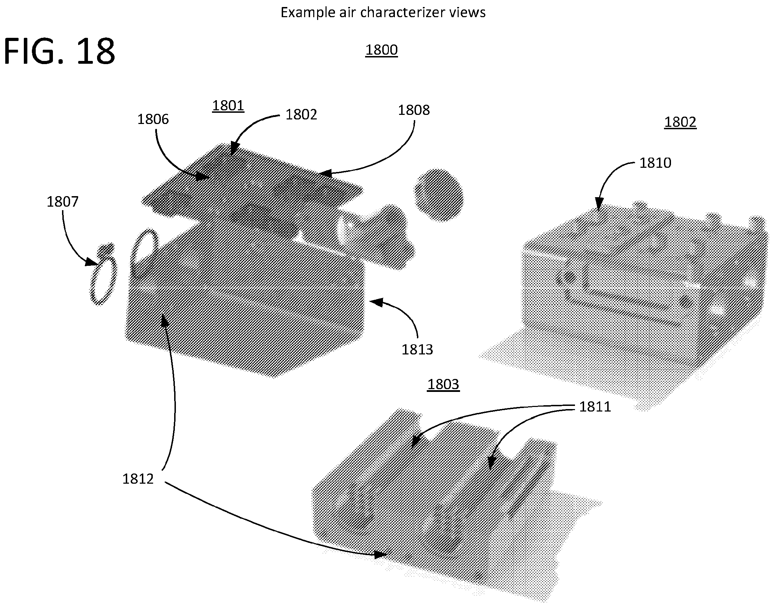

FIG. 18 illustrates views 1800 of an air characterizer and other components of an example portable ventilator. View 1803 shows a cutaway view of the air characterizer, with interior portions 1811 of the inhalation and exhalation channels visible. Also shown are coupling features 1812 for use in precise, leak-free joining of the air characterizer to an exhalation valve pressure regulator (not shown).

View 1801 shows a bottom perspective view of an air characterizer with channelbased sensor porting, as well as an electrical circuit board 1808 that couples with the air characterizer. As shown in view 1801, additional coupling components 1807, such as gaskets or O-rings, which may include radial sealing or face O-rings, may be used in providing leak-free joining of the air characterizer to an exhalation valve pressure regulator. This may assure, for example, a leak-free seal between continuous portions of the inhalation and exhalation channels that run between the air characterizer and the exhalation valve pressure regulator. On the opposite side 1813 of the air characterizer, similar coupling components are used in providing leak-free joining with the inhale port and exhale ports.

The electrical circuit board 1808 includes a number of sensors 1802. In the embodiment depicted, the sensors 1802 include (or are coupled with) small tubular coupling components 1806. View 1802 shows a top perspective view of the air characterizer, including other small tubular coupling components 1810. The electrical circuit board 1808 is configured to fixedly join with the air characterizer precisely, such that the small tubular coupling components 1806 of the electrical circuit board insert precisely into matching ones of the small tubular coupling components 1810 of the air characterizer. This may, in some embodiments, be used in forming small channels from the inhalation and exhalation channels of the air characterizer to each of the sensors 1802. In some embodiments, sealing components, such as O-rings, which may include radial or face O-rings, or gaskets, may be used in sealing individual sensor ports. In some embodiments or instances, however, a single sealing component may be used to seal multiple sensor ports. This porting allows pressure and flow sensors to be capable of sensing pressure and flow rate of gas passing through the inhalation and exhalation channels of the air characterizer. The arrangement allows for the sensing of pressure and flow rate of gas without the need for tubes connecting the ports of the pressure and flow sensors to the inhalation and exhalation channels of the air characterizer. The arrangement and precise matching provides a seal that is leak-free (or sufficiently leak-free) yet sufficiently flexible to remain leak-free under various potentially stressful conditions or circumstances, and associated potential stresses, such as minor torsions, expansions or contractions, such as may result from abrupt movement or dropping of the portable ventilator or potentially widely varying environmental conditions such as temperature range from -20 °C (range -40 to 0 °C) to 50 °C (range 40 to 60 °C). Furthermore, in some embodiments, flexible seals may be sufficiently flexible but not too flexible, such as by being so flexible as to negatively affect operation or create risk of leaks. As such, in embodiments, fixed yet flexible sealing or couplings may provide a particular range of flexibility as may be specifically required, or which provided range of flexibility falls into a suitable range in this regard, such as between a specified minimum and a maximum flexibility. Various other coupling and joining components and methods may be used in fixedly, or fixedly yet flexibly, joining components of the pneumatic manifold to each other, and in fixedly, or fixedly yet flexibly, coupling channels of the pneumatic manifold to sensors 1802 of the electrical circuit board 1808.

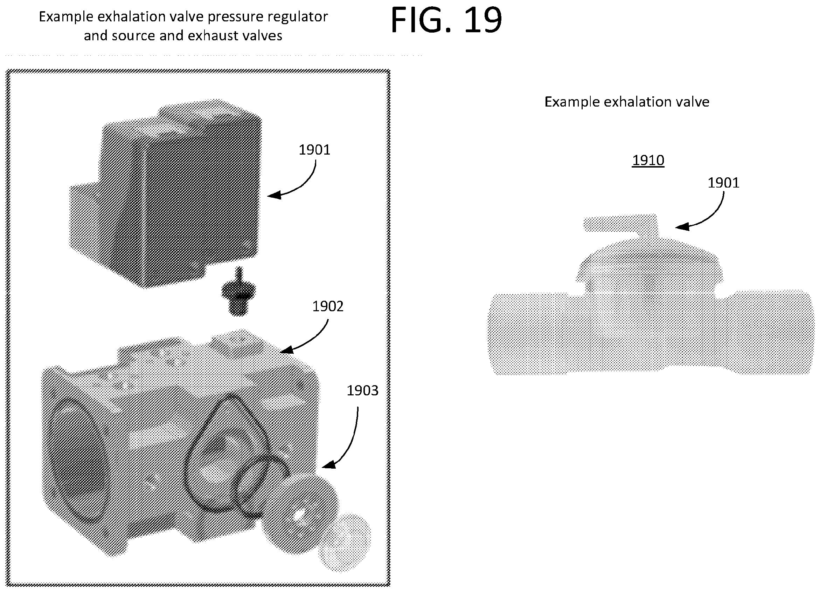

FIG. 19 illustrates an exploded view of an example exhalation valve pressure regulator 1902, example source and exhaust valves 1901, a view of an example exhalation valve 1910, and other components of an example portable ventilator. In some embodiments of a pneumatic manifold of a portable ventilator, the exhalation valve pressure regulator 1902 and the source and exhaust valves 1901 are configured to precisely join (in some embodiments, along with the use of one or more joining or sealing materials or components) such that channeling is formed to facilitate formation of a control path that leads continuously from the source and exhaust valves 1901 through the exhalation valve pressure regulator 1902 and ultimately to the exhalation valve 1910, which may be placed in the air characterizer or breathing circuit, for example. An exploded view 1903 of components of an example exhaust port is also shown.

FIG. 19 also illustrates an example exhalation valve 1910 which, in various embodiments, may be placed in the pneumatic manifold, such as in the air characterizer, or in the patient breathing circuit. Various other types of exhalation valves may be used in other embodiments, and various types of exhalation valve control may also be used, accordingly.

The exhalation valve 1910, as depicted, is pneumatically actuated. Exhalation control pressure is applied to a control port 1901 in the top area of the exhalation valve 1910 and pressurizes one of the two sides of the diaphragm of the exhalation valve 1910. The other of the two sides of the diaphragm is divided into two annular sections. One section is exposed to exhalation valve control pressure while the other section is exposed to ambient pressure. The balance or difference in pressures between the exhalation valve control pressure and the ambient pressure determines the position of the diaphragm and the effective resistance of the exhalation valve. In the embodiment depicted, the source and control valves 1901 may be arranged in a T-circuit with the control port 1901 of the exhalation valve 1910 to form an enclosed volume which holds the exhalation valve control pressure. In various embodiments, various different exhalation valve control techniques, models and algorithms may be used by the controller to control the exhalation valve 1910. As described herein, in some instances, the exhalation valve 1910 may be controlled jointly with the blower, such that, for example, the control of each takes into account the control and status of the other, so that control of both together causes the desired results or operation of the portable ventilator or one or more of its components.

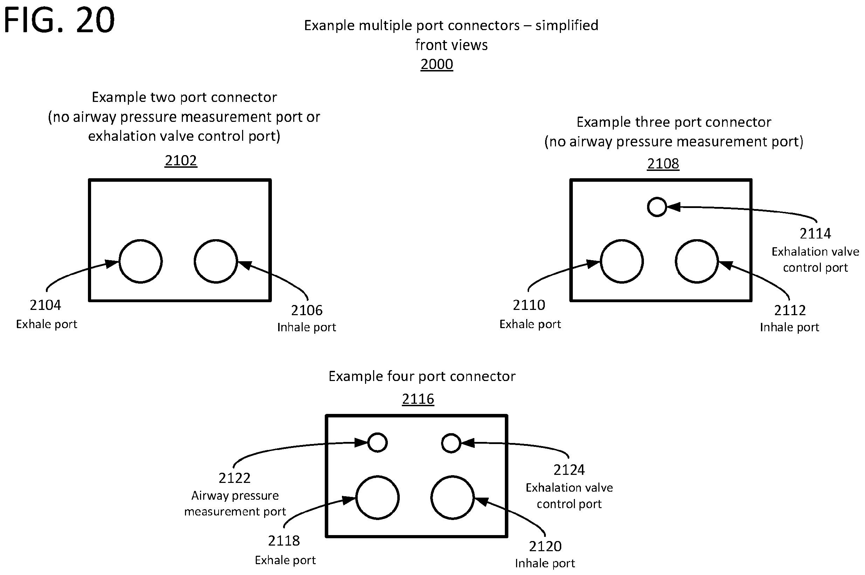

FIG. 20 includes block diagrams of example multiple port connectors for use with example portable ventilators. In each of the embodiments shown, each of the ports leads out of the portable ventilator and through the breathing circuit (not shown).

A two port connector 2102 is shown, including an exhale port 2104 and an inhale port 2106, but not including, for example, an airway pressure measurement port or an exhalation valve control port. Such a connector 2102 may be used in embodiments of a portable ventilator in which, for example, the exhalation valve is placed within the pneumatic manifold and pressure and flow sensors are placed within the pneumatic manifold, such as in the air characterizer, so that patient airway pressure can be determined or calculated without a pressure tap in the breathing circuit.

A three port connector 2108 is also shown, including an exhale port 2110, an inhale port 2112 and an exhalation valve control port 2114, but not including, for example, an airway pressure measurement port. Such a connector 2108 may be used in embodiments of a portable ventilator in which, for example, the exhalation valve is placed within the breathing circuit, but pressure and flow sensors are placed within the manifold, such as in the air characterizer, so that patient airway pressure can be determined or calculated without a pressure tap in the breathing circuit. In another example of a three port connector, the exhalation valve control port 2114 could be omitted, and, instead, an airway pressure measurement port could be included. Such a three port connector might be used in embodiments of a portable ventilator in which, for example, the exhalation valve is placed within the pneumatic module, and a pressure tap is included within the breathing circuit for use in measurement of patient airway pressure.

A four port connector 2116 is also shown, including an exhale port 2118, an inhale port 2120, an airway pressure measurement port 2122 and an exhalation valve control port 2124. Such a connector 2116 may be used in embodiments of a portable ventilator in which, for example, the exhalation valve is placed within the breathing circuit, and a pressure tap is included within the breathing circuit for use in measurement of patient airway pressure. Other embodiments of connectors may include and exclude different ones and combinations of ports, and may include one or more other ports not shown.

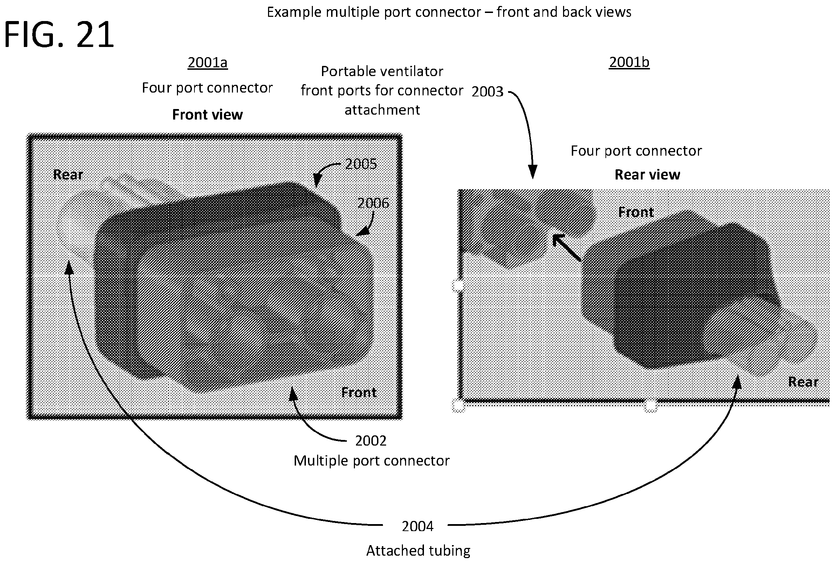

FIG. 21 illustrates views of an example four port multiple port connector 2002 for use with an example portable ventilator, which may be an example of the four port connector 2116 shown in FIG. 20. A front perspective view 2001a and a rear perspective view 2001b are shown. In each of the views 2001a, 2001b, tubing 2004 attached to the connector is shown. Although only a portion of tubing is shown, the tubing continues to couple with the patient breathing circuit. In the embodiment shown, the connector 2002 includes a front portion 2006 that attaches to the four ports at the front of the portable ventilator 2003 shown in the rear view 2001b, providing for a flexible but leak-free connection allowing gas to pass through each of the ports and tubing. In the embodiment shown, the connector 2002 also includes a central portion 2005, such as may be composed or of rubber or another flexible material, that connects to the front portion 2006. In some embodiments, the connector 2002 may be disposable and replaceable by a new connector, such as after one use or several uses.

In some embodiments, in practical application, the connector 2002 may be attached to the patient circuit prior to attachment to the portable ventilator. Prior to use of the portable respirator with a patient, a user/care provider attaches the connector 2002 to the portable ventilator, thereby, in one overall action or step (in a single overall connection), attaching the breathing circuit correctly to each of the four ports. This can provide simplification and save time in a potentially time-critical situation, such as relative to making connections to ports individually. Additionally, the connector 2002 may be configured to only be capable of attachment if the ports of the connector 2002 and the ports 2003 of portable ventilator are correctly aligned or matched to that the connection is proper, or may provide some indication or confirmation when proper alignment or connection is made (or not made), thereby ensuring proper connection (or warning of improper connection) and reducing or eliminating risk of user error in this regard. Furthermore, in some embodiments, a portable ventilator may be configured to automatically detect a type of use (e.g., adult or pediatric), and provide an indication accordingly. This may be of particular benefit, for example, in out-of-hospital or field settings, and for portable ventilator operators who may have relatively little training or experience, and who may be subject to urgency and many distractions, Furthermore, in some embodiments, connectors may be provided that are different for various different uses or indications, such as adult or pediatric (which may have sizing differences, for example), conventional (which may include a breathing circuit exhalation limb), High Flow Nasal Canula, or HFNC (which may not include a breathing circuit exhalation limb, and therefore may not include an exhale port). In some embodiments, connectors, or portable ventilators, may include text, graphical or other markings to indicate and identify such information and uses.

In the embodiment depicted in FIG. 21, the front ports 2003 of the portable ventilator may be described as recessed male, in which, when a connector is attached, the front ports extend into recessed connecting regions of the connector. In other embodiments, however, other configurations for the front ports 2003 are possible, such as flush female, in which the front ports include openings flush on the surface of the portable ventilator that includes the openings, and in which connecting portions of the connector extend into the openings. This and other potential other embodiments may provide advantages, such as elimination of potential contamination spots on the outside of the ports, providing greater or easier access for cleaning the inside areas of the portable ventilator gas flow channels, and use of more complex patterning and surface geometries on the connector, for which cleaning may be easier or less problematic than the portable ventilator, particularly if the connector is disposable.

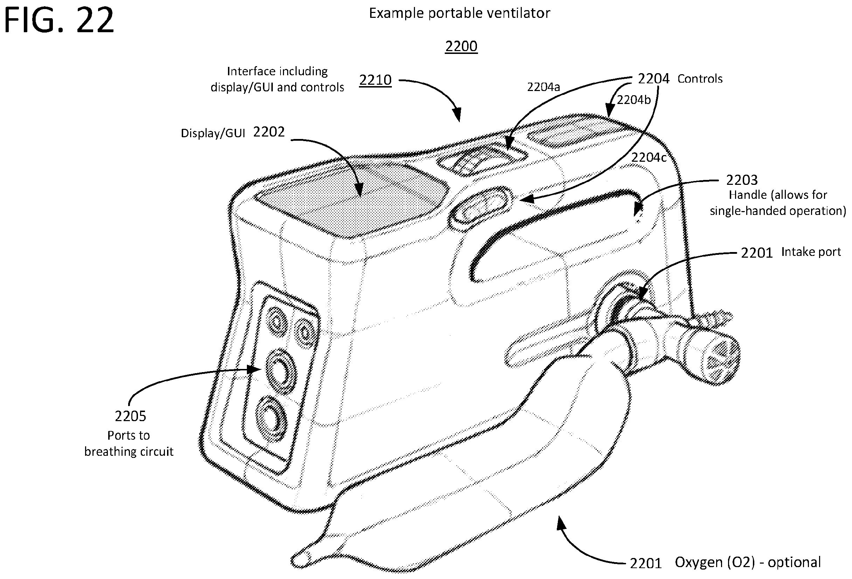

FIGs. 22-27 illustrate various embodiments of example portable ventilators. In various embodiments, portable ventilators are provided with a wide range of variations in features including shape, size, handle, and interface including display/GUI and controls, among other things. FIGs. 22-27 provide a few of many possible variations and combinations of features.

In the embodiments shown in FIGs. 22-27, each of the portable ventilators can be operated with a single hand, including grasping the portable ventilator and controlling its position and viewing at least some portion of a display/GUI, as well as operating one or more of the controls, potentially simultaneously. Each of the embodiments shown includes some form of handle and multiple controls. In each embodiment, for various circumstances in actual applications and use, the portable ventilator may be grasped, such as to allow for positional control and/or support of at least a portion of the weight of the portable ventilator, by one or more fingers (hereinafter called grasping finger(s)) of a single hand of the operator (which could be any individual finger (where a finger can include any of the five fingers including the thumb) or any combination of two or more fingers). In the embodiments depicted, while the portable ventilator is being so grasped, the operator is capable of seeing at least a portion of at least one display/GUI of the portable ventilator. Additionally, while the portable ventilator is being so grasped, the operator is capable of operating a first one or more controls using one or more fingers (hereinafter called first finger(s)) of the single hand (which may be any individual finger or any combination of two or more fingers), where the first finger(s) are different than the grasping finger(s). Furthermore, in some embodiments, while the portable ventilator is being so grasped and while the operator is operating the first one or more controls, the operator is capable of operating a second one or more controls using one or more fingers (hereinafter called second finger(s)) of the single hand (which may be any individual finger or any combination of two or more fingers), where the second finger(s) are different than the first finger(s) and the grasping finger(s). It is to be noted, however, that, in some embodiments, the operator grasps the portable ventilator with one or more gasping fingers and operates the one or more first controls, without necessarily operating, or being able to operate, one or more second controls, such as with one or more second fingers of the single hand.

In some embodiments, the portable ventilator may include one or more lights, lighted areas, lighted controls or other lighted features (hereinafter called lighted portions). In various embodiments, lighted portions may be white, any other color, multiple colors, or may include sub-portions of different colors, or may change colors. Additionally, in some embodiments, lighted portions (which may include more than one at a time) may always be lighted, may sometimes be lighted, may intermittently be lighted, may flash, or may light to display particular patterns. Patterns may be displayed including at one time or with different display features over a period of time, including patterns of portions or sub-portions being lighted or unlighted, patterns of colors, flashing or patterns of lighted and unlighted periods of particular lengths of time or varying particular lengths of time, etc. In some embodiments, lighted portions or associated patterns, and the particulars thereof, may vary based on various factors and circumstances that may relate, for example, to the portable ventilator, its condition or operation, the patient, the operator, or other factors. In some embodiments, lighted portions may convey particular conditions that may signal or warrant operator attention, or a particular type, degree or urgency of operator attention, such as alarms or alerts relating to portable ventilator operation, the patient or the operator. As some examples, conditions so conveyed may include signaling low or failing battery, estimated amount of battery life or operation time remaining, malfunction, possible malfunction, remote or internal control, or a concerning condition of the patient (such as low oxygen, low heart rate, low heart rate, etc.) or another physiological parameter. Conditions so conveyed may also include various types of alarms. In some embodiments, the alarms may include an indication of the urgency of the alarm, such as low priority or advisory, medium or high priority alarms. In some embodiments, alarm types may include, for example, patient safety alarms, such as may relate to, e.g., pulse oximetry monitoring, or patient circuit or exhalation valve issues. Alarms may also include environmental and use alarms, such as may relate to, e.g., device inputs, external power, battery, oxygen supply, fresh gas intake, barometric pressure and altitude issues. Alarms may also include portable ventilator self check alarms, such as may relate to, e,.g., internal communication, sensor operation, pneumatic system operation, pulse oximeter operation, or preventative maintenance issues.

As a further example, conditions so conveyed might concern a condition or physiological parameter of the operator, in instances in which operator physiological parameters may be monitored by the portable ventilator (such as by sensors on the handle, for instance) or otherwise. Of course, any of the foregoing types of information might also be conveyed via any or various portions of a display/GUI, such as via text, numbers, graphs, images, etc., as well as potentially audio via a speaker of the portable ventilator, or may be conveyed by combinations of display or media, such as may be coordinated, provide an overall multi-display or multimedia pattern, etc.

In some embodiments, the portable ventilator is configured such that a user grasps the portable ventilator by the handle with one or more fingers (which may include the thumb) of a single hand, while also, at least at some times, operating controls of the portable ventilator with one or more other fingers of the same single hand. In various embodiments, the handle may form an aperture through the housing, or may protrude from (or as) a portion of the housing, or may be separate from but fixed to the housing.

In various embodiments, the portable ventilator may be configured such that various combinations of fingers may be used to grasp the portable ventilator (one or more grasping fingers) and one or more other fingers may be used to operate controls (one or more first fingers). In various embodiments, for example, the grasping fingers may include: the thumb; the index finger (first finger from the thumb); the index finger and the middle finger (second finger from the thumb); the index finger, the middle finger and the ring finger (third finger from the thumb); the index finger, the middle finger, the ring finger and the pinkie (fourth finger from the thumb); the middle finger, the ring finger and the pinkie; the ring finger and the pinkie; the pinkie; or other fingers or combinations of fingers. In various embodiments, for example, the first fingers (used to operate one or more controls) may include the middle finger; the index finger; the middle finger and the index finger; the thumb; the thumb and the index finger; the thumb, the index finger and the middle finger; or other fingers or combinations of fingers.

For example, in some embodiments, a user may grasp the portable ventilator by the index fingers, or the index finger and one or more of the middle finger, the ring finger and the pinkie (such as, for example, by placing the grasping fingers around a portion of the bottom of the handle), while operating one or more controls using the thumb, the index finger, or the thumb and the index finger. In some example embodiments, the user may grasp the handle by the thumb (such as, for example, by placing the thumb around a portion of the bottom of the handle) while operating one or more controls using the index finger, the middle finger, or the index finger and the middle finger. Furthermore, in various embodiments, controls may be placed in various locations, such as on, or proximate to, the handle, so that, while grasping the handle by one or more grasping fingers, the user can also operate controls with one or more first fingers, such as by pressing or manipulating controls that may be located in various areas of the handle (such as the top or sides) as well as elsewhere on the housing (such as the top, sides, front or back).

In various embodiments, the grasping fingers may be used, for example, to grasp the portable ventilator, to grasp and hold in place the portable ventilator (such as while it is placed on a surface), to grasp and position or reposition the portable ventilator (such as while it is placed on a surface), to grasp and support a portion of the weight of the portable ventilator (such as while a portion of the weight of the portable ventilator is supported in some other way, such as by a surface or another hand, for example), or to grasp and support the entire weight of the portable ventilator (such as by holding the portable ventilator so that it is not touching or supported by any surface or in any other way).

In various embodiments, various types of controls may be provided, including physical and display-based controls, such as, for example, buttons and dials (which may include a knob). For example, various particular types of controls may include a pressable, shiftable, turnable, rotatable or moveable control, a scrollwheel, a soft key, or others. Various sized controls may be provided, from a fraction of an inch in length, width or height, such as 1/10 inch, 1/4 inch, 1/2 inch or 3/4 inch, to 1, 2, 3 or more inches. In various embodiments, various numbers of controls may be provided, such as 1, 2, 3, 4, 5, 6-10, 11-15 or 16-20 controls. Furthermore, in some embodiments, controls may be grouped close together in one or more different areas, such as 1, 2, 3, 4, 5 or 6-10 different areas. Such areas may include, for example, a top area of the handle, a side area of the handle, a bottom area of the handle, or an area of the housing other than the handle, such as a side or top area of the housing other than the handle.

Furthermore, one or more display areas may be provided, such as 1, 2, 3, 4, 5 or more display areas, and, in some embodiments, one or some of the controls may be provided on the display area as display based controls, for example. Display areas may include, for example, one or more display areas on the handle and one or more display areas on the housing other than the handle. A display area may be of any of various sizes. For example, a display are may have a length or width from a fraction of an inch in length, width or height, such as 1/10 inch, 1/4 inch, 1/2 inch or 3/4 inch, to 1, 2, 3, 4, 5, 6 or more inches. In various embodiments, a display area or control may be flush with a handle or housing surface, or may be raised or incut/depressed relative to a surrounding housing surface.

In some embodiments, controls, or areas with controls, may be located proximate to or on the handle, and may be located for practical or convenient access by one or more particular fingers (one or more first fingers) while the handle is being grasped by one or more grasping fingers, and the locations may be optimized based in part on the particular first fingers and grasping fingers, so that access is optimized relative to the anticipated positions of the fingers. For example, in some embodiments, controls may be located within a short distance from a handle opening, or a handle grasping area, such as from a fraction of an inch to several inches, such as 1/10 inch, 1/4 inch,1/2 inch or 3/4 inch, 1, 2, 3, 4, 5 or 6 inches, for example.

Furthermore, display areas may be located proximate to one or more controls or control areas, such as within a fraction of an inch to several inches, such as 1/10 inch, 1/4 inch, 1/2 inch or 3/4 inch, 1, 2, 3, 4, 5 or 6 inches, for example. Furthermore, display areas may be located for convenient, unobstructed or optimized viewing while the user is grasping the handle and operating the controls with the single hand. For example, in some embodiments, a display area may be located on the top of the handle, on the top of the handle in front of an anticipated grasped area of the handle, on a side of the handle, on the top of the handle in the back of the handle behind an anticipated grasped area of the handle, on top of the housing in front of the handle, on a side of the housing, or on other areas of the handle or the housing.

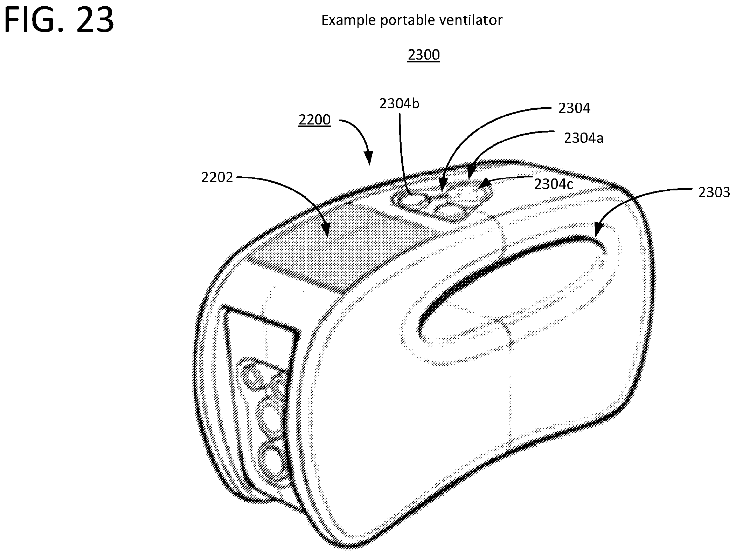

In the embodiment of a portable ventilator 2200 depicted in FIG. 22, four ports 2205 to the breathing circuit are provided, and an oxygen source 2201 is included and attached to the intake port 2201. The portable ventilator 2200 also includes an interface 2210 including at least one display/GUI 2202 and one or more controls 2204. The portable ventilator 2200 includes a handle 2203 that is formed as an opening through the housing of the portable ventilator, at least one display/GUI 2202, and controls, at least including controls 2204. As depicted, the controls 2204 include buttons, lighted buttons or lighted portions 2204b, 2204c and a physical scroll wheel 2204a. The scroll wheel 2204a may, for example, include features such as including texture for more secure and precise scrolling, being pressable to make or confirm as selection, being pressable one or multiple times quickly to make some particular confirmation or selection, being movable to some degree (such as front, back, left, or right) to make selections, etc. In the embodiment depicted in FIG. 22, as well as any of the embodiments depicted in FIGs. 23-27, the portable ventilator may have additional display/GUI features and controls that are not visible in the views shown. These may include controls on a portion of the handle of the portable ventilator, and may include controls that are not visible, or sometimes not visible, during operation of the portable ventilator and may be operated based at least in part on touch, felt position, haptic sensation, audio prompts, etc.

The embodiment of a portable ventilator 2300 shown in FIG. 23 includes a handle 2303, an interface 2200 including at least one display/GUI 2202 and one or more controls 2304. As can be seen, the portable ventilator 2300 shown in FIG. 23 differs from the portable ventilator 2200 shown in FIG. 22 in aspects including shape, pattern and size, overall and of particular features, including the handle 2303, display/GUI 2202 and controls 2304, providing an example of the great variety that is possible in these regards, in various embodiments.

In the embodiment depicted in FIG. 22, the handle 2203 forms an aperture through the housing of the portable ventilator 2200. Controls 2204 are located on a top area of the handle 2203 (or, in other embodiments, on the housing above the handle 2203), on the side of the handle 2203 (or, in other embodiments, on the side of the housing near the handle 2203), conveniently located close to a grasping area of the handle 2203 so that they can be operated by the single hand while the single hand is also grasping the handle 2203. The display/GUI 2202, which has a mostly rectangular cross-sectional (or mostly rectangular cuboid) shape but with a narrowing area toward the front, is located on the top of the housing in front of the handle 2203, conveniently located so as to be visible while the portable ventilator 2200 is being grasped and the controls 2204 are being operated. Overall, the housing has a roughly rectangular cross-sectional shape.

In the embodiment of a portable ventilator 2300 shown in FIG. 23, the controls 2304 include multiple types of buttons or other physical controls 2304a, 2304b. For example, control 2304a may be a flat, turnable knob in which a portion or displayed portion 2304c of the knob being currently turned to a particular position, such turned closest to the body of the operator, indicates a particular selected setting, etc. In other embodiments, controls, such as dials or knobs, may have markings to indicate different settings, or markings may be provided separately but proximate to the control, or no markings may be provided, for example.

In the embodiment depicted in FIG. 23, the top surface of the housing is slightly curved, and the display 2202 and the controls 2304 are also slightly curved to generally conform to the housing, with the display 2202 being located just in front of the controls 2304, and both the display 2202 and the controls 2304 being located close to the grasping area of the handle 2303. Additionally, the handle 2303 has a symmetric, oval shape. Furthermore, all of the controls 2304 are located on the top of the housing. Overall, viewed from a side, the housing has a slightly curved shape.

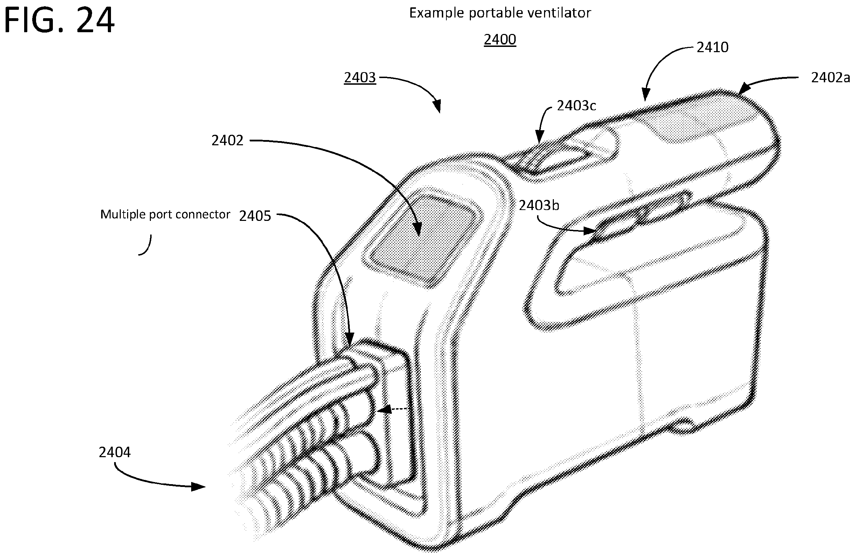

The embodiment of a portable ventilator 2400 shown in FIG. 24 includes an interface 2403 including at least one display/GUI 2402 and a handle 2410, and is shown with an attached four port multiport connector 2405, with a portion of the attached breathing circuit tubing 2404 also shown. The embodiment shown in FIG. 24 includes a handle 2410 that may be formed from a portion of the housing but not as an aperture through the housing. In the embodiment depicted, the handle 2410 includes controls 2403 including buttons, lighted buttons or lighted portions 2403a, 2403b, and a scroll wheel 2403c.

In the embodiment depicted in FIG. 24, the handle 2410 protrudes from a portion of the housing, extending at the top and toward the back of the portable ventilator 2400. As depicted, controls 2403 are included on the side and top, toward the front, of the handle 2410. Furthermore, the display 2402 is located on a portion of the housing in front of the handle 2410 and the controls 2403. Overall, the housing has a mostly roughly rectangular cross-sectional shape, but with a portion of the top front, including the display 2402, being slanted, and has the protruding handle 2410.

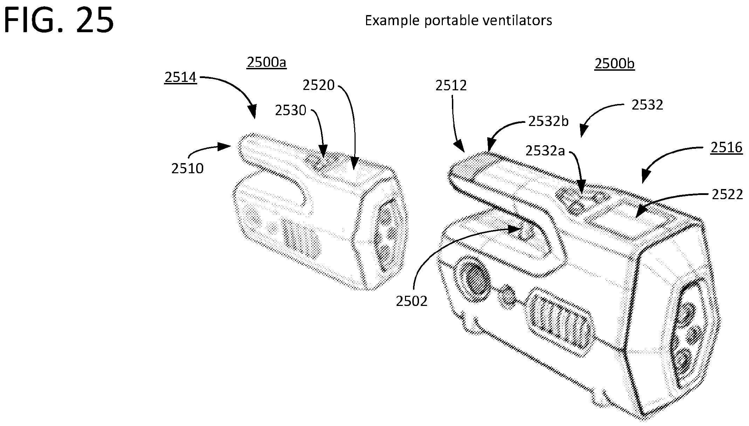

FIG. 25 shows two example embodiments of a portable ventilator 2500a, 2500b, each including a handle 2510, 2512 and an interface 2514, 2516 including at least one display/GUI 2520, 2522 including one or more controls 2530, 2532a, 2532b. Embodiment 2500b includes some visible differences relative to embodiment 2500a, including in overall shape surface patterning, and, in the embodiment 2500b, the controls 2532 include an additional button, lighted button, or lighted portion 2532b relative to embodiment 2500a. Furthermore, embodiment 2500b includes a handle 2512 that includes an additional grippable portion 2502, such as may be positioned and used between two grasping fingers, for example. In various embodiments, characteristics various shapes, patterning, control and display configurations may be optimized to best suit particular embodiments of the portable ventilator, as well as conditions or characteristics of an anticipated context, application, use or user. For example, different sizes and shapes of portable ventilators may be best optimized for particular environments and uses, and handles, controls and displays may be optimized to suit particular types of portable ventilators as well as be most usable and/or visible in anticipated applications or users (e.g., users of different hand sizes, or left or right handed uses, for example).

In embodiments depicted in FIG. 25, the handles 2510, 2512 protrude from the housing at the top and back of the device. Controls 2530, 2532a are located on the top front area of the handles 2510, 2512 (or, in some embodiments, on the housings near the handles 2510, 2512), while control 2532b is located on the top and at the back of the handle 2512, and the displays 2520, 2522 are located just in front of the controls 2530, 2532a. Overall, the housings have a mostly roughly rectangular cross-sectional shape, but are slightly thicker in the vertically central portion, and have the protruding handles 2510, 2512.

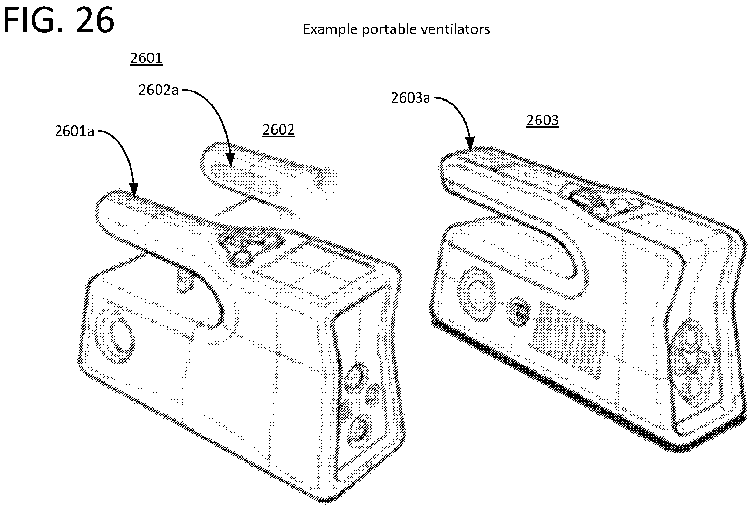

FIG. 26 shows multiple example embodiments of portable ventilators 2601, 2602, 2603, with differences including various overall shape and handle differences, as well as differences in the shape and position of button, lighted button or lighted portion 2601a (shaped as a narrow rectangle, located on the top and back of the protruding handle), 2602a (shaped as a narrow rectangle with curved ends, located on the side of the protruding handle), 2603a (shaped as a wider rectangle, located at the top of the protruding handle). Overall, the housings have a mostly roughly rectangular cross-sectional shape, but with slightly narrower vertically central portions, and with protruding handles.

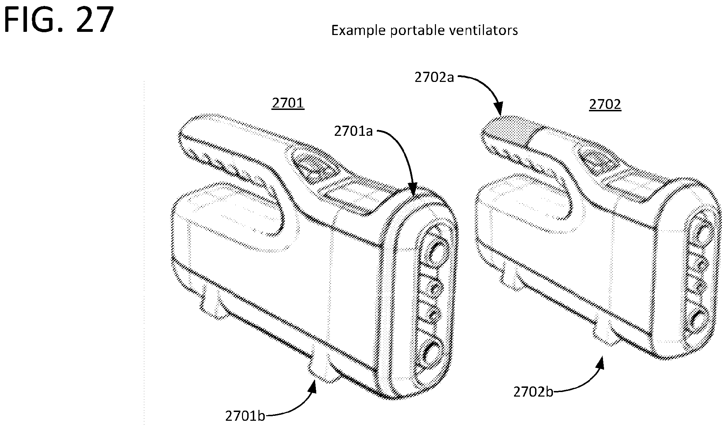

FIG. 27 shows two example embodiments of portable ventilators 2701, 2702 with differences in the shape and position of button, lighted button or lighted portion 2701a, 2702a. Each of the embodiments shown in FIG. 27 includes visible "feet" 2701b, 2702b, or portions for use in secure, level and/or slightly elevated/lifted placement of the portable ventilator 2701, 2702 on a surface or other device, for example.

FIGs. 28A-C illustrate simplified example display/GUI features of example portable ventilators. In various embodiments, various included controls, such as those shown in FIGs. 28A-C, may be included or placed in various locations or controls areas on a portable ventilator or its housing, which may include one or more physical controls areas, one or more display/GUIs, a handle, or others. It is noted that, in various embodiments of portable ventilators, a great variety of display/GUI features are possible The examples provided in each of FIGs. 28A-C may represent a full set of display/GUI features for a particular example portable ventilator, or a partial set, and some embodiments of portable ventilators may use various combinations of particular features shown in each FIGs. 28A-C, and/or other features, etc. Various of the features shown in FIGs. 28A-C may, in various embodiments, be located in various locations of a portable ventilator, such as a handle or elsewhere.

FIG. 28A illustrates a simplified example of included controls 4000 of an example portable ventilator 4000, including only ventilation related settings controls including a power switch 4004 and communication indicator light 4006 (or, in other embodiments, the communication indicator light could be, e.g., a remote control indicator light), which, in various embodiments, may be placed in various locations on the portable ventilator. The communication indicator light 4006 (or remote control indicator light) may be lit when communication with a remote control device or system is present (or, if a remote control indicator light, when remote control is occurring), or to indicate, for example, wired connection, wireless connection, Bluetooth connection, or one or more other forms or protocols of connection.

In various embodiments, a display/GUI of the portable ventilator 4000 may include, e.g., a liquid crystal display (LCD), organic LED (OLED), microLED, or other type of display. In some embodiments, the display/GUI may include touchscreen aspects. Furthermore, in some embodiments, the display/GUI may be user selectively hidden or displayed, and, when hidden, may provide some indication, such as a visible or flashing light, if user attention is needed.

In some embodiments, the display/GUI of the portable ventilator 4000, or one or more other portions or components of or included with the portable ventilator 4000, may be modular, and may be attachable to and removable from the portable ventilator 4000, such as an attachable and detachable LCD display module, a handle or a connector. Furthermore, in some embodiments, various different types of display modules may be attached to the portable ventilator 4000, such as display modules of varying complexity, so that the portable ventilator 4000 may be customizable by a user with regard to the display/GUI. Furthermore, in some embodiments, the display/GUI of the portable ventilator 4000 may itself be modularized and include multiple modular components, such as a top, middle and lower components, for example. Any of the components may be attachable and detachable, allowing mixing of different display components and associated customization of the overall display/GUI of the portable ventilator 4000.

In some embodiments, patient circuits of, or coupleable with, the portable ventilator 4000 may be configured such that all portions of the patient circuits are attached, and they attach as a modular unit to the portable ventilator 4000.

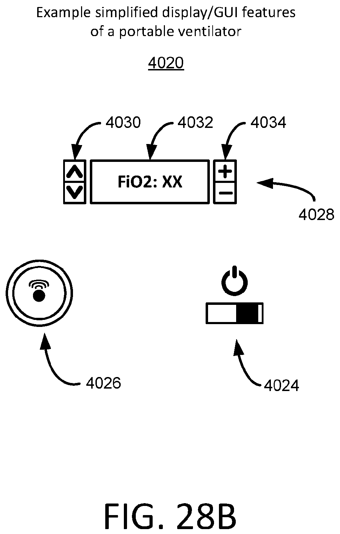

FIG. 28B illustrates simplified example included controls 4020 of an example portable ventilator, including ventilation related settings controls such as a power switch 4024, communication or remote control light 4026 (which features may, for example, be similar to the corresponding features 4004, 4006 as depicted in FIG. 28A), and display/GUI features or other display/GUI features 4028.

The display/GUI features may include a central display/GUI area 4032, up and down arrow selection buttons 4030 and plus and minus selection buttons 4034. In the embodiment shown, a user can use the up and down arrow selection buttons 4030 to scroll through multiple items for display, some or all of which may be subject to potential setting or adjustment by the user. Scrollable items may include, for example, a mode or modes of operation (e.g., one or more non-ventilation modes or one or more ventilation modes) and various parameter settings, such as patient physiology related parameter settings or ventilation related parameter settings, among other things. In the example shown, the current Fi02 setting of the portable ventilator 4020 is shown in the central display/GUI area 4032 (no actual setting is shown, but, in some embodiments, FiO2 may vary between, e.g., 21% and 100%). As an example, a user may have used the up and down arrow selection buttons 4030 to select the Fi02 parameter setting for display. Once displayed, the user may be able to change the displayed current setting by using the plus and minus selection buttons 4034 to increase or decrease it (potentially subject to potential limits, confirmation, etc.). In various embodiments, the changed displayed setting may be implemented without further indication to the user (potentially unless user confirmation is required), or some indication to the user may be provided (e.g., the display/GUI may momentarily blink or change color, or some audio confirmation may be provided, such as a sound or one or more words).

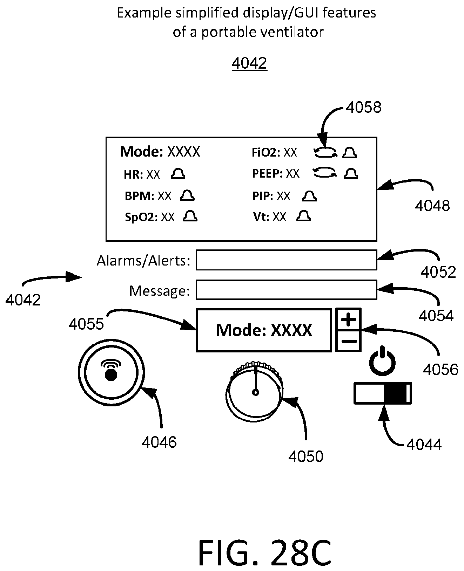

FIG. 28C illustrates simplified example included controls 4042 of an example portable ventilator 4000, including ventilation related settings controls including a power switch 4044 and communication or remote control indicator light 4046, which may be similar to those 4004, 4024, 4006, 4026 depicted in FIGs. 28A-B, for example. The included controls 4042 also include display/GUI area 4055 and plus and minus selection arrows 4056, which may be, in some ways, similar to the central display/GUI area 4032 and plus and minus selection arrows 4034 as depicted in FIG. 28B. However, in the embodiment depicted in FIG. 28C, no up and down arrows are provided. In this embodiment, instead of using up and down arrows to select an item for display (and potentially for the user to change a setting), a dial 4050 is provided. In some embodiments, for example, the user may turn the dial 4050 clockwise or counterclockwise to scroll through and change the currently displayed item in the display/GUI area 4055 (or other areas of the display/GUI) and may press the dial in to make certain selections, adjustments, changes, confirmations. In some embodiments, a scroll wheel may be included, allowing, for example, scrolling to choose a displayed item and pressing to select the item, such as to choose or dismiss an alarm, for example. In various embodiments, however, many variations may be used with regard to sets of included controls, such as different breadths and size of a set of included controls, particular types and items of controls, and different ways in which a user may interact or provide different forms of input.

In the embodiment depicted in FIG. 28C, a larger top display/GUI area 4048 displays various information associated with various ventilation related settings. The top display/GUI area 4048 includes display of a current mode of operation, such as a current type of ventilation that is being provided by the portable ventilator. The top display/GUI area also includes display of various patient and ventilation related parameters. These include, on the left side, HR, BPM and SpO2, and, on the right side, FiO2, PEEP, PIP and Vt. Sets of double arrows 4058 are shown next to the values for FiO2 and PEEP. These may indicate that FiO2 CLC and PEEP CLC are currently on/operating. In various embodiments, if FiO2 CLC or PEEP CLC was not currently on/operating, the corresponding sets of double arrows might not appear, or might appear but include a visible cross out, for example, to provide an appropriate visual indication to the user.

In a central area of the included controls 4042, an alarms/alerts display/GUI area 4052 and a message area 4052 are provided. The alarms/alerts display area 4052 may be used for display of any of various alarms or alerts for the user, such as may relate to concerning or unsafe patient or ventilation conditions or parameters. In some embodiments, displayed alarms may be coded or color coded, such as may indicate the priority or urgency of the alarm. For example, alarms displayed in green, yellow or red colors, or including associated green, yellow or red displayed lighted areas, may indicate increasing levels of priority or urgency. The message area 4054 may be used for display of any of various messages for the user, such as may include CSG, or may provide additional information relating to a provided alert or alarm, for example. In some embodiments, user scrolling may be used (such as using the dial 4050) to scroll through and change the currently displayed item in the alarms/alerts display/GUI area 4052 or the message area 4054. For example, in some embodiments, the user may be able to scroll up and down through a list of saved alerts or alarms, or a list of saved messages.

In some embodiments, a portable ventilator 4102, an example of which is provided in FIG. 1, may provide mechanical ventilation incorporating one or more forms of CLC, such as FiO2 CLC or PEEP CLC. In various embodiments, the CLC (or one or more forms) may be implemented via remote control by one or more remote control devices or systems, or via control by the portable ventilator, or with aspects of both. In some embodiments, control including CLC may or may not be subject to user interaction or include CSG. In various embodiments, if user interaction is included, it may take place at any of various devices, such as the portable ventilator 4102, a remote control device or another device or system in the environment.

The term CLC, as used herein, may refer to control of one or more ventilation related or patient related parameters, such as with relatively little or no required user action, participation or intervention, and can include reference to, but is not limited to reference to, fully automated or fully automatically regulated control. CLC may include, for example, device facilitated or algorithmically facilitated tracking, control and adjustment of one or more parameters, which may or may not include user involvement or participation. Where user involvement or participation is included, it may include, for example, confirming a suggested or recommended ventilation related setting change or configuration, deciding on implementing a course of action, selecting one of several suggested courses of action, responding to a presented alert or alarm, or other decisions, choices or actions. User involvement or participation could also include, for example, setting or changing a parameter, where a closed loop control algorithm proceeds from there, initially according to the user-set or user-changed parameter setting. In various embodiments, if there is user involvement, it may be, for example, among other things, in whole or in part user-initiated, or in whole or in part prompted, suggested, recommended or required.

In some embodiments, closed loop control may be utilized but may be subject to manual adjustment or override by the user. For example, in some embodiments, although FiO2 and PEEP may be algorithmically and automatically controlled, a user may be able to intervene and manually change the Fi02 and/or PEEP setting. In some embodiments, following any manual adjustments, closed loop control of FiO2 and/or PEEP may resume from that point, at least until any further manual adjustments are made.

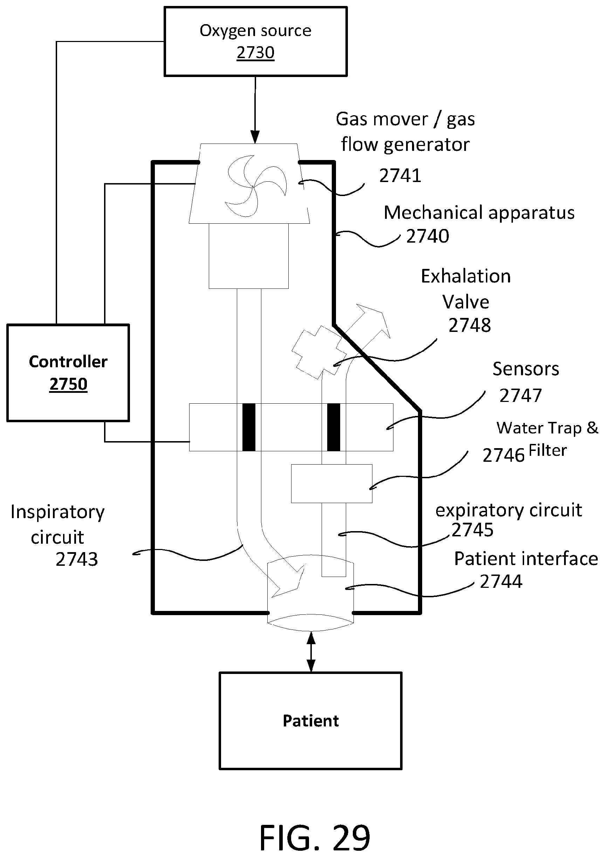

FIG. 29 illustrates aspects of an example of the mechanical ventilation apparatus for a ventilation system such as a portable ventilator, including a controller 2750 and connected to an oxygen source 2730, used in providing mechanical ventilation to a patient. The patient interface 2744 may include an appropriate gas delivery device, such as an intubation tube, mask, nasal cannula, etc. The mechanical ventilation apparatus 2740 further includes a gas mover/gas flow generator 2741, such as a blower, an expiratory circuit 2745 and an exhalation valve 2748 placed, for example, within the expiratory circuit . Both the inspiratory circuit 2743 and the expiratory circuit 2745 include sensors 2747. The sensors 2747 may include, for example, but not limited to, the pneumotachometer 275, the airway pressure sensor 274, and the spirometer 276. The sensors 2747 enable the ventilation system 280 to measure the patient's respiratory efforts as well as the performance of the ventilation system 280 when providing mechanical respiratory assistance to the patient. The sensors 2747 may generate and provide data to the CSG processor module 120, the data including but not limited to flow rate, tidal volume and minute volume (Ve), respiratory mechanics (e.g., resistance and compliance) and spirometry, and may include, for example, forced vital capacity (FVC), forced vital capacity at 1 second (FEV1) and peak expiratory flow rate (PEF or PEFR). In addition, the patient monitor/defibrillator 285 or another medical device may provide, for example, capnography and/or oximetry data, such as oxyhemoglobin and carboxyhemoglobin saturation and mainstream or other capnographic data such as end tidal CO2 (EtCO2). This data may allow, for example, for calculation of CO2 elimination rate and volumetric capnography, which may include using flow data from the ventilation system 280.

**In** some embodiments, for patients who require supplemental oxygen (02), the ventilation system may provide a number of methods to support oxygenation by invasive and non-invasive ventilation. For example, one method may include using a small reservoir bag system that allows entrainment from an O2 flow source. In some embodiments, in a first method, the user may manage the O2 delivery to the patient while monitoring the oxygen saturation (SpO2) measured by the patient monitor/defibrillator 285 or another medical device. A second method may make use of an innovative smart O2 valve (SOV) or module, which may, for example, attach to the inspiratory circuit 2743, a high-pressure O2 cylinder/source and the patient monitor/defibrillator or another medical device This may, for example, provide for automatic control of the patient's oxygenation using physiologic closed loop control (PCLC) where, the SpO2 signal may be used to regulate the output of the SOV. A third method may provide for the functional integration of a portable O2 concentrator (POC), which may, for example, use PCLC to regulate the O2 output of the POC and the O2 entrainment into the ventilation system 280.

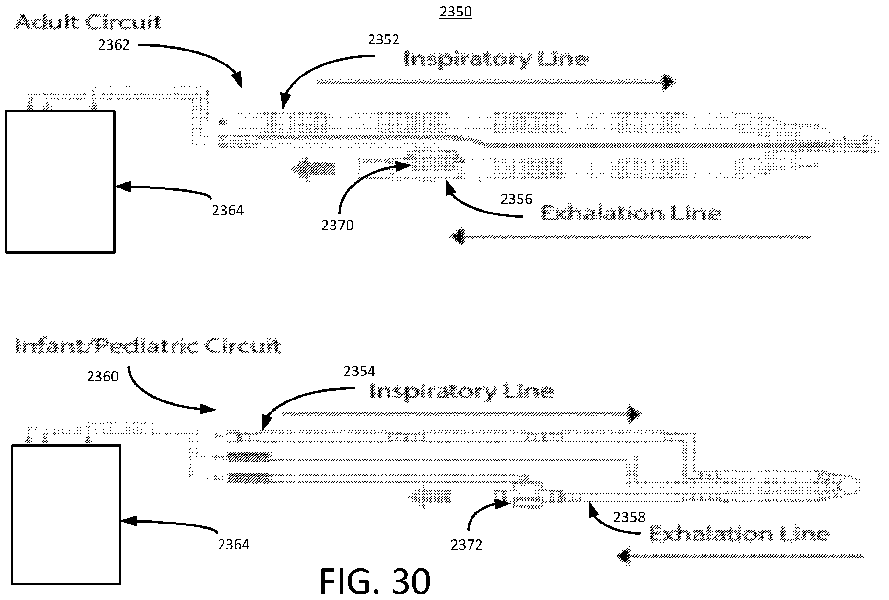

FIG. 30 illustrates aspects of example patient circuits 2350 that can be used with a portable ventilator 2364, in accordance with embodiments of the present disclosure. Depicted aspects include an adult circuit 2362, including an inspiratory line 2352 and an expiratory line 2356, and an infant/pediatric circuit 2360, including an inspiratory line 2354 and an expiratory line 2358. As depicted, each of the adult circuit 2362 and the pediatric circuit 2360 includes an exhalation valve 2370, 2372. However, in some embodiments, an exhalation valve may not be included in the patient circuits 2350 but may be included, for example, in the pneumatic manifold of the portable ventilator. Additionally, although not shown in the embodiments depicted, in some embodiments, the patient circuits 2350 may include a patient airway pressure tap.

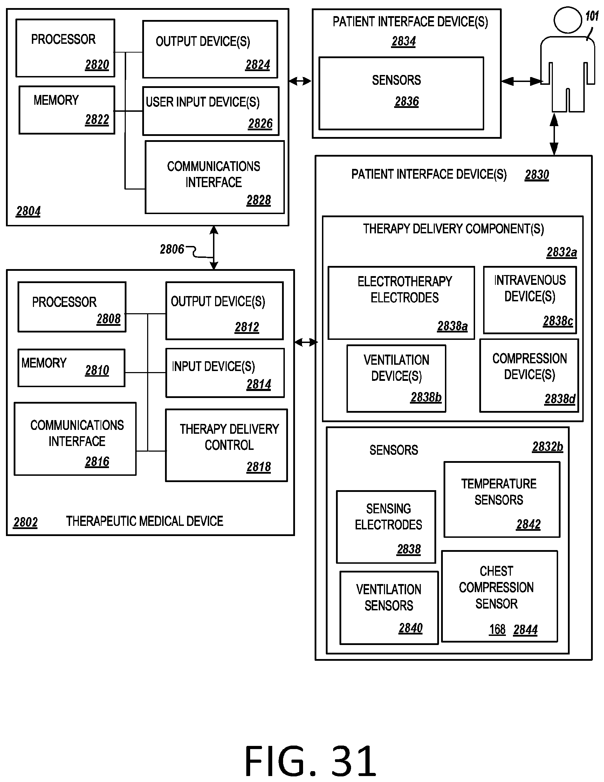

FIG. 31 illustrates an example of components of various devices described with reference to FIGs. 1-30. The components 2808, 2810, 2812, 2814, 2816, and 2818 are communicatively coupled (directly and/or indirectly) to each other for bi-directional communication. Similarly, the components 2820, 2822, 2824, 2826, and 2828 are communicatively coupled (directly and/or indirectly) to each other for bi-directional communication.

In some implementations, the components 2808, 2810, 2816, and/or 2818 of the therapeutic medical device 2802 may be combined into one or more discrete components and components 2816 and/or 2818 may be part of the processor 2808. The processor 2808 and the memory 2810 may include and/or be coupled to associated circuitry in order to perform the functions described herein. Additionally, the components 2820, 2822, and 2828 of companion device 2804 may be combined into one or more discrete components and component 2828 may be part of the processor 2820. The processor 2820 and the memory 2821 may include and/or be coupled to associated circuitry in order to perform the functions described herein.

In some implementations, the therapeutic medical device 2802 may include the therapy delivery control module 2818. For example, the therapy delivery control module 2818 may be an electrotherapy delivery circuit that includes one or more high-voltage capacitors configured to store electrical energy for a pacing pulse or a defibrillating pulse. The electrotherapy delivery circuit may further include resistors, additional capacitors, relays and/or switches, electrical bridges such as an H-bridge (e.g., including a plurality of insulated gate bipolar transistors or IGBTs), voltage measuring components, and/or current measuring components. As another example, the therapy delivery control module 2818 may be a compression device electro-mechanical controller configured to control a mechanical compression device. As a further example, the therapy delivery control module 2818 may be an electro-mechanical controller configured to control drug delivery, temperature management, ventilation, and/or other type of therapy delivery.

The therapeutic medical device 2802 may incorporate and/or be configured to couple to one or more patient interface devices 2830. The patient interface devices 2830 may include one or more therapy delivery component(s) 2832a and one or more sensor(s) 2832b. Similarly, the companion device 2804 may be adapted for medical use and may incorporate and/or be configured to couple to one or more patient interface device(s) 2834. The patient interface device(s) 2834 may include one or more sensors 2836. The sensor(s) 2836 may be substantially as described herein with regard to the sensor(s) 2832b.

The sensor(s) 2832b and 2836 may include sensing electrodes (e.g., the sensing electrodes 2838), ventilation and/or respiration sensors (e.g., the ventilation and/or respiration sensors 2830), temperature sensors (e.g., the temperature sensor 2842), chest compression sensors (e.g., the chest compression sensor 2844), etc. In some implementations, the information obtained from the sensors 2832b and 2836 can be used to generate information displayed at the therapeutic medical device 2802 and simultaneously at the display views at companion device 2804 and described above. In one example, the sensing electrodes 2838 may include cardiac sensing electrodes. The cardiac sensing electrodes may be conductive and/or capacitive electrodes configured to measure changes in a patient's electrophysiology to measure the patient's ECG information. The sensing electrodes 2838 may further measure the transthoracic impedance and/or a heart rate of the patient. The ventilation and/or respiration sensors 2830 may include spirometry sensors, flow sensors, pressure sensors, oxygen and/or carbon dioxide sensors such as, for example, one or more of pulse oximetry sensors, oxygenation sensors (e.g., muscle oxygenation/pH), O2 gas sensors and capnography sensors, impedance sensors, and combinations thereof. The temperature sensors 2842 may include an infrared thermometer, a contact thermometer, a remote thermometer, a liquid crystal thermometer, a thermocouple, a thermistor, etc. and may measure patient temperature internally and/or externally. The chest compression sensor 2844 may include one or more motion sensors including, for example, one or more accelerometers, one or more force sensors, one or more magnetic sensors, one or more velocity sensors, one or more displacement sensors, etc. The chest compression sensor 2844 may provide one or more signals indicative of the chest motion to the therapeutic medical device 2802 via a wired and/or wireless connection. The chest compression sensor 2844 may be, for example, but not limited to, a compression puck, a smart-phone, a hand-held device, a wearable device, etc. The chest compression sensor 2844 may be configured to detect chest motion imparted by a rescuer and/or an automated chest compression device (e.g., a belt system, a piston system, etc.). The chest compression sensor 2844 may provide signals indicative of chest compression data including displacement data, velocity data, release velocity data, acceleration data, force data, compression rate data, dwell time data, hold time data, blood flow data, blood pressure data, etc. In an implementation, the defibrillation and/or pacing electrodes may include or be configured to couple to the chest compression sensor 2844.

In various implementations, the sensors 2832b and 2836 may include one or more sensor devices configured to provide sensor data that includes, for example, but not limited to ECG, blood pressure, heart rate, respiration rate, heart sounds, lung sounds, respiration sounds, end tidal CO₂, saturation of muscle oxygen (SMO₂), oxygen saturation (e.g., SpO₂ and/or PaO₂), cerebral blood flow, point of care laboratory measurements (e.g., lactate, glucose, etc.), temperature, electroencephalogram (EEG) signals, brain oxygen level, tissue pH, tissue fluid levels, images and/or videos via ultrasound, laryngoscopy, and/or other medical imaging techniques, near-infrared spectroscopy, pneumography, cardiography, and/or patient movement. Images and/or videos may be two-dimensional or three-dimensional, such a various forms of ultrasound imaging.

The one or more therapy delivery components 2832a may include electrotherapy electrodes (e.g., the electrotherapy electrodes 2838a), ventilation device(s) (e.g., the ventilation devices 2838b), intravenous device(s) (e.g., the intravenous devices 2838c), compression device(s) (e.g., the compression devices 2838d), etc. For example, the electrotherapy electrodes 2838a may include defibrillation electrodes, pacing electrodes, and combinations thereof. The ventilation devices 2838b may include a tube, a mask, an abdominal and/or chest compressor (e.g., a belt, a cuirass, etc.), etc. and combinations thereof. The intravenous devices 2838c may include drug delivery devices, fluid delivery devices, and combinations thereof. The compression devices 2838d may include mechanical compression devices such as abdominal compressors, chest compressors, belts, pistons, and combinations thereof. In various implementation, the therapy delivery component(s) 2832a may be configured to provide sensor data and/or be coupled to and/or incorporate sensors. For example, the electrotherapy electrodes 2838a may provide sensor data such as transthoracic impedance, ECG, heart rate, etc. Further the electrotherapy electrodes 2838a may include and or be coupled to a chest compression sensor. As another example, the ventilation devices 2838b may be coupled to and/or incorporate flow sensors, gas species sensors (e.g., oxygen sensor, carbon dioxide sensor, etc.), etc. As a further example, the intravenous devices 2838c may be coupled to and/or incorporate temperature sensors, flow sensors, blood pressure sensors, etc. As yet another example, the compression devices 2838d may be coupled to and/or incorporate chest compression sensors, patient position sensors, etc. The therapy delivery control modules 2818 may be configured to couple to and control the therapy delivery component(s) 2832a, respectively.

The one or more sensor(s) 2832b and 2836 and/or the therapy delivery component(s) 2832a may provide sensor data. The patient data provided at the display screens of the therapeutic medical device 2802and companion device 2804 may display the sensor data. For example, the therapeutic medical device 2802 may process signals received from the sensor(s) 2832b and/or the therapy delivery component(s) 2832a to determine the sensor data. Similarly, the companion device 2804 may process signals received from the sensor(s) 2836 and/or sensor data from the sensors 2832b received via the therapeutic medical device 2802to determine the sensor data.

While certain embodiments have been described, these embodiments have been presented by way of example only and are not intended to limit the scope of the present disclosures. Indeed, the novel methods, apparatuses and systems described herein can be embodied in a variety of other forms; furthermore, various omissions, substitutions and changes in the form of the methods, apparatuses and systems described herein can be made without departing from the scope the present disclosures. The scope of the present invention is defined by the claims that follow.

## Claims

1. An apparatus for ventilating a patient or treating a patient experiencing respiratory distress, the apparatus comprising:
a portable ventilator (900, 2364), comprising:
a housing (902);
a mechanical ventilation apparatus, disposed within the housing, for providing mechanical ventilation to the patient, comprising:
a gas flow generator (912, 1053) disposed within the housing, and
a gas delivery apparatus (910), disposed at least partially within the housing, coupled with the gas flow generator, the gas delivery apparatus comprising:
a rigid pneumatic manifold (1050), comprising an exhalation channel and an inhalation channel, configured to couple with a breathing circuit extending from the housing and configured to interface with the patient at least in part for delivery of gas to the patient, and
at least one electrical circuit board (1052), comprising at least one pressure sensor and at least two flow sensors comprising a first flow sensor and a second flow sensor, the at least one electrical circuit board being fixedly joined with the pneumatic manifold such that the first flow sensor is capable of measuring a first gas flow rate through the exhalation channel and such that the second flow sensor is capable of measuring a second gas flow rate through the inhalation channel; and
a controller (918), disposed within the housing, the controller comprising a processor (920) and a memory (916), the controller being configured to control the mechanical ventilation apparatus of the portable ventilator in providing the mechanical ventilation to the patient.

2. The apparatus of claim 1, wherein the at least one pressure sensor (914) comprises a first pressure sensor and a second pressure sensor, and wherein the at least one electrical circuit board is configured such that the first pressure sensor is capable of measuring a first gas pressure through the exhalation channel and such that the second pressure sensor is capable of measuring a second gas pressure through the inhalation channel.

3. The apparatus of claim 1, wherein the at least one pressure sensor (914) comprises a first, a second, a third and a fourth pressure sensor, and wherein the at least one electrical circuit board is configured such that the first and second pressure sensors are capable of measuring a first gas pressure through the exhalation channel and such that the third and fourth pressure sensors are capable of measuring a second gas pressure through the inhalation channel.

4. The apparatus of claim 1, wherein the at least one electrical circuit board and the pneumatic manifold are configured so as to be joined so as to create a fixed but flexible gas leak-free seal between the pneumatic manifold and at least one pressure sensor and between the pneumatic manifold and each of the at least two flow sensors.

5. The apparatus of claim 4, wherein one of:
the fixed but flexible gas leak-free seal comprises at least one O-ring;
the fixed but flexible gas leak-free seal comprises at least one gasket; or
the fixed but flexible gas leak-free seal has a temperature tolerance range of at least between 0 and 40 degrees Celsius.

6. The apparatus of any preceding claim, wherein the at least one electrical circuit board is rigid.

7. The apparatus of any preceding claim, wherein the portable ventilator (900) comprises an exhalation valve (440, 801, 1910) configured for use in restriction of gas flow through at least a portion of the exhalation channel; and, optionally,
wherein the exhalation valve (440, 801, 1910) is configured for use, during the providing of the mechanical ventilation to the patient, in maintaining specified exhalation periods and in maintaining a specified baseline airway pressure, BAP.

8. The apparatus of claim 7, wherein the exhalation valve (440, 801, 1910) is one of:
a proportional valve;
a pneumatically actuated valve; and
a diaphragm valve.

9. The apparatus of any preceding claim, wherein the gas flow generator comprises a blower (436, 912), and, optionally, the gas flow generator comprises a centrifugal blower.

10. The apparatus of claim 9, wherein the portable ventilator is configured such that all gas flow generated by the blower (436, 912) is delivered into an inhalation limb of the breathing circuit.

11. The apparatus of claim 7, wherein the portable ventilator (900) comprises, disposed within the housing, an exhalation valve pressure regulator (404, 1054, 1202, 1302, 1502) configured for use in providing a specified actuation pressure to the exhalation valve.

12. The apparatus of any preceding claim, wherein the portable ventilator (900) comprises, disposed within the housing, an air characterizer (1501) comprising the at least one electrical circuit board.

13. The apparatus of claim 11, wherein the portable ventilator (900) comprises, disposed within the housing, an air characterizer (1501) comprising the at least one electrical circuit board, and wherein the exhalation valve pressure regulator and the air characterizer are configured to fixedly join so as to form at least a portion of an exhalation valve control channel extending from the exhalation valve pressure regulator to the air characterizer.

14. The apparatus of claim 7,
wherein the portable ventilator (900) comprises, disposed within the housing, an air characterizer (1501) comprising the at least one electrical circuit board,
wherein the exhalation valve is disposed within the breathing circuit (452, 610, 812, 913) and
wherein the apparatus comprises an exhalation valve control tube extending from the breathing circuit to within the housing of the portable ventilator and configured for use in providing actuation pressure to the exhalation valve.

15. The apparatus of claim 14, comprising a patient airway pressure measurement tube extending from the breathing circuit to within the housing of the portable ventilator and configured for use in allowing measuring of a patient airway pressure using at least a second pressure sensor disposed within the housing; and
wherein the apparatus comprises a connector, the connector comprising:
a first port configured for attachment of an exhalation limb of the breathing circuit;
a second port configured for attachment of an inhalation limb of the breathing circuit;
a third port configured for attachment of the exhalation valve control tube; and
a fourth port configured for attachment of the patient airway measurement tube;
wherein the connector is configured to fixedly attach to the portable ventilator so as to couple the exhalation limb of the breathing circuit with the exhalation channel of the pneumatic manifold, to couple the inhalation limb of the breathing circuit with the inhalation channel of the pneumatic manifold, to couple the exhalation valve control tube with the pneumatic manifold, and to couple the patient airway measurement tube with the pneumatic manifold.

## Patentansprüche

1. Gerät zur Beatmung eines Patienten oder zur Behandlung eines Patienten, der unter Atemnot leidet, das Gerät umfassend:
einen tragbaren Ventilator (900, 2364), umfassend:
ein Gehäuse (902);
ein im Gehäuse angeordnetes mechanisches Beatmungsgerät zum Bereitstellen einer mechanischen Beatmung für den Patienten, umfassend:
einem in dem Gehäuse angeordneten Gasflussgenerator (912, 1053), und
ein Gasabgabegerät (910), das mindestens teilweise innerhalb des Gehäuses angeordnet und mit dem Gasflussgenerator gekoppelt ist, das Gasabgabegerät umfassend:
einen starren pneumatischen Verteiler (1050), umfassend einen Ausatmungskanal und einen Einatmungskanal, der konfiguriert ist, um mit einem Atemkreislauf zu koppeln,
der sich von dem Gehäuse erstreckt, und der konfiguriert ist, um mindestens teilweise mit dem Patienten in Verbindung zu treten, um dem Patienten Gas zuzuführen, und
mindestens eine elektrische Schaltungsplatte (1052), die mindestens einen Drucksensor und mindestens zwei Durchflusssensoren umfasst, umfassend einen ersten Durchflusssensor und einen zweiten Durchflusssensor, wobei die mindestens eine elektrische Schaltungsplatte fest mit dem pneumatischen Verteiler verbunden ist, sodass der erste Durchflusssensor in der Lage ist, eine erste Gasdurchflussrate durch den Ausatmungskanal zu messen, und sodass der zweite Durchflusssensor in der Lage ist, eine zweite Gasdurchflussrate durch den Einatmungskanal zu messen; und
eine Steuerung (918), die in dem Gehäuse angeordnet ist, wobei die Steuerung einen Prozessor (920) und einen Speicher (916) umfasst, wobei die Steuerung konfiguriert ist, um das mechanische Beatmungsgerät des tragbaren Ventilators beim Bereitstellen der mechanischen Beatmung für den Patienten zu steuern.

2. Gerät nach Anspruch 1, wobei der mindestens eine Drucksensor (914) einen ersten Drucksensor und einen zweiten Drucksensor umfasst, und wobei die mindestens eine elektrische Schaltungsplatte konfiguriert ist, sodass der erste Drucksensor in der Lage ist, einen ersten Gasdruck durch den Ausatmungskanal zu messen, und sodass der zweite Drucksensor in der Lage ist, einen zweiten Gasdruck durch den Einatmungskanal zu messen.

3. Gerät nach Anspruch 1, wobei der mindestens eine Drucksensor (914) einen ersten, einen zweiten, einen dritten und einen vierten Drucksensor umfasst, und wobei die mindestens eine elektrische Schaltungsplatte konfiguriert ist, sodass der erste und der zweite Drucksensor in der Lage sind, einen ersten Gasdruck durch den Ausatmungskanal zu messen, und sodass der dritte und der vierte Drucksensor in der Lage sind, einen zweiten Gasdruck durch den Einatmungskanal zu messen.

4. Gerät nach Anspruch 1, wobei die mindestens eine elektrische Schaltungsplatte und der pneumatische Verteiler konfiguriert sind, um so verbunden zu werden, dass eine feste, aber flexible, gasleckfreie Dichtung zwischen dem pneumatischen Verteiler und mindestens einem Drucksensor und zwischen dem pneumatischen Verteiler und jedem der mindestens zwei Flusssensoren entsteht.

5. Gerät nach Anspruch 4, wobei eines von:
der festen, aber flexiblen, gasdichten Dichtung mindestens einen O-Ring umfasst;
der festen, aber flexiblen, gasdichten Dichtung mindestens eine Dichtung umfasst; oder
der festen, aber flexiblen, gasdichten Dichtung mindestens einen Temperaturtoleranzbereich zwischen 0 und 40 Grad Celsius aufweist.

6. Gerät nach einem der vorstehenden Ansprüche, wobei die mindestens eine elektrische Schaltungsplatte starr ist.

7. Gerät nach einem der vorstehenden Ansprüche, wobei der tragbare Ventilator (900) ein Ausatmungsventil (440, 801, 1910) umfasst, das konfiguriert ist, um den Gassdurchfluss durch mindestens einen Abschnitt des Ausatmungskanals zu begrenzen; und, optional,
wobei das Ausatmungsventil (440, 801, 1910) konfiguriert ist, um während des Bereitstellens der mechanischen Beatmung des Patienten spezifizierte Ausatmungsperioden und einen spezifizierten Grundlinien-Atemwegsdruck, BAP, aufrechtzuerhalten.

8. Gerät nach Anspruch 7, wobei das Ausatmungsventil (440, 801, 1910) eines von Folgendem ist:
ein proportionales Ventil;
ein pneumatisch betätigtes Ventil; und
ein Membranventil.

9. Gerät nach einem der vorstehenden Ansprüche, wobei der Gasdurchflussgenerator ein Gebläse (436, 912) umfasst, und optional umfasst der Gasdurchflussgenerator ein Zentrifugalgebläse.

10. Gerät nach Anspruch 9, wobei der tragbare Ventilator konfiguriert ist, sodass der gesamte vom Gebläse (436, 912) erzeugte Gasdurchfluss in ein Einatmungsglied des Atemkreislaufs geleitet wird.

11. Gerät nach Anspruch 7, wobei der tragbare Ventilator (900) einen im Gehäuse angeordneten Druckregler (404, 1054, 1202, 1302, 1502) für das Ausatmungsventil umfasst, der konfiguriert ist, um einen bestimmten Betätigungsdruck für das Ausatmungsventil bereitzustellen.

12. Gerät nach einem der vorstehenden Ansprüche, wobei der tragbare Ventilator (900) in dem Gehäuse einen Luftcharakterisierer (1501) umfasst, der mindestens eine elektrische Schaltungsplatte umfasst.

13. Gerät nach Anspruch 11, wobei der tragbare Ventilator (900) einen in dem Gehäuse angeordneten Luftcharakterisierer (1501) umfasst, der die mindestens eine elektrische Schaltungsplatte umfasst, und wobei der Ausatmungsventildruckregler und der Luftcharakterisierer konfiguriert sind, um fest miteinander verbunden zu werden, sodass sie mindestens einen Abschnitt eines Ausatmungsventilsteuerkanals bilden, der sich von dem Ausatmungsventildruckregler zu dem Luftcharakterisierer erstreckt.

14. Gerät nach Anspruch 7,
wobei der tragbare Ventilator (900) in dem Gehäuse einen Luftcharakterisierer (1501) umfasst, der mindestens eine elektrische Schaltungsplatte umfasst,
wobei das Ausatmungsventil innerhalb des Atemkreislaufs (452, 610, 812, 913) angeordnet ist und
wobei das Gerät einen Ausatmungsventilsteuerungsschlauch umfasst, der sich vom Atemkreislauf bis in das Gehäuse des tragbaren Ventilators erstreckt und zur Verwendung beim Bereitstellen des Betätigungsdrucks für das Ausatmungsventil konfiguriert ist.

15. Gerät nach Anspruch 14, umfassend einen Schlauch zur Messung des Drucks in den Atemwegen des Patienten, der sich von dem Atemkreislauf bis in das Gehäuse des tragbaren Ventilators erstreckt und konfiguriert ist, um das Messen des Drucks in den Atemwegen des Patienten unter Verwendung mindestens eines zweiten Drucksensors, der in dem Gehäuse angeordnet ist, zu ermöglichen; und wobei das Gerät einen Verbinder umfasst, der Verbinder umfassend:
einen ersten Anschluss, der für die Befestigung eines Ausatmungsglieds des Atemkreislaufs konfiguriert ist;
einen zweiten Anschluss, der für die Befestigung eines Einatmungsglieds des Atemkreislaufs konfiguriert ist;
einen dritten Anschluss, der für die Befestigung des Steuerschlauchs des Ausatmungsventils konfiguriert ist; und
einen vierten Anschluss, der für die Befestigung des Patientenatemwegsmessschlauchs konfiguriert ist;
wobei der Verbinder konfiguriert ist, um fest mit dem tragbaren Ventilator verbunden zu werden, um das Ausatmungsglied des Atemkreislaufs mit dem Ausatmungskanal des pneumatischen Verteilers zu koppeln, um das Einatmungsglied des Atemkreislaufs mit dem Einatmungskanal des pneumatischen Verteilers zu koppeln, um den Ausatmungsventilsteuerschlauch mit dem pneumatischen Verteiler zu koppeln und um den Atemwegsmessschlauch des Patienten mit dem pneumatischen Verteiler zu koppeln.

## Revendications

1. Appareil pour ventiler un patient ou traiter un patient souffrant d'une détresse respiratoire, l'appareil comprenant :
un ventilateur portable (900, 2364), comprenant :
un boîtier (902) ;
un appareil de ventilation mécanique, disposé à l'intérieur du boîtier, pour fournir une ventilation mécanique au patient, comprenant :
un générateur de flux de gaz (912, 1053) disposé à l'intérieur du boîtier, et
un appareil de distribution de gaz (910), disposé au moins partiellement à l'intérieur du boîtier, couplé au générateur de flux de gaz, l'appareil de distribution de gaz comprenant :
un collecteur pneumatique rigide (1050), comprenant un canal d'expiration et un canal d'inspiration, configuré pour se coupler à un circuit respiratoire se prolongeant à partir du boîtier et configuré pour s'interfacer avec le patient au moins en partie pour distribuer du gaz au patient, et
au moins une carte de circuit électrique (1052), comprenant au moins un capteur de pression et au moins deux capteurs de flux, comprenant un premier capteur de flux et un second capteur de flux, l'au moins une carte de circuit électrique étant reliée de manière fixe au collecteur pneumatique de telle sorte que le premier capteur de flux est capable de mesurer un premier débit de gaz à travers le canal d'expiration et de telle sorte que le second capteur de flux est capable de mesurer un second débit de gaz à travers le canal d'inspiration ; et
un dispositif de commande (918), disposé à l'intérieur du boîtier, le dispositif de commande comprenant un processeur (920) et une mémoire (916), le dispositif de commande étant configuré pour commander l'appareil de ventilation mécanique du ventilateur portable pour fournir la ventilation mécanique au patient.

2. Appareil selon la revendication 1, dans lequel l'au moins un capteur de pression (914) comprend un premier capteur de pression et un deuxième capteur de pression, et dans lequel l'au moins une carte de circuit électrique est configurée de telle sorte que le premier capteur de pression est capable de mesurer une première pression de gaz à travers le canal d'expiration et de telle sorte que le deuxième capteur de pression est capable de mesurer une seconde pression de gaz à travers le canal d'inspiration.

3. Appareil selon la revendication 1, dans lequel l'au moins un capteur de pression (914) comprend un premier, un deuxième, un troisième et un quatrième capteur de pression, et dans lequel l'au moins une carte de circuit électrique est configurée de telle sorte que les premier et deuxième capteurs de pression sont capables de mesurer une première pression de gaz à travers le canal d'expiration et de telle sorte que les troisième et quatrième capteurs de pression sont capables de mesurer une seconde pression de gaz à travers le canal d'inspiration.

4. Appareil selon la revendication 1, dans lequel l'au moins une carte de circuit électrique et le collecteur pneumatique sont configurés de manière à être reliés de façon à créer un joint étanche aux fuites de gaz fixe mais flexible entre le collecteur pneumatique et au moins un capteur de pression et entre le collecteur pneumatique et chacun des au moins deux capteurs de flux.

5. Appareil selon la revendication 4, dans lequel l'un des éléments suivants :
le joint étanche aux fuites de gaz fixe mais flexible comprend au moins un joint torique ;
le joint étanche aux fuites de gaz fixe mais flexible comprend au moins un joint d'étanchéité ; ou
le joint étanche aux fuites de gaz fixe mais flexible a une plage de tolérance de température comprise entre au moins 0 et 40 degrés Celsius.

6. Appareil selon une quelconque revendication précédente, dans lequel l'au moins une carte de circuit électrique est rigide.

7. Appareil selon une quelconque revendication précédente, dans lequel le ventilateur portable (900) comprend une valve d'expiration (440, 801, 1910) configurée pour être utilisée pour restreindre le flux de gaz à travers au moins une partie du canal d'expiration ; et, éventuellement,
dans lequel la valve d'expiration (440, 801, 1910) est configurée pour être utilisée, lors de la fourniture de la ventilation mécanique au patient, pour maintenir des périodes d'expiration spécifiées et pour maintenir une pression des voies respiratoires de base, BAP, spécifiée.

8. Appareil selon la revendication 7, dans lequel la valve d'expiration (440, 801, 1910) est l'une des suivantes :
une valve proportionnelle ;
une valve actionnée pneumatiquement ; et
une valve à membrane.

9. Appareil selon une quelconque revendication précédente, dans lequel le générateur de flux de gaz comprend une soufflante (436, 912), et, éventuellement, le générateur de flux de gaz comprend une soufflante centrifuge.

10. Appareil selon la revendication 9, dans lequel le ventilateur portable est configuré de telle sorte que tout le flux de gaz généré par la soufflante (436, 912) est délivré dans une branche d'inspiration du circuit respiratoire.

11. Appareil selon la revendication 7, dans lequel le ventilateur portable (900) comprend, disposé à l'intérieur du boîtier, un régulateur de pression de valve d'expiration (404, 1054, 1202, 1302, 1502) configuré pour être utilisé pour fournir une pression d'actionnement spécifiée à la valve d'expiration.

12. Appareil selon une quelconque revendication précédente, dans lequel le ventilateur portable (900) comprend, disposé à l'intérieur du boîtier, un caractérisateur d'air (1501) comprenant l'au moins une carte de circuit électrique.

13. Appareil selon la revendication 11, dans lequel le ventilateur portable (900) comprend, disposé à l'intérieur du boîtier, un caractérisateur d'air (1501) comprenant l'au moins une carte de circuit électrique, et dans lequel le régulateur de pression de valve d'expiration et le caractérisateur d'air sont configurés pour se relier de manière fixe de manière à former au moins une partie d'un canal de commande de valve d'expiration se prolongeant à partir du régulateur de pression de valve d'expiration vers le caractérisateur d'air.

14. Appareil selon la revendication 7,
dans lequel le ventilateur portable (900) comprend, disposé à l'intérieur du boîtier, un caractérisateur d'air (1501) comprenant l'au moins une carte de circuit électrique,
dans lequel la valve d'expiration est disposée à l'intérieur du circuit respiratoire (452, 610, 812, 913) et
dans lequel l'appareil comprend un tube de commande de valve d'expiration se prolongeant à partir du circuit respiratoire vers l'intérieur du boîtier du ventilateur portable et configuré pour être utilisé pour fournir une pression d'actionnement à la valve d'expiration.

15. Appareil selon la revendication 14, comprenant un tube de mesure de pression des voies respiratoires de patient se prolongeant à partir du circuit respiratoire vers l'intérieur du boîtier du ventilateur portable et configuré pour être utilisé pour permettre de mesurer une pression des voies respiratoires de patient à l'aide d'au moins un deuxième capteur de pression disposé à l'intérieur du boîtier ; et
dans lequel l'appareil comprend un connecteur, le connecteur comprenant :
un premier orifice configuré pour la fixation d'une branche d'expiration du circuit respiratoire ;
un deuxième orifice configuré pour la fixation d'une branche d'inspiration du circuit respiratoire ;
un troisième orifice configuré pour la fixation du tube de commande de valve d'expiration ; et
un quatrième orifice configuré pour la fixation du tube de mesure des voies respiratoires de patient ;
dans lequel le connecteur est configuré pour se fixer de manière fixe au ventilateur portable de manière à coupler la branche d'expiration du circuit respiratoire avec le canal d'expiration du collecteur pneumatique, à coupler la branche d'inspiration du circuit respiratoire avec le canal d'inspiration du collecteur pneumatique, à coupler le tube de commande de valve d'expiration avec le collecteur pneumatique, et à coupler le tube de mesure des voies respiratoires de patient avec le collecteur pneumatique.
